(19) Europäisches Patentamt
European Patent Office
Office européen des brevets

(11) EP 1 741 702 A1

(12) EUROPEAN PATENT APPLICATION
published in accordance with Art. 158(3) EPC

(43) Date of publication:
**10.01.2007 Bulletin 2007/02**

(21) Application number: **05737345.8**

(22) Date of filing: **27.04.2005**

(51) Int Cl.:
***C07D 211/82*** *(2006.01)*

(86) International application number:
**PCT/JP2005/008065**

(87) International publication number:
**WO 2005/105743 (10.11.2005 Gazette 2005/45)**

(84) Designated Contracting States:
**AT BE BG CH CY CZ DE DK EE ES FI FR GB GR HU IE IS IT LI LT LU MC NL PL PT RO SE SI SK TR**

(30) Priority: **28.04.2004 JP 2004134631**
**15.11.2004 JP 2004330260**

(71) Applicant: **ONO PHARMACEUTICAL CO., LTD.**
**Osaka-shi, Osaka 541-8526 (JP)**

(72) Inventors:
- **NAKAI, Hisao,**
**c/o ONO PHARMACEUTICAL CO., LTD.**
**Mishima-gun, Osaka 6188585 (JP)**
- **YAMAMOTO, Shingo,**
**c/o ONO PHARMACEUTICAL CO., LTD.**
**Mishima-gun, Osaka 6188585 (JP)**
- **SUGITANI, Masafumi,**
**c/o ONO PHARMACEUTICAL CO. LTD**
**Mishima-gun, Osaka 6188585 (JP)**

(74) Representative: **Henkel, Feiler & Hänzel**
**Maximiliansplatz 21**
**D-80333 München (DE)**

# (54) NITROGEN-CONTAINING HETEROCYCLIC COMPOUNDS AND MEDICINAL USE THEREOF

(57) The present invention provides a compound having p38 MAP kinase inhibitory activity. Because of having p38 MAP kinase inhibitory activity, the compounds represented by the formula (I):

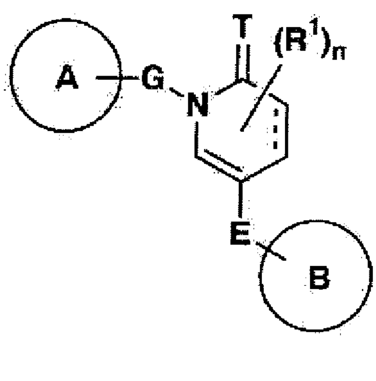

(I)

wherein each symbol is as defined in the description, salts thereof, N-oxides thereof, solvates thereof, or prodrugs thereof are useful in preventing and/or treating diseases that are supposedly caused or deteriorated by abnormal production of cytokines including inflammatory cytokines or chemokines, or by overresponse thereto, namely cytokine-mediated diseases such as inflammatory diseases, respiratory diseases, cardiovascular diseases and bone diseases.

EP 1 741 702 A1

## Description

TECHNICAL FIELD

The present invention relates to a nitrogen-containing heterocyclic compound that has p38 MAP kinase inhibitory activity and is useful as drug medicine, the process for preparation thereof and the use thereof.

BACKGROUND ART

p38 mitogen-activated protein (MAP) kinase (p38α /Mpk2 /RK /SAPK2a /CSBP) (hereinafter referred to as "p38MAP kinase") was cloned as an enzyme which induces tyrosine phosphorylation in monocyte after stimulation with lipopolysaccharide (LPS) (Nature, 372, 739 (1994)), and is activated by various extracellular stimuli [physical stimuli (osmotic shock, heat shock, UV irradiation, and so forth), chemical stimuli (endotoxin, hydrogen peroxide, arsenic trioxide, an inflammatory cytokine, a growth factor, and so forth), and so on]. Also, since p38 MAP kinase is involved in the production of an inflammatory cytokine (such as tumor necrosis factor-α (TNF-α), interleukin-1 (IL-1), IL-6, IL-8, and so forth), a chemokine, and so on, an association between the activation of this enzyme and diseases is strongly suggested. Therefore, an improvement effect on various disease symptoms typified by inflammatory diseases is expected by suppression of p38 MAP kinase activation.

Accordingly, a p38 MAP kinase inhibitor is expected to be useful in prevention and/or treatment of those diseases that are supposedly caused or deteriorated by abnormal production of cytokines including inflammatory cytokine or chemokine, or by over response thereto, namely cytokine-mediated diseases such as various inflammatory diseases [for example, inflammation, dermatitis, atopic dermatitis, hepatitis, nephritis, glomerulonephritis, pancreatitis, psoriasis, gout, Addison's disease, arthrititis (e.g. rheumatoid arthritis, osteoarthritis, rhumatoid spondylitis, gouty arthritis, synovitis, etc.), inflammatory ocular diseases, inflammatory pulmonary diseases (e.g. chronic pneumonia, silicosis, pulmonary sarcoidosis, pulmonary tuberculosis, adult respiratory distress syndrome (ARDS), severe acute respiratory syndrome (SARS), *etc.*), inflammatory bowel diseases *(e.g.* Crohn's disease, ulcerative colitis, *etc.),* allergic diseases (*e.g*. allergic dermatitis, allergic rhinitis, *etc.),* autoimmune disease, autoimmune hemolytic anemia, systemic lupus erythematosus, rheumatism, Castleman's disease, immune rejection accompanying transplantation (e.g. graft versus host reaction, *etc.),* and so forth], central nervous system disorders [for example, central neuropathy (e.g. cerebrovascular disease such as cerebral hemorrhage and cerebral infarction, head trauma, spinal cord injury, cerebral edema, multiple sclerosis, *etc.),* neurodegenerative disease (e.g. Alzheimer's disease, Parkinson's disease, amyotrophic lateral sclerosis (ALS), AIDS encephalopathy, *etc.*), meningitis, Creutzfeldt-Jakob syndrome, and so forth], respiratory diseases [for example, asthma, chronic obstructive pulmonary disease (COPD), and so forth], cardiovascular diseases [for example, angina, heart failure (*e.g.* congestive heart failure, acute heart failure, chronic heart failure, *etc.*), myocardial infarction *(e.g.* acute myocardial infarction, myocardial infarction prognosis, *etc.*), atrial myxoma, arteriosclerosis, hypertension, dialysis-induced hypotension, thrombosis, disseminated intravascular coagulation (DIC), reperfusion injury, restenosis after percutaneous transluminal coronary angioplasty (PTCA), and so forth], urinary diseases [for example, renal failure, and so forth], metabolic diseases or endocrine diseases [for example, diabetes, and so forth], bone diseases [for example, osteoporosis, and so forth], cancerous diseases [for example, malignant tumor (e.g. tumor growth and metastasis, *etc.*), multiple myeloma, plasma cell leukemia, carcinemia, and so forth], and infectious diseases [for example, viral infection (*e.g.* cytomegalovirus infection, influenza virus infection, herpes virus infection, corona virus infection, *etc.*), cachexia associated with infections, cachexia caused by acquired immune deficiency syndrome (AIDS), toxemia (e.g. sepsis, septic shock, endotoxin shock, gram negative bacterial sepsis, toxic shock syndrome, severe acute respiratory syndrome (SARS) accompanying virus infection, *etc.*), and so forth], and so on.

On the other hand, WO 03/043988 discloses that the compounds represented by general formula (A) or the non-toxic salts thereof are useful as p38 MAP kinase inhibitors :

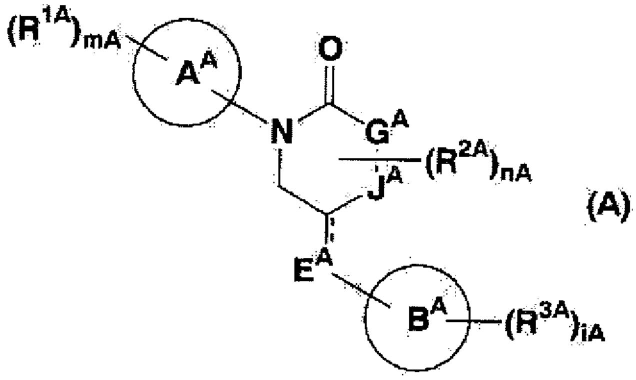

(A)

wherein

$A^A$ represents a C5-10 mono- or poly-cyclic carbon ring, or a 5- to 10-membered mono- or poly-cyclic hetero ring containing 1 to 5 nitrogen atom(s), 1 to 2 oxygen atom(s) and/or 1 sulfur atom ;

$R^{1A}$ represents (1) C1-8 alkyl, (2) C2-8 alkenyl, (3) C2-8 alkynyl, (4) halogen, (5) - $OR^{4A}$, (6) -$NR^{5A}R^{6A}$, (7) -$NR^{7A}COR^{8A}$, (8) -$CONR^{9A}R^{10A}$, (9) -$COOR^{11A}$, (10) - $SO_2NR^{12A}R^{13A}$,(11) -$NR^{14A}SO_2R^{15A}$, (12) -$SR^{16A}$, (13) -$S(O)R^{17A}$, (14) -$SO_2R^{18A}$, (15) - $NR^{22A}COOR^{23A}$, (16) -$NR^{24A}CONR^{25A}R^{26A}$, (17) -$COR^{27A}$, (18) nitro, (19) cyano, (20) trifluoromethyl, (21) trifluoromethoxy, (22) $Cycl^A$, or the like;

$R^{4A}$-$R^{18A}$ and $R^{22A}$-$R^{27A}$ each independently represent a hydrogen atom, C1-8 alkyl, $Cycl^A$, or the like ;

$Cycl^A$ represents a C5-10 mono- or poly-cyclic carbon ring or the like (with the proviso that, the carbon ring or the like may be substituted with 1 to 5 $R^{48A}$(s));

$R^{48A}$ represents C1-8 alkyl, halogen, nitro, cyano, or the like ;

$R^{2A}$ represents C1-8 alkyl, -$OR^{20A}$, -$NR^{64A}R^{65A}$, -$COOR^{66A}$, -$CONR^{67A}R^{68A}$,-$NR^{69A}COR^{70A}$, -$SO_2R^{71A}$, -$SO_2NR^{72A}R^{73A}$, -$NR^{74A}SO_2R^{75A}$, -$NR^{76A}COOR^{77A}$, $Cyc2^A$, or the like;

$R^{20A}$ and $R^{64A}$-$R^{77A}$ each independently represents hydrogen, C1-8 alkyl, $Cyc2^A$, or the like;

$Cyc2^A$ represents a C5-6 mono-cyclic carbon ring or the like (with the proviso that, the carbon ring or the like may be substituted by 1 to 5 substituent(s) such as C1-8 alkoxy, halogen or the like) ;

$G^A$ and $J^A$ each independently represents a carbon, nitrogen, oxygen, or sulfur atom;

$E^A$ represents C1-4 alkylene, -O-, -S-, or the like (with the proviso that, the C1-4 alkylene may be substituted by 1 to 5 substituent(s) such as C1-8 alkoxy, halogen, hydroxy, or the like) ;

$B^A$ represents a C5-10 mono- or poly-cyclic carbon ring, or a 5- to 10- membered mono- or poly-cyclic hetero ring containing I to 4 nitrogen atom(s), 1 to 2 oxygen atom(s) and/or 1 sulfur atom ;

$R^{3A}$ represents C1-8 alkyl, C2-8 alkenyl, C2-8 alkynyl, halogen, -$OR^{81A}$, -$NR^{82A}R^{83A}$, -$NR^{84A}COR^{85A}$, -$CONR^{86A}R^{87A}$, -$COOR^{88A}$, -$SO_2NR^{89A}R^{90A}$, -$NR^{91A}SO_2R^{92A}$, -$SR^{93A}$,-$S(O)R^{94A}$, -$SO_2R^{95A}$, -$NR^{96A}COOR^{97A}$, -$NR^{98A}CONR^{99A}R^{100A}$, -$OCONR^{101A}R^{102A}$, nitro, cyano, trifluoromethyl, trifluoromethoxy, $Cyc4^A$, or the like ;

$R^{81A}$-$R^{102A}$ each independently represents hydrogen, C1-8 alkyl, $Cyc4^A$, or the like ;

$Cyc4^A$ represents a C5-10 mono- or poly-cyclic carbon ring or the like (with the proviso that, the carbon ring or the like may be substituted by 1 to 5 substituent(s) such as C1-8 alkoxy, halogen or the like) ;

mA represents 0 or an integer of 1 to 5 ;

nA represents 0 or an integer of 1 to 7 ;

iA represents 0 or an integer of 1 to 12, with the proviso that, only necessary part of the meanings of the symbols in the formula were excerpted.

Also, DE10002509 discloses that the compounds represented by general formula (X) are useful as IL-12 inhibitors :

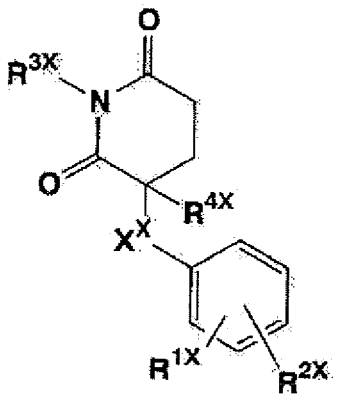

(X)

wherein
$R^{1X}$ and $R^{2X}$ each represents hydrogen, chlorine, fluorine, hydroxy, or the like ;
$R^{3X}$ represents hydrogen, hydroxy, or $-CH_2NR^{6X}R^{7X}$ ($R^{6X}$ and $R^{7X}$ are taken together to form pyrrolidine, piperidine, morpholine, or the like) ;
$R^{4X}$ represents hydrogen, C1-3 alkyl, fluorine, trifluoromethyl, or difluoromethyl ;
$X^X$ represents $-(CH_2)_{nX}-NR^{8X}-$, $-(CH_2)_{nx}-S-$, $-(CH_2)_{qx}-$, or the like, with the proviso that, only necessary part of the meanings of the symbols in the formula were excerpted.
Further, WO 00/043384 discloses that the compounds represented by general formula (Y) are useful as anti-inflammatory agents :

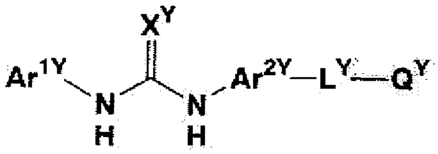

(Y)

wherein
$Ar^{1Y}$ represents an optionally substituted hetero ring or the like ;
$Ar^{2Y}$ represents an optionally substituted phenyl group or the like;
$L^Y$ represents C1-10 alkylene or the like ;
$Q^Y$ represents phenyl or the like which may be substituted by halogen or the like ;
$X^Y$ represents oxygen or sulfur, with the proviso that, only necessary part of the meanings of the symbols in the formula were excerpted.

Also, WO 03/076405 discloses that the compounds represented by general formula (Z) are useful as p38 MAP kinase inhibitors :

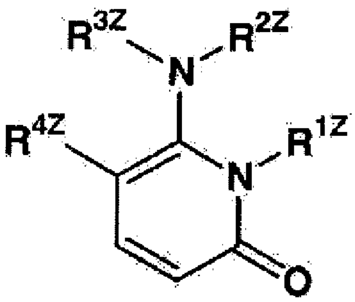

(Z)

wherein
$R^{1Z}$ represents hydrogen, C1-8 alkyl, C6-10 aryl, heteroaryl, C3-8 cycloalkyl, or hetero ring (wherein C1-8 alkyl, C6-10 aryl, heteroaryl C3-8 cycloalkyl or hetero ring may be substituted by 0 to 3 $R^{1-1Z}$(S));

$R^{2Z}$ represents hydrogen, amino, mono- or di-C1-6 alkylamino, C3-8 cycloalkylamino, C6-10 arylamino, C1-8 alkyl, C6-10 aryl, heteroaryl, C3-8 cycloalkyl, or hetero ring (wherein mono- or di-C1-6 alkylamino, C3-8 cycloalkylamino, C6-10 arylamino, C1-8 alkyl, C6-10 aryl, heteroaryl, C3-8 cycloalkyl, or hetero ring may be substituted by 0 to 3 $R^{2-1Z}$(s));
$R^{3Z}$ represents hydrogen or C1-6 alkyl ;
$R^{4Z}$ represents -$COR^{4-1Z}$ (wherein $R^{4-1Z}$ represents C6-10 aryl or heteroaryl) ; with the proviso that, $R^{1Z}$, $R^{2Z}$ and $R^{3Z}$ do not represent hydroxy at the same time, with the proviso that, only necessary part of the meanings of the symbols in the formula were excerpted.

Moreover, WO 01/096308 discloses that the compounds represented by general formula (W), the salts thereof, or the hydrate thereof have an inhibitory effect on AMPA receptor and/or kainic acid receptor :

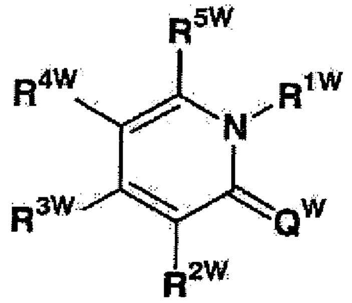

(W)

wherein
$Q^w$ represents NH, O or S;
$R^{1W}$, $R^{2W}$, $R^{3W}$, $R^{4W}$ and $R^{5W}$ represent, samely or differently, hydrogen, halogen, C1-6 alkyl, or -$X^W$-$A^W$ (wherein $X^W$ represents bond, an optionally substituted C1-6 alkylene, an optionally substituted C2-6 alkenylene, an optionally substituted C2-6 alkynylene, -O-, -S-, -CO-, -SO-, -$SO_2$-, -N($R^{6W}$)-, -N($R^{7W}$)-CO-, -CO-N($R^{8W}$)-, -N($R^{9W}$)-$CH_2$-, -$CH_2$-N($R^{10W}$)-, - $CH_2$-CO-, -CO-$CH_2$-, -N($R^{11W}$)-$S(O)_{mW}$-, -$S(O)_{nW}$-N($R^{12W}$)-, -$CH_2$-$S(O)_{pW}$-, -$S(O)_{qW}$-$CH_2$-, - $CH_2$-O-, -O-$CH_2$-, -N($R^{13W}$)-CO-N($R^{14W}$)-, or -N($R^{15W}$)-CS-N($R^{16W}$)- (wherein $R^{6W}$, $R^{7W}$, $R^{8W}$, $R^{9W}$ $R^{10W}$, $R^{11W}$, $R^{12W}$, $R^{13W}$, $R^{14W}$, $R^{15W}$ and $R^{16W}$ represent hydrogen, C1-6 alkyl or C1-6 alkoxy; mW, nW, pW and qW each independently represents 0 or an integer of 1 or 2); $A^W$ represents, C3-8 cycloalkyl, C3-8 cycloalkenyl, non-aromatic 5- to 14- membered hetero ring, aromatic C6-14 hydrocarbon ring or aromatic 5- to 14- membered hetero ring, and these rings are optionally substituted by substituent respectively);
with the proviso that, three of $R^{1W}$, $R^{2W}$, $R^{3W}$, $R^{4W}$ and $R^{5W}$ samely or differently represent -$X^w$-$A^w$ and residual two always represent hydrogen, halogen, or C1-6 alklyl;
provided that in the above-mentioned definition, the cases where (1) Q is O; $R^{1W}$ and $R^{5W}$ are hydrogen; and $R^{2W}$, $R^{3W}$ and $R^{4W}$ are phenyl groups, (2) Q is O; $R^{1W}$ and $R^{4w}$ are hydrogen; and $R^{2W}$, $R^{3W}$ and $R^{5W}$ are phenyl groups, and (3) Q is O; $R^{1W}$ and $R^{2W}$ are hydrogen; and $R^{3W}$, $R^{4W}$ and $R^{5W}$ are phenyl groups, are excluded.

DISCLOSURE OF THE INVENTION

Problem that the Invention is to Solve

It is earnestly desired to develop p38 MAP kinase inhibitors useful for prevention and/or treatment of various diseases typically such as inflammatory diseases, which are excellent in oral absorption, and can be safely administered.

Means for Solving the Problem

As a result of the present inventors intensively studied to find compounds which are useful as agents for treatment of various diseases typically such as inflammatory diseases by suppressing the activity of p38 MAP kinase as the subject, they found that the object was accomplished by novel nitrogen-containing heterocyclic compounds represented by the following formula (I), and thus the present invention has been completed.

Thus, the present invention relates to the followings.

(1) A compound represented by the formula (I):

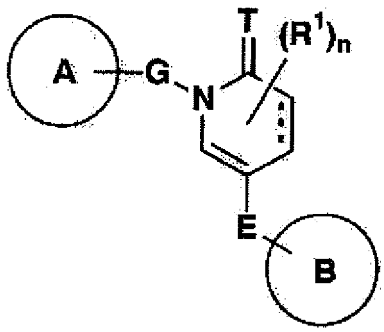

(I)

wherein ring A and ring B each independently represent an cyclic group which may be substituted;
E represents a spacer having 1 to 4 atoms in the main chain thereof;
G represents a bond, an oxygen atom, a sulfur atom which may be oxidized or a methylene group which may be substituted;
$R^1$ represents a substituent;
T represents an oxygen atom or a sulfur atom;
represents a single bond or a double bond;
n represents 0 or an integer of 1 to 5, and
wherein when n represents 2 or more, each of $R^1$'s are the same or different, a salt thereof, an N-oxide thereof, a solvate thereof, or a prodrug thereof.
(2) The compound according to (1), wherein ring A is a benzene ring which may be substituted.
(3) The compound according to (1), wherein ring B is a benzene ring which may be substituted.
(4) The compound according to (3), wherein ring B is substituted by a fluorine atom.
(5) The compound according to (1), wherein E is C1-4 alkylene, C1-4 alkylene substituted by hydroxy, C1-4 alkylene substituted by oxo, C1-4 alkylene substituted by C1-4 alkylene, C1-4 alkylene substituted by C1-4 alkoxy, -S-, -C(=N-$NH_2$)- or -C(=N-$OR^2$)- in which $R^2$ represents a hydrogen atom or a substituent.
(6) The compound according to (1), wherein E is -C(=N-$OR^2$)- in which $R^2$ has the same meaning as $R^2$ defined in (5).
(7) The compound according to (1), wherein is a double bond.
(8) The compound according to (1), wherein T is an oxygen atom.
(9) The compound according to (1), wherein G is a bond.
(10) The compound according to (1), which is represented by the formula (Ia-2) or the formula (Ia-3):

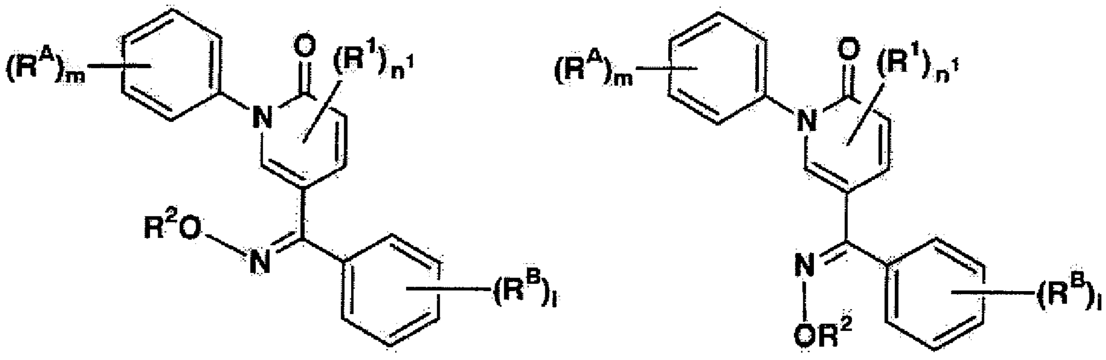

(Ia-2) (Ia-3)

wherein $R^A$ and $R^B$ each independently represent a substituent;
m and i each independently represents 0 or an integer of 1 to 5, wherein when m represents 2 or more, each of $R^A$'s are the same or different, and when i represents 2 or more, each of $R^B$'s are the same or different;
$n^1$ represents 0 or an integer of 1 to 3, wherein when $n^1$ represents 2 or more, each of $R^1$'s are the same or different; and $R^1$ and $R^2$ have the same meanings as $R^1$ and $R^2$ defined in (1) and (5), or a mixture of the compound represented by the formula (Ia-2) and the compound represented by the formula (Ia-3) in an arbitrary ratio.
(11) The compound according to (10), which is represented by the formula (Ia-3):

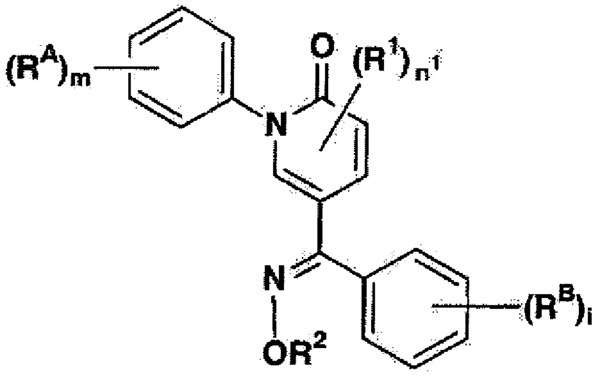

(Ia-3)

wherein $R^1$, $R^2$, $R^A$, $R^B$, m, i and $n^1$ have the same meanings as $R^1$, $R^2$, $R^A$, $R^B$, m, i and $n^1$ defined in (1), (5) and (10).

(12) The compound according to (10) or (11), wherein $R^2$ is

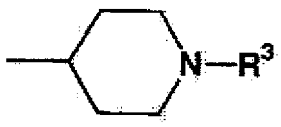

in which $R^3$ represents a hydrogen atom or a substituent.

(13) The compound according to (12), wherein $R^3$ is a hydrogen atom, methyl, ethyl, or dimethylaminoethyl.

(14) The compound according to (10) to (13),
wherein $R^A$ is methyl, ethyl, methoxy, or a halogen atom; and
m is an integer of 1 to 5, and
wherein when m is 2 or more, each of $R^A$'s are the same or different.

(15) The compound according to (10) to (13),
wherein $R^B$ is methyl or a halogen atom; and
i is an integer of 1 to 5, and
wherein when i is 2 or more, each of $R^B$'s are the same or different.

(16) The compound according to (1), which is
5-{(2,4-difluorophenyl){[(1-methyl-4-piperidinyl)oxy]imino}methyl)-1-(2,6-dimethylphenyl)-2(1H)-pyridinone,
5-{(2,4-difluorophenyl)[({1-[2-(dimethylamino)ethyl]-4-piperidinyl}oxy)imino]methyl}-1-(2,6-dimethylphenyl)-2(1H)-pyridinone,
5-{(Z)-(2,4-difluorophenyl)[(4-piperidinyloxy)imino]methyl}-1-(4-fluoro-2,6-dimethylphenyl)-2(1H)-pyridinone,
5-((Z)-(2,4-difluorophenyl){[(1-methyl-4-piperidinyl)oxy]imino}methyl)-1-(4-fluoro-2,6-dimethylphenyl)-2(1H)-pyridinone,
5-((Z)-(2,4-difluorophenyl){[(1-ethyl-4-piperidinyl)oxy]imino}methyl)-1-(4-fluoro-2,6-dimethylphenyl)-2(1H)-pyridinone,
5-((E)-(2,4-difluorophenyl){[(1-methyl-4-piperidinyl)oxy]imino}methyl)-1-(4-fluoro-2,6-dimethylphenyl)-2(1H)-pyridinone,
1-(2,6-difluorophenyl)-5-{(2,4-difluorophenyl)[(4-piperidinyloxy)imino]methyl}-2(1H)-pyridinone,
5-{(2,4-difluorophenyl)[(4-piperidinyloxy)imino]methyl}-1-(2-methoxy-6-methylphenyl)-2(1H)-pyridinone,
1-(2,6-diethylphenyl)-5-{(2,4-difluorophenyl)[(4-piperidinyloxy)imino]methyl}- 2(1H)-pyridinone,
1-(2-chloro-6-methylphenyl)-5-{(2,4-difluorophenyl)[(4-piperidinyloxy)imino]methyl}-2(1H)-pyridinone,
5-{(2,4-difluorophenyl)[(4-piperidinyloxy)imino]methyl}-1-(2-ethyl-6-methylphenyl)-2(1H)-pyridinone,
5-{(2,4-difluorophenyl)[(4-piperidinyloxy)imino]methyl}-1-(2,6-dimethoxyphenyl)-2(1H)-pyridinone,
1-(2,6-difluorophenyl)-5-((Z)-(2,4-difluorophenyl){[(1-methyl-4-piperidinyl)oxy]imino}methyl)-2(1H)-pyridinone,
1-(2,6-difluorophenyl)-5-((E)-(2,4-difluorophenyl){[(1-methyl-4-piperidinyl)oxy]imino} methyl)-2(1H)-pyridinone,
5-((2,4-difluorophenyl) {[(1-methyl-4-pipefidinyl)oxy]imino}methyl)-1-(2-methoxy-6-methylphenyl)-2(1H)-pyridinone,
1-(2,6-diethylphenyl)-5-((2,4-difluorophenyl){[(1-methyl-4-piperidinyl)oxy]imino}methyl)-2(1H)-pyridinone,
1-(2-chloro-6-methylphenyl)-5-((2,4-difluorophenyl){[(1-methyl-4-piperidinyl)oxy]imino}methyl)-2(1H)-pyridinone,
5-((2,4-difluorophenyl){[(1-methyl-4-piperidinyl)oxy]imino}methyl)-1-(2-ethyl-6-methylphenyl)-2(1H)-pyridinone,
5-((2,4-difluorophenyl){[(1-methyl-4-piperidinyl)oxy]imino}methyl)-1-(2,6-dimethoxyphenyl)-2(1H)-pyridinone,

5-((2,4-difluorophenyl){[(1-methyl-4-piperidinyl)oxy]imino}methyl)-1-methyl-2(1H)-pyridinone,
5-((E)-(2,4-difluorophenyl){[(1-ethyl-4-piperidinyl)oxy]imino}methyl)-1-(2,6-dimethylphenyl)-2(1H)-pyridinone,
5-((Z)-(2,4-difluorophenyl){[(1-ethyl-4-piperidinyl)oxy]imino}methyl)-1-(2,6-dimethylphenyl)-2(1H)-pyridinone,
5-{(2,4-difluorophenyl)[(4-piperidinylmethoxy)imino]methyl}-1-(2,6-dimethylphenyl)-2(1H)-pyridinone,
5-((2,4-difluorophenyl){[(1-methyl-4-piperidinyl)methoxy]imino}methyl)-1-(2,6-dimethylphenyl)-2(1H)-pyridinone,
1-(2,6-dichlorophenyl)-5-{(2-fluoro-4-methylpheny)[(4-piperidinyloxy)imino]methyl}-2(1H)-pyridinone, or
1-(2,6-dichlorophenyl)-5-{(4-fluoro-2-methylphenyl)[(4-piperidinyloxy)imino]methyl}-2(1H)-pyridinone.

(17) The compound according to (1), which is
5-((2,4-difluorophenyl) {[(1-methyl-4-piperidinyl)oxy]imino}methyl)-1-(2,6-dimethylphenyl)-2(1H)-pyridinone,
5-((Z)-(2,4-difluorophenyl){[(1-methyl-4-piperidinyl)oxy]imono}methyl-1-(4-fluoro-2,6-dimethylphenyl)-2(1H)-pyridinone,
1-(2,6-difluorophenyl)-5-((Z)-(2,4-difluorophenyl){[(1-methyl-4-piperidinyl)oxy]imino}methyl)-2(1H)-pyridinone,
5-((2,4-difluorophenyl){[(1-methyl-4-piperidinyl)oxy]imino}methyl)-1-mesityl-2(1H)-pyridinone, or
5-((Z)-(2,4-difluorophenyl){[(1-ethyl-4-pipendinyi)oxy]immo]methyl)-1-(2,6-dimethylphenyl)-2(1H)-pyridinone.

(18) A compound represented by the formula (I-R):

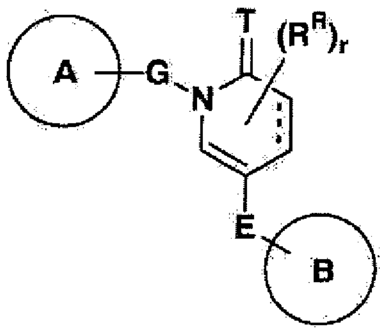

(I-R)

wherein $R^R$ represents a substituent; and
r represents an integer of 2 to 5,
wherein each of $R^R$'s are the same or different, and other symbols have the same meanings as defined in (1), and wherein at least two of $R^R$'s are taken together with a carbon atom which bound them to represent a cyclic group which may be substituted, a salt thereof, an N-oxide thereof, a solvate thereof, or a prodrug thereof.

(19) A pharmaceutical composition comprising the compound represented by the formula (I) according to (1), a salt thereof, an N-oxide thereof, a solvate thereof, or a prodrug thereof.

(20) The composition according to (19), which is a p38 MAP kinase inhibitor and/or a TNF-$\alpha$ production inhibitor.

(21) The composition according to (19), which is an agent for the prevention and/or treatment of cytokine-mediated diseases.

(22) The composition according to (21), wherein the cytokine-mediated disease is an inflammatory disease, a cardiovascular disease, a respiratory disease and/or a bone disease.

(23) The composition according to (22), wherein the inflammatory disease is rheumatoid arthritis.

(24) A pharmaceutical composition comprising the compound represented by the formula (I) according to (1), a salt thereof, an N-oxide thereof, a solvate thereof, or a prodrug thereof; in conbination with one or two or more kinds selected from a non-steroidal anti-inflammatory agent, a disease modifying anti-rheumatic drug, an anticytokine protein preparation, a cytokine inhibitor, an immunomodulator, a steroidal agent, an adhesion molecule inhibitor, an elastase inhhibitor, a cannabinoid-2 receptor stimulant, a prostaglandin, a prostaglandin synthase inhibitor, a phosphodiesterase inhibitor and a metalloproteinase inhibitor.

(25) A method for the inhibition of p38 MAP kinase and/or the inhibition of TNF-$\alpha$ production in mammal, which comprises administering to a mammal an effective amount of the compound represented by the formula (I) according to (1), a salt thereof, an N-oxide thereof, a solvate thereof, or a prodrug thereof.

(26) A method for the prevention and/or treatment of cytokine-mediated diseases in mammal, which comprises administering to a mammal an effective amount of the compound represented by the formula (I) according to (1), a salt thereof, an N-oxide thereof, a solvate thereof, or a prodrug thereof.

(27) Use of the compound represented by the formula (I) according to (1), or a salt thereof, an N-oxide thereof, a solvate thereof, or a prodrug thereof for the manufacture of an agent for the inhibition of p38 MAP kinase and/or the inhibition of TNF-$\alpha$ production.

(28) Use of the compound represented by the formula (I) according to (1), or a salt thereof an N-oxide thereof, a solvate thereof, or a prodrug thereof for the manufacture of an agent for the prevention and/or treatment of a cytokine-mediated disease.

(29) A pharmaceutical composition comprising the compound represented by the formula (I-R) according to (18), a salt thereof , an N-oxide thereof, a solvate thereof, or a prodrug thereof

(30) The composition according to (29), which is a p38 MAP kinase inhibitor and/or a TNF-α production inhibitor.

(31) The composition according to (29), which is an agent for the prevention and/or treatment of cytokine-mediated diseases.

(32) The composition according to (31), wherein the cytokine-mediated disease is an inflammatory disease, a cardiovascular disease, a respiratory disease and/or a bone disease.

(33) The composition according to (32), wherein the inflammatory disease is rheumatoid arthritis.

(34) A pharmaceutical composition comprising the compound represented by the formula (I-R) according to (18), or a salt thereof, an N-oxide thereof, a solvate thereof, or a prodrug thereof; in conbination with one or two or more kinds selected from a non-steroidal anti-inflammatory agent, a disease modifying anti-rheumatic drug, an anticytokine protein preparation, a cytokine inhibitor, an immunomodulator, a steroidal agent, an adhesion molecule inhibitor, an elastase inhhibitor, a cannabinoid-2 receptor stimulant, a prostaglandin, a prostaglandin synthase inhibitor, a phosphodiesterase inhibitor and a metalloproteinase inhibitor.

(35) A method for the inhibition of p38 MAP kinase and/or the inhibition of TNF-α production in mammal, which comprises administering to a mammal an effective amount of the compound represented by the formula (I-R) according to (18), a salt therof, an N-oxide thereof, a solvate thereof, or a prodrug thereof

(36) A method for the prevention and/or treatment of cytokine-mediated diseases in mammal, which comprises administering to a mammal an effective amount of the compound represented by the formula (I-R) according to (18), a salt therof, an N-oxide thereof, a solvate thereof, or a prodrug thereof

(37) Use of the compound represented by the formula (I-R) according to (18), a salt thereof, an N-oxide thereof, a solvate thereof, or a prodrug thereof for the manufacture of an agent for the inhibition of p38 MAP kinase and/or the inhibition of TNF-α production.

(38) Use of the compound represented by the formula (I-R) according to (18), a salt thereof, an N-oxide thereof, a solvate thereof, or a prodrug thereof for the manufacture of an agent for prevention and/or treatment of a cytokine-mediated disease.

(39) A process for production of the compound represented by the formula (I) according to (1) or the formula (I-R) according to (18), a salt thereof, an N-oxide thereof, a solvate thereof, or a prodrug thereof

Effect of the Invention

The compounds of the present invention have p38 MAP kinase inhibitory activity and are low toxicity, so they are useful as agents for treatment of cytokine-mediated diseases such as inflammatory diseases, central nervous system disorders, respiratory diseases, cardiovascular diseases, urinary diseases, metabolic diseases, endocrine diseases, bone diseases, cancerous diseases, infectious diseases, and so forth.

BEST MODE FOR CARRYING OUT THE INVENTION

In the description of the present invention, the "cyclic group" in the "optionally substituted cyclic group" represented by ring A or ring B includes, for example, a carbon ring, a hetero ring, and so forth. Said "carbon ring" only has to be a carbon ring, and there is no particular limitation for the number of atoms that constitute said "carbon ring". As preferable carbon ring, for example, a "C5-10 mono- or poly-cyclic carbon ring" and so forth can be cited. It includes, for example, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, teterahydronaphthalene, perhydronaphthalene ring, and so forth. Also, said "C5-10 mono- or poly-cyclic carbon ring" includes, a spiro-fused poly-cyclic carbon ring and a bridged poly-cyclic carbon ring, too. It includes, for example, spiro[4.4]nonane, spiro[4.5]decane, bicyclo[2.2.1]heptane, bicyclo[2.2.1]hept-2-ene, bicyclo[3.1.1]heptane, bicyclo [3.1.1]hept-2-ene, bicyclo[2.2.2]octane, bicyclo[2.2.2]oct-2-ene, adamantane, noradamantane ring, and so forth.

Said "hetero ring" only has to be a hetero ring, and there is no particular limitation for the number of atoms that constitute said "hetero ring". As preferable hetero ring, for example, a "5- to 10- membered mono- or poly-cyclic hetero ring" and so forth can be cited. As said "5- to 10- membered mono- or poly-cyclic hetero ring", a "5- to 10-membered mono-or poly-cyclic hetero ring containing 1 to 5 nitrogen atom(s), 1 to 2 oxygen atom(s) and/or 1 sulfur atom, a spiro-fused poly-cyclic hetero ring, and a bridged poly-cyclic hetero ring" and so forth can be cited. It includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazan, benzothiadiazole, benzotriazole, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxolane, dioxane, dioxaindan, benzodioxane, chroman, azaspiro[4.4]nonane, oxazaspiro[4.4]nonane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azabicyclo[2.2.1]heptane, oxabicyclo[2.2.1]heptane, azabicyclo[3.1.1]heptane, azabicyclo[3.2.1]octane, oxabicyclo[3.2.1]octane, azabicyclo[2.2.2] octane, diazabicyclo[2.2.2]octane ring, and so forth. Among these, as a "5-to 10-membered mono- or poly-cyclic aromatic hetero ring containing 1 to 4 nitrogen atom(s), 1 to 2 oxygen atom(s) and/or 1 sulfur atom", for example, pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthyridine, quinoxaline, quinazoline, cinnoline, benzoxazole, benzothiazole, benzimidazole, benzofurazan, benzothiadiazole, benzotriazole ring and so forth can be cited.

In the description of the present invention, there is no particular limitation for the "substituent" in the "optionally substituted cyclic group" represented by ring A or ring B so long as it can be a substituent. Said "substituent" includes, for example, (1) an optionally substituted aliphatic hydrocarbon group, (2) a substitient selected from the Group I shown below, (3) an optionally substituted C5-10 carbon ring, (4) an optionally substituted 5- to 10-membered hetero ring, or the like. One to twelve substituent(s), preferably one to three substituent(s) among these optional substituents may be located any position where substitution is possible.

There is no particular limitation for the "substituent" in the "optionally substituted C5-10 carbon ring" or the "optionally substituted 5- to 10-membered hetero ring " so long as it can be a substituent. Said "substituent" includes, for example, (1) a substitient selected from the Group I shown below, (2) an optionally substituted 5- to 6-membered cyclic group shown below, or the like. One to five substituent(s) among these optional substituents may be located any position where substitution is possible.

The "aliphatic hydrocarbon group" in the "optionally substituted aliphatic hydrocarbon group" includes, for example, a "straight or branched C1-8 aliphatic hydrocarbon group", and so forth. Said "straight or branched C1-8 aliphatic hydrocarbon group" includes, for example, C1-8 alkyl (e.g. methyl, ethyl, propyl, isopropyl, butyl, sec-butyl, tert-butyl, pentyl, hexyl, heptyl, octyl, *etc.),* C2-8 alkenyl (*e.g.* vinyl, propenyl, butenyl, pentenyl, hexenyl, heptenyl, octenyl, butadienyl, pentadienyl, hexadienyl, heptadienyl, octadienyl, hexatrienyl, heptatrienyl, octatrienyl, *etc.*), C2-8 alkynyl (*e.g.* ethynyl, propynyl, butynyl, pentynyl, hexynyl, heptynyl, octynyl, butadiynyl, pentadiynyl, hexadiynyl, heptadiynyl, octadiynyl, hexatriynyl, heptatriynyl, octatriynyl, *etc*), and so forth.

There is no particular limitation for the "substituent" in the "optionally substituted aliphatic hydrocarbon group" so long as it can be a substituent. Said "substituent" includes, for example, (1) a substitient selected from the Group I shown below, (2) an optionally substituted C5-10 carbon ring, (3) an optionally substituted 5- to 10-membered hetero ring, or the like. One to five substituent(s) among these optional substituents may be located any position where substitution is possible. Here, there is no particular limitation for the "substituent" in the "optionally substituted C5-10 carbon ring" or the "optionally substituted 5- to 10-membered hetero ring" so long as it can be a substituent. Said "substituent" includes, for example, (1) a substitient selected from the Group I shown below, (2) an optionally substituted 5- to 6-membered cyclic group shown below, or the like. One to five substituent(s) among these optional substituents may be located any position where substitution is possible.

<Group I>

(a) halogen atom (e.g. chlorine, bromine, fluorine, iodine atom), (b) -$OR^{a1}$, (c) -$NR^{a1}R^{a2}$, (d) - $NR^{a1}COR^{a2}$,(e) -$CONR^{a1}R^{a2}$, (f) -$COOR^{a1}$, (g) -$SO_2NR^{a1}R^{a2}$, (h) -$NR^{a1}SO_2R^{a2}$, (i) -$SR^{a1}$, (j) - $S(O)R^{a1}$, (k) -$SO_2R^{a1}$, (1) -$NR^{a1}COOR^{a2}$, (m) -$NR^{a1}CONR^{a2}R^{a3}$, (n) -$COR^{a1}$, (o) nitro, (p) cyano, (q) trifluoromethyl, (r) trifluoromethoxy, (s) -$OCONR^{a1}R^{a2}$, (t) -$CONR^{a1}NR^{a2}R^{a3}$ and (u) oxo [in these groups, $R^{a1}$, If and $R^{a3}$ each independently represents a hydrogen atom, an optionally substituted C1-8 alkyl, an optionally substituted C5-10 carbon ring, or an optionally substituted 5- to 10-membered hetero ring]. Here, the "C1-8 alkyl" in the "optionally substituted C1-8 alkyl" represented by $R^{a1}$, $R^{a2}$ and $R^{a3}$ has the same meaning as defined above. Also, there is no particular limitation for the "substituent" in the "optionally substituted C1-8 alkyl", the "optionally substituted C5-10 carbon ring" and the "optionally substituted 5- to 10-membered hetero ring" so long as it can be a substituent. Said "substituent" includes, for example, (1) a substitient selected from the Group II shown below, (2) an optionally substituted C5-10 carbon ring, (3) an optionally substituted 5- to 10-membered hetero ring, or the like. One to five substituent(s) among these optional substituents may be located any position where substitution is possible. There is no particular limitation for the "substituent" in the "optionally substituted C5-10 carbon ring" or the "optionally substituted 5- to 10-membered hetero ring" so long as it can be a substituent. Said "substituent" includes, for example, (1) a substitient selected from the Group II shown below, (2) an optionally substituted 5- to 6-membered cyclic group shown below, or the like. One to five substituent(s) among these optional substituents may be located any position where substitution is possible.

<Group II>

(a) -$OR^{b1}$, (b) -$NR^{b1}R^{b2}$, (c) -$NR^{b1}COR^{b2}$, (d) -$CONR^{b1}R^{b2}$, (e) -$COOR^{b1}$, (f) -$SO_2NR^{b1}R^{b2}$, (g) -$NR^{b1}SO_2R^{b2}$, (h) -$CONR^{b1}NR^{b2}R^{b3}$ and (i) -$CONR^{b1}OR^{b2}$ [in these groups, $R^{b1}$, $R^{b2}$ and $R^{b3}$ each independently represents a hydrogen atom, an optionally substituted C1-8 alkyl, an optionally substituted C5-10 carbon ring, or an optionally substituted 5- to 10-membered hetero ring]. Here, the "C1-8 alkyl" in the "optionally substituted C1-8 alkyl" represented by $R^{b1}$, $R^{b2}$ and $R^{b3}$ has the same meaning as defined above. Also, there is no particular limitation for the "substituent" in the "optionally substituted C1-8 alkyl" so long as it can be a substituent. Said "substituent" includes, for example, (1) a substitient selected from the Group III shown below, (2) an optionally substituted C5-10 carbon ring, (3) an optionally substituted 5- to 10-membered hetero ring, or the like. One to five substituent(s) among these optional substituents may be located any position where substitution is possible. There is no particular limitation for the "substituent" in the "optionally substituted C5-10 carbon ring" or the "optionally substituted 5- to 10-membered hetero ring" so long as it can be a substituent. Said "substituent" includes, for example, (1) a substitient selected from the Group III shown below, (2) an optionally substituted 5- to 6-membered cyclic group shown below, or the like. One to five substituent(s) among these optional substituents may be located any position where substitution is possible.

<Group III>

(a) -$OR^{c1}$ and (b) -$NR^{c1}R^{c2}$ [in these groups, $R^{c1}$ and $R^{c2}$ each independently represents a hydrogen atom, an optionally substituted C1-8 alkyl, an optionally substituted C5-10 carbon ring, or an optionally substituted 5- to 10-membered hetero ring]. Here, the "C1-8 alkyl" in the "optionally substituted C1-8 alkyl" represented by $R^{c1}$ and $R^{c2}$ has the same meaning as defined above. Also, there is no particular limitation for the "substituent" in the "optionally substituted C1-8 alkyl" so long as it can be a substituent. Said "substituent" includes, for example, (1) an optionally substituted C5-10 carbon ring, (2) an optionally substituted 5- to 10-membered hetero ring, or the like. One to five substituent(s) among these optional substituents may be located any position where substitution is possible. There is no particular limitation for the "substituent" in the "optionally substituted C5-10 carbon ring" or the "optionally substituted 5- to 10-membered hetero ring" so long as it can be a substituent. Said "substituent" includes, for example, (1) a substitient selected from the Group IV shown below, (2) an optionally substituted 5- to 6-membered cyclic group shown below, or the like. One to five substituent(s) among these optional substituents may be located any position where substitution is possible.

The "C5-10 carbon ring" in the "optionally substituted C5-10 carbon ring" in the "substituent" of ring A or ring B has the same meaning as the "C5-10 mono- or poly-cyclic carbon ring" defined above. Also, the "5- to 10-membered hetero ring" in the "optionally substituted 5- to 10-membered hetero ring" has the same meaning as the "5- to 10-membered mono- or poly-cyclic hetero ring" defined above.

<Group IV>

(a) C1-8 alkyl (having the same meaning as define above), (b) halogen atom (having the same meaning as defined above), (c) nitro, (d) cyano, (e) -$OR^{d1}$, (f) -$NR^{d1}R^{d2}$, (g) -$COOR^{d1}$, (h) - $COR^{d1}$, (i) -$CONR^{d1}R^{d2}$, (j) -$NR^{d1}COR^{d2}$, (k) -$SO_2NR^{d1}R^{d2}$, (1) -$NR^{d1}SO_2R^{d2}$, (m) -$SR^{d1}$, (n) - $SO_2R^{d1}$, (o) oxo, and (p) thioxo [in these groups, $R^{d1}$ and $R^{d2}$ each independently represents a hydrogen atom or a C1-8 alkyl (having the same meaning as defined above)].

The "5- to 6-membered cyclic group" in the "optionally substituted 5- to 6-membered cyclic group" in the "substituent" of ring A or ring B includes, for example, a "C5-6 mono-cyclic carbon ring", a"5- to 6-membered mono-cyclic hetero ring", or the like. Said "C5-6 mono-cyclic carbon ring" includes, for example, cyclopentane, cyclohexane, cyclopentene, cyclohexene, cyclopentadiene, cyclohexadiene, benzene ring or the like. On the other hand, as the "5- to 6-membered mono-cyclic hetero ring", for example, a "5- to 6-membered mono-cyclic hetero ring containing 1 to 4 nitrogen atom(s), 1 to 2 oxygen atom(s) and/or 1 sulfur atom", and so forth can be cited. Said "5- to 6-membered mono-cyclic hetero ring containing 1 to 4 nitrogen atom(s), 1 to 2 oxygen atom(s) and/or 1 sulfur atom" includes, for example, pyrrole, imidazole, triazole, tetrazole, pyrazole, pyridine, pyrazine, pyrimidine, pyridazine, furan, pyran, thiophene, thiopyran, oxazole, isoxazole, thiazole, isothiazole, furazan, oxadiazole, oxazine, oxadiazine, thiadiazole, thiazine, thiadiazine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrothiadiazole, tetrahydrothiadiazole (thiadiazolidine), dihydrothiazine, tetrahydrothiazine, dihydrothiadiazine, tetrahydrothiadiazine, morpholine, thiomorpholine, oxathiane, dioxolane, dioxane ring or the like. Here, as the "5- to 6-membered cyclic group" in the "optionally substituted 5- to 6-membered cyclic group", for example, the "C5-6 mono-cyclic carbon ring", the "5- to 6-membered mono-cyclic hetero ring", or the like can be cited preferably. More preferably, for example, the "C5-6 mono-cyclic carbon ring" and so forth can be cited, and most preferably, for example, benzene ring and so forth can be cited.

There is no particular limitation for the "substituent" in the "optionally substituted 5-to 6-membered cyclic group" in the "substituent" of ring A or ring B so long as it can be a substituent. Said "substituent" includes, for example, (1) C1-8 alkyl (having the same meaning as define above), (2) C1-8 alkoxy (e.g. methoxy, ethoxy, propoxy, isopropoxy, butoxy, sec-butoxy, tert-butoxy, pentyloxy, hexyloxy, heptyloxy, octyloxy, *etc.*), (3) halogen atom (having the same meaning as defined above), (4) trifluoromethyl, (5) trifluoromethoxy, or the like. One to five substituent(s) among these optional substituents may be located any position where substitution is possible.

The "substituent" in the "optionally substituted benzene ring" has the same meaning as the "substituent" in the "optionally substituted cyclic group" represented by ring A or ring B defined above.

In the description of the present invention, the "substituent" represented by $R^A$ and $R^B$ has the same meaning as the "substituent" in the "optionally substituted cyclic group" represented by ring A or ring B defined above.

In the description of the present invention, there is no particular limitation for the "substituent" represented by $R^1$ so long as it can be a substituent. Said "substituent" includes, for example, (1) an optionally substituted C1-8 alkyl, (2) an optionally substituted 5-to 6-membered cyclic group defined above, or (3) a substitient selected from the Group V shown below. Said "substituent" includes, for example, "an optionally substituted 5- to 6-membered cyclic ring", (2) a substitient selected from the Group V shown below. One to five substituent(s) among these optional substituents may be located any position where substitution is possible.

<Group V>

(a) -$OR^{e1}$, (b) -$NR^{e1}R^{e2}$, (c) -$COOR^{e1}$, (d) -$CONR^{e1}R^{e2}$, (e) -$NR^{e1}COR^{e2}$, (f) -$SO_2R^{e1}$, (g) - $SO_2NR^{e1}R^{e2}$, (h) -$NR^{e1}SO_2R^{e2}$ and (i) -$NR^{e1}COOR^{e2}$ [wherein $R^{e1}$ and $R^{e2}$ each independently represents a hydrogen atom or an optionally substituted C1-8 alkyl group]. Here, the "C1-8 alkyl" in the "optionally substituted C1-8 alkyl" represented by $R^{e1}$ and $R^{e2}$ has the same meaning as defined above. Also, there is no particular limitation for the "substituent" in the "optionally substituted C1-8 alkyl" represented by $R^{e1}$ and $R^{e2}$ so long as it can be a substituent. Said "substituent" includes, for example, the "optionally substituted 5- to 6-membered cyclic group" and so forth. One to five substituent (s) among these optional substituents may be located any position where substitution is possible.

In the description of the present invention, the "substituent" represented by $R^R$ has the same meaning as the "substituent" represented by $R^1$ defined above.

In the description of the present invention, the "optionally substituted cyclic group" formed by two of $R^R$ and a carbon atom which bound them has the same meaning as the "optionally substituted cyclic group" represented by ring

A or ring B.

In the description of the present invention, the "spacer having 1 to 4 atom(s) in its main chain" represented by E means a space where 1 to 4 atom(s) of the main chain are stood in a row. In this case, the "number of atoms in its main chain" is counted in such a manner that the number of atoms in its main chain become minimum. Said "spacer having 1 to 4 atom(s) in its main chain" includes, for example, an optionally substituted C1-4 alkylene, an optionally substituted C2-4 alkenylene, an optionally substituted C2-4 alkynylene, or the like. Here, as the "C1-4 alkylene", for example, $-CH_2-$, $-(CH_2)_2-$, $-(CH_2)_3-$, $-(CH_2)_4-$, or the like can be cited. As the "C2-4 alkenylene", for example, -CH=CH-, $-CH_2-CH=CH-$, $-CH=CH-CH_2-$, $-(CH_2)_2-CH=CH-$, $-CH=CH-(CH_2)_2-$, $-CH_2-CH=CH-CH_2-$, or the like can be cited. As the "C2-4 alkynylene", for example, -C≡C-, -CH2-C≡C-, $-C≡C-CH_2-$, $-(CH_2)_2-C≡C-$, $-C=C-(CH_2)_2-$, $-CH_2-C≡C-CH_2-$, or the like can be cited. Further, any carbon atom in the C1-4 alkylene, C2-4 alkenylene, and C2-4 alkynylene defined above may be replaced by oxygen, sulfur, or optionally substituted nitrogen atom. Here, there is no particular limitation for the "substituent" in the "optionally substituted nitrogen atom" so long as it can be a substituent. Said "substituent" includes, for example, an optionally substituted C1-8 alkyl, and so forth. Said "C1-8 alkyl" in the "optionally substituted C1-8 alkyl" has the same meaning as defined above. Also, there is no particular limitation for the "substituent" in the "optionally substituted C1-8 alkyl" so long as it can be a substituent. Said "substituent" includes, for example, (1) hydroxy, (2) the "optionally substituted 5- to 6-membered cyclic group" defined above or (3) an optionally protected amino (wherein the "optionally protected amino" means amino whose 1 or 2 hydrogen atom(s) are optionally substituted by arbitrary substituent. Said "substituent" includes,for example, (1) the "optionally substituted cyclic group" defined above", (2) the "optionally substituted aliphatic hydrocarbon group" defined above, or the like.) and one to five substituent(s) among these optional substituents may be located any position where substitution is possible.

There is no particular limitation for the "substituent" in the "optionally substituted C1-4 alkylene", the "optionally substituted C2-4 alkenylene" and the "optionally substituted C2-4 alkynylene" as the "spacer having 1 to 4 atom(s) in its main chain" represented by E so long as it can be a substituent. Said "substituent" includes, for example, (1) C1-8 alkyl (having the same meaning as defined above), (2) C1-8 alkoxy (having the same meaning as defined above), (3) halogen atom (having the same meaning as defined above), (4) hydroxy, (5) oxo, (6) thioxo, (7) amino, (8) =N-CN, (9) $=N-OR^2$ [in these groups, $R^2$ is a hydrogen atom or substituent, and the "substituent" represented by $R^2$ has the same meaning as the "optionally substituted cyclic group" defined above.], (10) $=CR^4R^5$ [$R^4$ and $R^5$ each independently represents a hydrogen atom, "C1-8 alkyl" optionally substituted by the "optionally protected amino" (having the same meaning as defined above) or C1-8 alkoxy (having the same meaning as defined above), or $R^4$ and $R^5$ are taken together with a carbon atom which bound them to represent an optionally substituted cyclohexane ring or an optionally substituted piperidine ring. There is no particular limitation for the "substituent" in the "optionally substituted cyclohexane ring" or the "optionally substituted piperidine ring" so long as it can be a substituent. Said "substituent" includes, for example, (a) the "optionally substituted 5- to 6-membered cyclic group" defined above or a (b) C1-8 alkyl optionally substituted by the "optionally substituted 5- to 6-membered cyclic group" defined above], (11) $=N-NR^6R^7$ [in these groups, $R^6$ and $R^7$ each independently represents a hydrogen atom or an optionally substituted C1-8 alkyl (wherein the "C1-8 alkyl" has the same meaning as defined above and there is no particular limitation for the "substituent". Said "substituent" has the same meaning as, for example, the "substituent" in the "optionally substituted nitrogen atom" defined above.)], or the like. One to five substituent(s) among these optional substituents may be located any position where substitution is possible.

In the description of the present invention, there is no particular limitation for the "substituent" represented by $R^3$. Said "substituent" includes, for example, (1) a substitient selected from the Group I defined above, (2) the "optionally substituted 5-to 6-membered cyclic group" defined above, or the like. One to five substituent(s) among these optional substituents may be located any position where substitution is possible.

In the description of the present invention, the "substituent" in the "optionally substituted methylene group" represented by G has the same meaning as the "substituent" in the "optionally substituted C1-4 alkylene" defined above.

In the present invention, any rings, any groups and any atoms represented by ring A; ring B; $R^A$, $R^B$, $R^1$, $R^R$, E; $R^2$, $R^3$, G, and T are all preferable. Especially, embodiments as described in Examples are preferred. Hereinafter, preferable groups, preferable rings and preferable atoms are listed, and all symbols as used herein have the same meanings as those defined above.

In the present invention, preferable example of the "cyclic group" in the "optionally substituted cyclic group" represented by ring A includes, for example, the "c5-10 mono- or poly-cyclic carbon ring", the "5- to 10-membered mono- or poly-cyclic hetero ring", and so forth. More preferable example includes, for example, the "C5-6 mono-cyclic aromatic carbon ring", the "5- to 6-membered mono-cyclic aromatic hetero ring containing 1 to 2 nitrogen atom(s), 1 oxygen atom and/or 1 sulfur atom", or the like, and most preferable example includes, for example, benzene, pyridine, thiophene ring, or the like. Further, as the preferable example of the "substituent" in the "optionally substituted cyclic group" represented by ring A, for example, the "optionally substituted 5- to 10-membered hetero ring", C1-8 alkyl, halogen atom, $-NR^{a1}R^{a2}$, $-NR^{a1}COR^{a2}$, $-COOR^{a2}$, $-CONR^{a1}R^{a2}$, $-COR^{a1}$,$-SO_2NR^{a1}R^{a2}$, $-NR^{a1}SO_2R^{a2}$, C1-4 alkyl substituted by $-OR^{a1}$, or the like can be cited. More preferable example include, for example, C1-4 alkyl, halogen atom, $-CONR^{a1}R^{a2}$,

$-NR^{a1}R^{a2}$,$-NR^{a1}COR^{a2}$, C1-4 alkyl substituted by $-OR^{a1}$, or the like, and most preferable example includes, for example, methyl, ethyl, fluorine atom, chlorine atom, bromine atom, or the like.

In the present invention, preferable example of the "cyclic group" in the "optionally substituted cyclic group" represented by ring B includes, for example, the "c5-10 mono- or poly-cyclic carbon ring", the "5- to 10-membered mono- or poly-cyclic hetero ring", and so forth. More preferable example includes, for example, the "C5-10 mono- or poly-cyclic aromatic carbon ring", the "5- to 10-membered mono- or poly-cyclic aromatic hetero ring", or the like, and most preferable example includes, for example, benzene, naphthalene ring, or the like. Further, as the preferable example of the "substituent" in the "optionally substituted cyclic group" represented by ring B, for example, C1-8 alkyl, halogen atom, $-OR^{a1}$, $-NR^{a1}COR^{a2}$, $-CONR^{a1}R^{a2}$, $-NR^{a1}COOR^{a2}$, $-NR^{a1}CONR^{a2}R^{a3}$, $-OCONR^{a1}R^{a2}$, C1-8 alkyl substituted by $-NR^{a1}COR^{a2}$, C1-8 alkyl substituted by $-CONR^{a1}R^{a2}$, C1-8 alkyl substituted by $-NR^{a1}COOR^{a2}$, C1-8 alkyl substituted by $-NR^{a1}CONR^{a2}R^{a3}$, C1-8 alkyl substituted by $-OCONR^{a1}R^{a2}$, or the like can be cited. More preferable example include, for example, C1-4 alkyl, halogen atom, methoxy, $-NR^{a1}CONR^{a2}R^{a3}$, $-OCONR^{a1}R^{a2}$, C1-4 alkyl substituted by $-NR^{a1}CONR^{a2}R^{a3}$, C1-4 alkyl substituted by $-OCONR^{a1}R^{a2}$, C1-4 alkyl substituted by $-CONR^{a1}R^{a2}$, or the like, and most preferable example includes, for example, methyl, ethyl, fluorine atom, chlorine atom, methoxy, $-OCONR^{a1}R^{a2}$, $-CH_2-CONR^{a1}R^{a2}$, $-CH_2-NR^{a1}CONR^{a2}R^{a3}$, $-CH_2-OCONR^{a1}R^{a2}$, or the like.

In the present invention, preferable example of $R^A$ includes, for example, C1-8 alkyl, halogen atom, $-OR^{a1}$, $-NR^{a1}R^{a2}$, $-NR^{a1}COR^{a2}$, $-COOR^{a2}$, $-CONR^{a1}R^{a2}$, $-COR^{a1}$, $-SO_2NR^{a1}R^{a2}$, $-NR^{a1}SO_2R^{a2}$, C1-4 alkyl substituted by $-OR^{a1}$, or the like. More preferable example includes, for example, C1-4 alkyl,halogen atom, methoxy, $-CONR^{a1}R^{a2}$, $-NR^{a1}R^{a2}$,$-NR^{a1}COR^{a2}$, C1-4 alkyl substituted by $-OR^{a1}$, or the like, and most preferable example includes, for example, methyl, ethyl, fluorine atom, chlorine atom, bromine atom, methoxy, or the like.

In the present invention, preferable example of $R^B$ includes, for example, C1-8 alkyl, halogen atom, $-OR^{a1}$, $-NR^{a1}COR^{a2}$, $-CONR^{a1}R^{a2}$, $-NR^{a1}COOR^{a2}$, $-NR^{a1}CONR^{a2}R^{a3}$, $-OCONR^{a1}R^{a2}$, C1-8 alkyl substituted by $-NR^{a1}COR^{a2}$, C1-8 alkyl substituted by $-CONR^{a1}R^{a2}$, C1-8 alkyl substituted by $-NR^{a1}COOR^{a2}$, C1-8 alkyl substituted by $-NR^{a1}CONR^{a2}R^{a3}$, C1-8 alkyl substituted by $-OCONR^{a1}R^{a2}$ or the like. More preferable example includes, for example, C1-4 alkyl, halogen atom, $-NR^{a1}CONR^{a2}R^{a3}$, $-OCONR^{a1}R^{a2}$, C1-4 alkyl substituted by $-NR^{a1}CONR^{a2}R^{a3}$, $C_{1-4}$ alkyl substituted by $-OCONR^{a1}R^{a2}$, $C_{1-4}$ alkyl substituted by-$CONR^{a1}R^{a2}$, or the like, and most preferable example includes, for example, methyl, fluorine atom, chlorine atom, $-OCONR^{a1}R^{a2}$, $-CH_2-CONR^{a1}R^{a2}$, $-CH_2-NR^{a1}CONR^{a2}R^{a3}$, $-CH_2-OCONR^{a1}R^{a2}$, or the like, and especially preferable example includes methyl, fluorine atom or chlorine atom.

In the present invention, preferable example of $R^1$ includes, for example, Cl-8 alkyl, $-OR^{e1}$, $-COOR^{e1}$, C1-4 alkyl substituted by the optionally substituted 5- to 6-membered cyclic group, or the like. More preferable example includes, for example, C1-4 alkyl, $-OR^{e1}$, $-COOR^{e1}$, or the like, and most preferable example includes, for example, methyl, ethyl, -OH, $-OCH_3$, -COOH, $-COOCH_3$, or the like.

In the present invention, preferable example of the "cyclic group" in the "optionally substituted cyclic group" formed by two of $R^R$ and a carbon atom which bound them includes, for example, the "C5-10 mono- or poly-cyclic carbon ring", the "mono- or poly-cyclic hetero ring", or the like. More preferable example includes, for example, cyclopentane, cyclohexane, cycloheptane, cyclopentene, cyclohexene, cycloheptene, cyclopentadiene, cyclohexadiene, cycloheptadiene, benzene, pyrrole, imidazole, pyrazole, pyridine, pyrazine, pyrimidine, pyridazine, furan, thiophene, oxazole, isoxazole, thiazole, isothiazole ring, or the like. Further, as the preferable example of

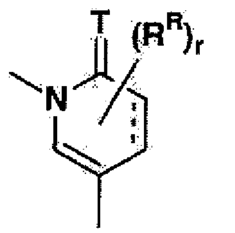

(wherein all symbols have the same meanings as defned above, with the proviso that, at least two of $R^R$ are taken together with a carbon atom which bound them to represent an optionally substituted cyclic group) in the gereral formula (I-R), for example,

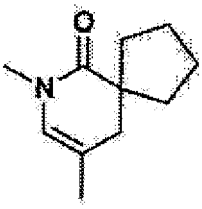 , 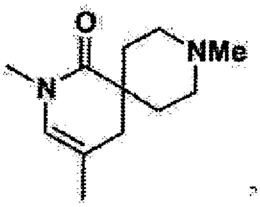 , 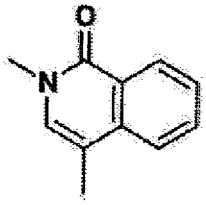 or 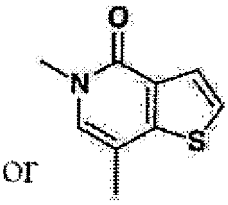

, or the like can be cited.

In the present invention, preferable example of the "spacer having 1 to 4 atom(s) in its main chain" includes, for example, C1-4 alkylene, C1-4 alkylene substituted by hydroxy, C1-4 alkylene substituted by oxo, C1-4 alkylene substituted by C1-4 alkyl, C1-4 alkylene substituted by C1-4 alkoxy, -S-, $-C(=N-NH_2)-$, $-C(=N-OR^2)-$, or the like. More preferable example includes, for example, methylene, hydroxymethylene, methoxymethylene, carbonyl, $-C(=N-OR^2)-$, or the like, and most preferable example includes, for example, $-C(=N-OR^2)-$, or the like.

In the present invention, preferable example of $R^2$ includes, for example, hydrogen atom, C1-8 alkyl, C1-8 alkyl substituted by $-OR^{a1}$, the optionally substituted cyclic group, C1-8 alkyl substituted by the optionally substituted cyclic group, or the like. More preferable example includes, for example, hydrogen atom, C1-4 alkyl substituted by $-OR^{a1}$, the optionally substituted cyclic group, C1-4 alkyl substituted by the optionally substituted cyclic group, or the like, and most preferable example includes, for example, the optionally substituted cyclic group, or the like. Especially preferable example includes, for example, the optionally substituted piperidine ring, or the like.

In the present invention, preferable example of $R^3$ includes, for example, hydrogen atom, C1-8 alkyl, C1-8 alkyl substituted by $-OR^{a1}$, C1-8 alkyl substituted by $-NR^{a1}R^{a2}$, or the like. More preferable example includes, for example, hydrogen atom, C1-8 alkyl, dimethylaminoethyl, or the like, and most preferable example includes, for example, hydrogen atom, methyl, ethyl, isopropyl, tert-butyl, dimethylaminoethyl, or the like.

In the present invention, preferable example of G includes the optionally substituted methylene or a bond, and more preferable example includes a bond.

In the present invention, preferable example of T includes oxygen atom.

In the present invention, preferable example of n includes 0 or an integer of 1 to 2.

In the present invention, preferable example of n1 includes 0 or an integer of 1, and more preferable example includes 0.

In the present invention, preferable example of r includes an integer of 2.

In the present invention, preferable example of m includes 0 or an integer of 1 to 3, and more preferable example includes an integer of 2 or 3.

In the present invention, preferable example of i includes 0 or an integer of 1 to 3, and more preferable example includes an integer of 2.

In the present invention, a compound represented by general formula (I) comprising a combination of preferable groups, preferable rings, and preferable atoms as defined above, or its salt, N-oxide or solvate, or a prodrug thereof is preferable.

More preferably, a compound represented by general formula (I-1):

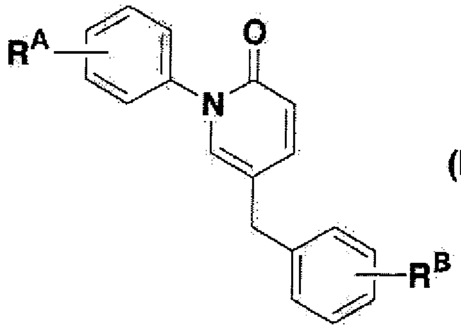

(I-1)

(wherein $R^A$ and $R^B$ have the same meanings as the "substituent" in the "optionally substituted cyclic group" defined above.) , general formula (I-2):

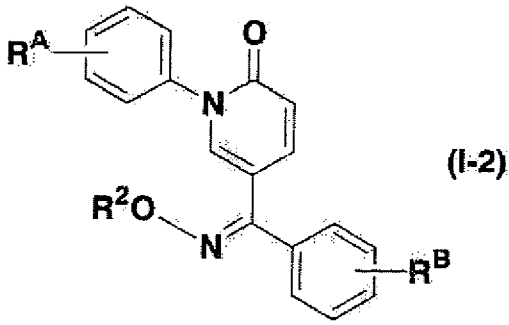

(I-2)

(wherein all symbols have the same meanings as defined above.) , general formula (1-3):

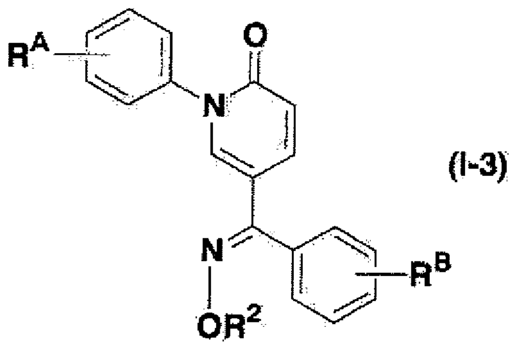

(I-3)

(wherein all symbols have the same meanings as defined above.) general formula (1-4).

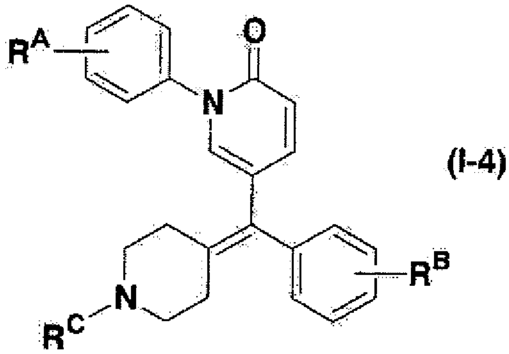

(I-4)

(wherein $R^C$ has the same meaning as the "substituent" in the "optionally substituted piperidine ring" formed by $R^4$ and $R^5$ together with a carbon atom which bound them defined above, $R^A$ and $R^B$ have the same meanings as defined above.) , general formula (I-1a):

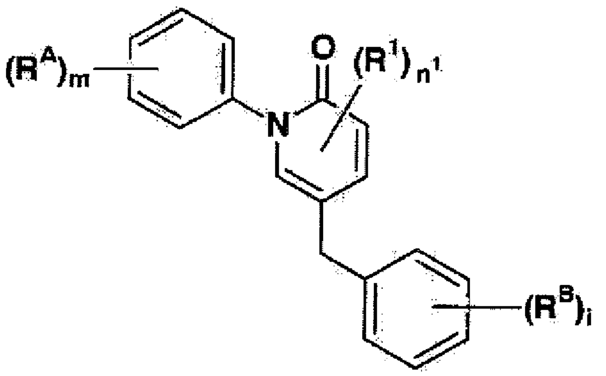

(I-1a)

(wherein all symbols have the same meanings as defined above.) , general formula (Ia):

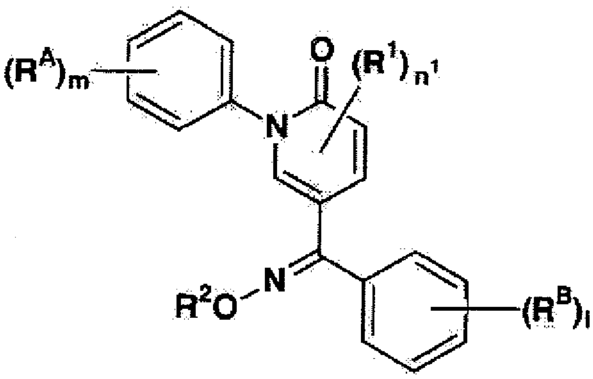

(Ia)

(wherein all symbols have the same meanings as defined above.) , general formula (Ia-2):

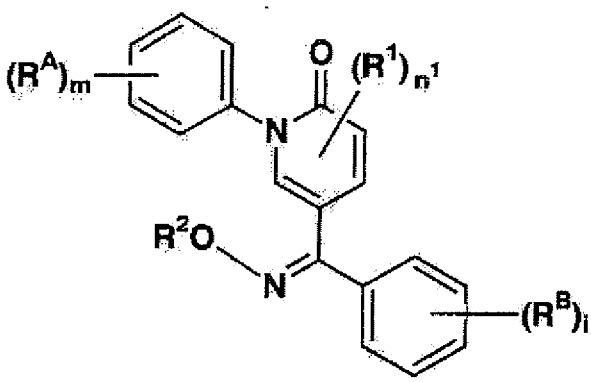

(Ia-2)

(wherein all symbols have the same meanings as defined above.) , general formula (Ia-3):

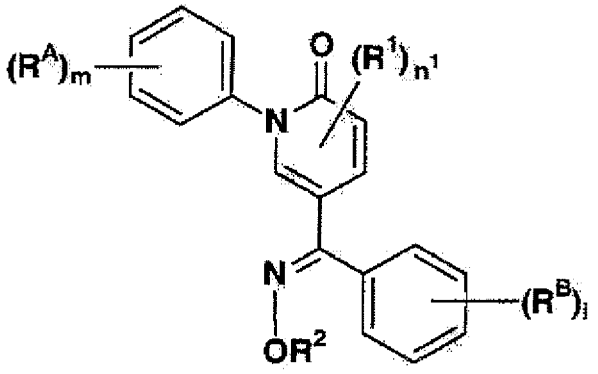

(Ia-3)

(wherein all symbols have the same meanings as defined above.) , general formula (Ib):

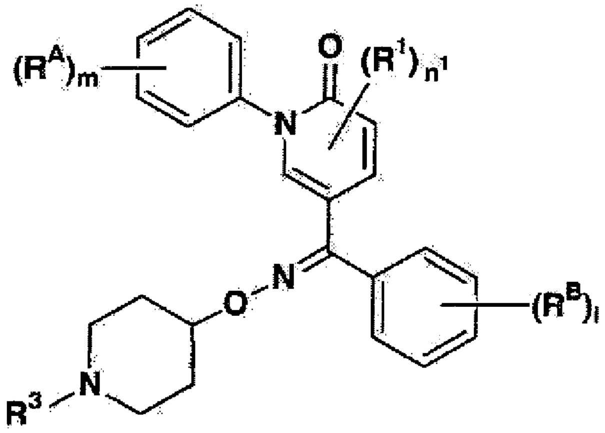

(Ib)

(wherein all symbols have the same meanings as defined above.) , general formula (Ib-2):

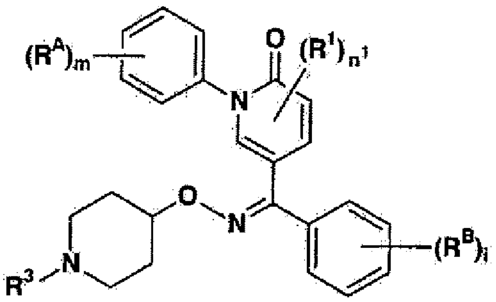

(Ib-2)

(wherein all symbols have the same meanings as defined above.) , general formula (Ib-3):

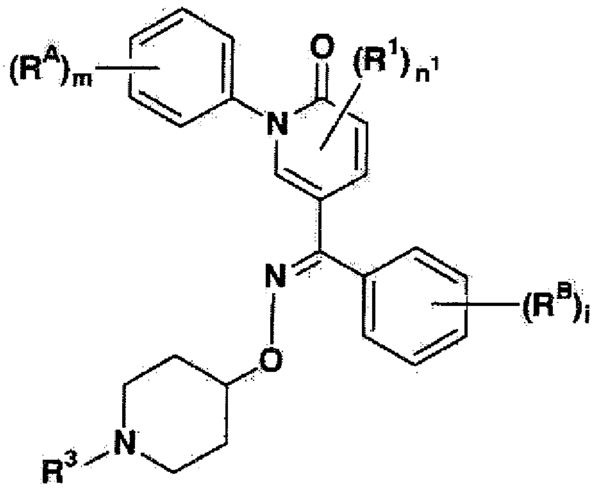

(Ib-3)

(wherein all symbols have the same meanings as defined above.) , general formula (Ib-2-1):

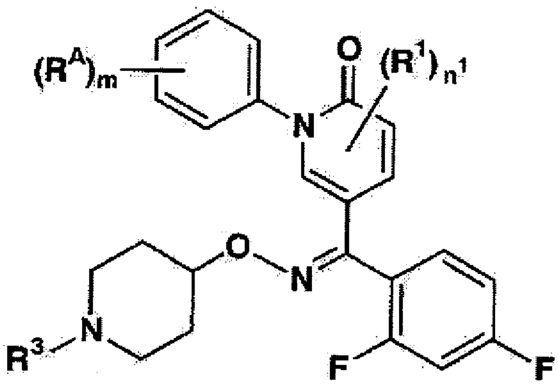

(Ib-2-1)

(wherein all symbols have the same meanings as defined above.) , or general formula (Ib-3-1):

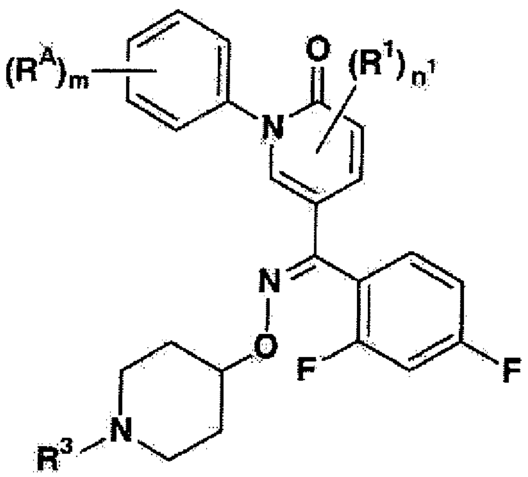

(Ib-3-1)

(wherein all symbols have the same meanings as defined above.)
, or its salt, N-oxide or solvate, or a prodrug thereof can be cited. Among these, for example, a compound represented by the general formula (I-2), (I-3), (Ia), (Ia-2), (Ia-3), (Ib), (Ib-2), (Ib-3), (Ib-2-1), or (Tb-3-1) defined above, or its salt, N-oxide or solvate, or a prodrug thereof can be cited preferably. Most preferably, for example, a compound represented by the general formula (I-3), (Ia-3), (Ib-3), or (Ib-3-1) defined above, or its salt, N-oxide or solvate, or a prodrug thereof

can be cited.

Also, in the present invention, all compounds described in the Example are preferable. Especially, 5-((2,4-difluorophenyl){[(1-methyl-4-piperidinyl)oxy]imino}methyl)-1-(2,6-dimethylphenyl)-2(1H)-pyridinone, 5-{(2,4-difluorophenyl)[({1-[2-(dimethylamino)ethyl]-4-piperidinyl}oxy)imino]methyl}-1-(2,6-dimethylphenyl)-2(1H)-pyridinone, 5-{(Z)-(2,4-difluorophenyl)[(4-piperidinyloxy)imino]methyl}-1-(4-fluoro-2,6-dimethylphenyl)-2(1H)-pyridinone, 5-((Z)-(2,4-difluorophenyl){[(1-methyl-4-piperidinyl)oxy]imino}methyl)-1-(4-fluoro-2,6-dimethylphenyl)-2(1H)-pyridinone, 5-((Z)-(2,4-difluorophenyl){[(1-ethyl-4-piperidinyl)oxy]imino}methyl)-1-(4-fluoro-2,6-dimethylphenyl)-2(1H)-pyridinone, 5-((E)-(2,4-difluorophenyl){[(1-methyl-4-piperidinyl)oxy]imino}methyl}-1-(4-fluoro-2,6-dimethylphenyl)-2(1H)-pyridinone, 1-(2,6-difluorophenyl)-5-{(2,4-difluorophenyl)[(4-piperidinyloxy)imino]methyl}-2(1H)-pyridinone, 5-{(2,4-difluorophenyl)[(4-piperidinyloxy)imino]methyl}-1-(2-methoxy-6-methylphenyl)-2(1H)-pyridinone, 1-(2,6-diethylphenyl)-5-{(2,4-difluorophenyl)[(4-piperidinyloxy)imino]methyl}-2(1H)-pyridinone, 1-(2-chloro-6-methylphenyl)-5-{(2,4-difluorophenyl)[(4-piperidinyloxy)imino]methyl}-2(1H)-pyridinone, 5-{(2,4-difluorophenyl}[(4-piperidinyloxy)imino]methyl}-1-(2-ethyl-6-methylphenyl)-2(1H)-pyridinone, 5-{(2,4-difluorophenyl)[(4-piperidinyloxy)imino]methyl}-1-(2,6-dimethoxyphenyl)-2(1H)-pyridinone, 1-(2,6-difluorophenyl)-5-((Z)-(2,4-difluorophenyl){[(1-methyl-4-piperidinyl)oxy]imino}methyl)-2(1H)-pyridinone, 1-(2,6-difluorophenyl)-5-((E)-(2,4-difluorophenyl){[(1-methyl-4-piperidinyl)oxy]imino}methyl)-2(1H)-pyridinone, 5-((2,4-difluorophenyl){[(1-methyl-4-piperidinyl)oxy]imino}methyl)-1-(2-methoxy-6-methylphenyl)-2(1H)-pyridinone, 1-(2,6-diethylphenyl)-5-((2,4-difluorophenyl){[(1-methyl-4-piperidinyl)oxy]imino}methyl)-2(1H)-pyridinone, 1-(2-chloro-6-methylphenyl)-5-((2,4-difluorophenyl) {[(1-methyl-4-piperidinyl)oxy]imino}methyl)-2(1H)-pyridinone, 5-((2,4-difluorophenyl){[(1-methyl-4-piperidinyl)oxy]imino}methyl)-1-(2-ethyl-6-methylphenyl)-2(1H)-pyridinone, 5-((2,4-difluorophenyl){[(1-methyl-4-piperidinyl)oxy]imino}methyl)-1-(2,6-dimethoxyphenyl)-2(1H)-pyridinone, 5-((2,4-difluorophenyl){[(1-methyl-4-piperidinyl)oxy]imino}methyl)-1-mesityl-2(1H)-pyridinone, 5-((E)-(2,4-difluorophenyl) {[(1-ethyl-4-piperidinyl)oxy]imino}methyl)-1-(2,6-dimethylphenyl)-2(1H)-pyridinone, 5-((Z)-(2,4-difluorophenyl){[(1-ethyl-4-piperidinyl)oxy]imino}methyl)-1-(2,6-dimethylphenyl)-2(1H)-pyridinone, 5-{(2,4-difluorophenyl)[(4-piperidinylmethoxy)imino]methyl}-1-(2,6-dimethylphenyl)-2(1H)-pyridinone, 5-((2,4-difluorophenyl) {[(1-methyl-4-piperidinyl)methoxy]imino}methyl)-1-(2,6-dimethylphenyl)-2(1H)-pyridinone, 1-(2,6-dichlorophenyl)-5-{(2-fluoro-4-methylphenyl)[(4-piperidinyloxy)imino]methyl}-2(1H)-pyridinone or 1-(2,6-dichlorophenyl)-5-{(4-fluoro-2-methylphenyl)[(4-piperidinyloxy)imino]methyl}-2(1H)-pyridinone is preferable, and among of these, 5-((2,4-difluorophenyl){[(1-methyl-4-piperidinyl)oxy]immo}methyl)-1-(2,6-dimethylphenyl)-2(1H)-pyridinone, 5-((Z)-(2,4-difluorophenyl){[(1-methyl-4-piperidinyl)oxy]imino}methyl)-1-(4-fluoro-2,6-dimethylphenyl)-2(1H)-pyridinone, 1-(2,6-difluorophenyl)-5-((Z)-(2,4-difluorophenyl){[(1-methyl-4-piperidinyl)oxy]imino}methyl)-2(1H)-pyridinone, 5-((2,4-difluorophenyl){[(1-methyl-4-piperidinyl)oxy]imino}methyl)-1-mesityl-2(1H)-pyridinone or 5-((Z)-(2,4-difluorophenyl){ [(1-ethyl-4-piperidinyl)oxy]imino}methyl)-1-(2,6-dimethylphenyl)-2(1H)-pyridinone is more preferable.

Unless otherwise specified, any isomers are all included in the present invention. For example, as structural isomer, linear or branched ones are included in the alkyl, alkoxy, and alkylene groups. Further, the present invention includes isomers due to double bond, ring, and fused ring (E-form, Z-form, cis-form, trans-form), isomers due to the presence of asymmetric carbon atom (R-form, S-form, α-form, β-form, enantiomer, diastereomer), optically active compounds with optical rotation (D-form, L-form, d-form, l-form), compounds with axial asymmetry (atropisomer), polar compounds obtained by chromatographic separation (high polar compound, low polar compound), equilibrium compounds, and mixtures of these compounds in an arbitrary ratio (for example, about from 95:5 to 55:45 (mass ratio)), and racemates. Moreover, the present invention includes all tautomers.

Salt, N-oxide, solvate and prodrug

Pharmacologically acceptable salts are all included in the salts of compounds represented by general formula (I). The pharmacologically acceptable salts are preferably those which are non-toxic and soluble in water. Examples of suitable salts are salts of alkali metals (e.g. potassium, sodium, lithium, *etc.*), salts of alkaline earth metals (e.g. calcium, magnesium, *etc.*), ammonium salts (*e.g.* tetramethylammonium salt, tetrabutylammonium salt, *etc.*), salts of organic amines (*e.g.* triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc.*), acid addition salts [inorganic acid salts (e.g. hydrochloride, hydrobromide, hydroiodide, sulfate, phosphate, nitrate, *etc.*), organic acid salts (*e.g.* acetate, trifluoroacetate, lactate, tartarate, oxalate, fumarate, maleate, benzoate, citrate, methanesulfonate, ethanesulfonate, benzenesulfonate, toluenesulfonate, isethionate, glucronate, gluconate, *etc.), etc.*].

Further, such salts include quaternary ammonium salts. The quaternary ammonium salts can be those wherein the nitrogen atom in the compound represented by general formula (I) is quaternized by $R^0$ group. Examples of $R^0$ are a C1-8 alkyl group, and a phenyl-substituted C1-8 alkyl group.

The N-oxides of compounds represented by general formula (I) are ones wherein the nitrogen atom of the compound represented by general formula (I) is oxidized. Also, the N-oxides of the present invention may be present in the form of alkaline (earth) metal salts, ammonium salts, organic amine salts or acid addition salts.

Suitable solvates of compounds represented by general formula (I) includes, for example, a solvate with water or an alcoholic solvent (*e.g.* ethanol, *etc.).* The solvates are preferably non-toxic and soluble in water. The solvates of the present invention includes solvates of alkaline (earth) metal salts, ammnoium salts, organic amine salts, acid addition salts or N-oxides of the compounds of the present invention represented by general formula (1),

The compounds of the present invention may be converted into the above salts, the above N-oxides, or the above solvates by the known method.

The prodrugs of the compounds represented by general formula (I) are those which can be converted into the compounds of the general formula (I) of the present invention by the *in vivo* action of enzymes or gastric acid. Examples of the prodrugs of compounds represented by general formula (I) are (1) those wherein the amino group is acylated, alkylated, or phosphorylated (for example, compounds wherein the amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, t-butylated, etc.), when compounds represented by general formula (I) contain an amino group; (2) those wherein the hydroxy group is acylated, alkylated, phosphorylated, or borated (for example, compounds wherein the hydroxy group is acetylated, palmitoylated, propanoylated, pivaloylated, succinylated, fumarylated, alanylated, dimethylaminocabonylated, *etc.),* when compounds represented by general formula (I) contain a hydroxy group; and (3) those wherein the carboxyl group is esterified, or amidated (for example, compounds wherein the carboxyl group is converted into an ester such as ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, and cyclohexyloxycarbonylethyl ester, or compounds wherein the carboxyl group is methylamidated). These compounds can be prepared by the conventional method. The prodrug of the compound represented by general formula (I) is any one of hydrates and non-hydrates. The prodrug of the compound represented by general formula (I) is a compound which may be converted into a compound represented by general formula (I) under physiological conditions as described in Development of Drugs, Molecule Design, vol.7, pp.163-198, (1990), published by Hirokawa Publishing Co., Japan. Further, the compound represented by general formula (I) may be labelled with an isotope (*e.g.* $^{3}$H, $^{14}$C, $^{35}$S, $^{125}$I, *etc.*).

Mechanical IUPAC nomenclature of the compounds of the present invention was performed using a computer program ACD/NAME (Trade Name) available from Advanced Chemistry Development Co. For example, the following compound was named 1-(2,6-dichlorophenyl)-5-(2,4-difluorobenzoyl)pyridin-2(1H)-one.

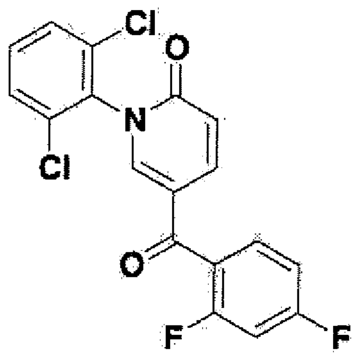

Process for preparation of the compounds of the present invention

The compounds represented by general formula (I) can be prepared by the known method, for example, an appropriately improved or combined method of Methods (A) to (F) shown below, similar methods thereof, the method as described in Examples, and the method described in Comprehensive Organic Transformations: A Guide to Functional Group Preparations, 2nd Edition (Richard C. Larock, John Wiley & Sons Inc., 1999). The starting material in each preparation method shown below may be used in the form of a salt. Such salt used is the salt of compounds represented by general formula (I) as defined above.

(A) Among the compounds represented by general formula (I) of the present invention, a compound wherein T represents O, and  represents a single bond, that is, a compound represented by general formula (II-A):

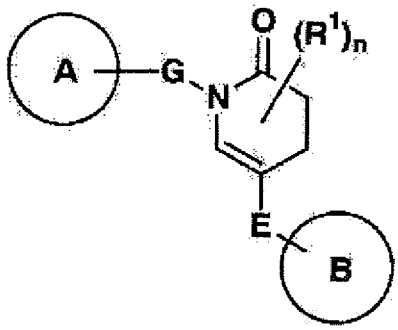

(II-A)

(wherein all symbols have the same meanings as defined above.) can be prepared by subjecting a compound represented by general formula (III-A):

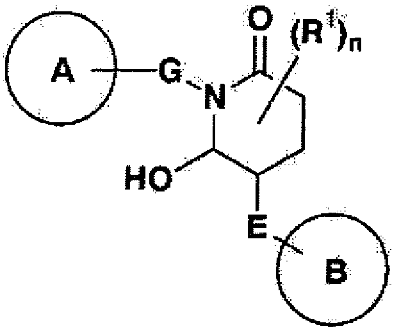

(III-A)

(wherein all symbols have the same meanings as defined above.) to a dehydration reaction.

The dehydration reaction is well known *per se.* For example, it may be carried out by reacting a compound represented by general formula (III-A) with base (*e.g.* an inorganic base such as a hydride of alkaline metal or alkaline earth metal (e.g. sodium hydride, potassium hydride, *etc.*), a hydroxide of alkaline metal or alkaline earth metal (e.g. sodium hydroxide, potassium hydroxide, *etc.*), an alkyllithium (e.g. n-butyllithium, sec-butyllithium, tert-butyllithium, *etc.*), an alkoxide of alkaline metal (e.g. sodium methoxide, sodium ethoxide, *etc.),* an alkaline metal (e.g. sodium metal, potassium metal, *etc.) etc.,* an organic base such as an alkylamine (e.g. riethylamine, tributylamine, diisopropylethylamine, *etc.),* an aromatic amine (e.g. N,N-dimethylaniline, pyridine, lutidine, collidine, 4-(dimethylamino)pyridine, *etc.),* DBU (1,8-diazabicyclo[5.4.0]undec-7-ene), *etc.,* an amide of metal such as lithium diisopropylamide, lithium hexamethyldisilazide, potassium hexamethyldisilazide, sodium hexamethyldisilazide, *etc.*) and acid halide *(e.g.* p-toluenesulfonyl chloride, methanesulfonyl chloride, acetyl chloride, *etc.)* in an organic solvent *(e.g.* an aromatic hydrocarbon such as benzene, toluene, xylene, *etc.,* a halogenated hydrocarbon such as dichloromethane, chloroform, *etc.*, an ester such as ethyl acetate *etc.*; these solvents can be used alone, or used as a mixed solvent containing two or more solvents thereof at an optional ratio, for example, about 1:1 to 1:10 as necessary) or without solvent at about -78 °C to 100°C. Though it is easily understood by those skilled in the art, in the case the compounds represented by general formula (II-A) of the present invention and the compounds represented by general formula (III-A) used as starting materials contain an amino group, a hydroxy group, a mercapto group, or a carboxyl group, that are unspecified in structural formula, such compounds can be prepared by subjecting to the reaction after appropriate protection of the said group in advance, and then removing the protecting group.

As the amino-protecting group, there are exemplified benzyloxycarbonyl group, t-butoxycarbonyl group, allyloxycarbonyl (Alloc) group, 1-methyl-1-(4-biphenyl)ethoxycarbonyl (Bpoc) group, trifluoroacetyl group, 9-fluorenylmethoxycarbonyl group, benzyl (Bn) group, p-methoxybenzyl group, benzyloxymethyl (BOM) group, 2-(trimethylsilyl)ethoxyrnethyl (SEM) group, and so on.

As the hydroxy-protecting group, there are exemplified methyl group, trityl group, methoxymethyl (MOM) group, 1 -ethoxyethyl (EE) group, methoxyethoxymethyl (MEM) group, 2-tetrahydropyranyl(THP) group, trimethylsilyl (TMS) group, triethylsilyl (TES) group, t-butyldimethylsilyl (TBDMS) group, t-butyldiphenylsilyl (TBDPS) group, acetyl (Ac) group, pivaloyl group, benzoyl group, benzyl (Bn) group, p-methoxybenzyl group, allyloxycarbonyl (Alloc) group, and 2,2,2-trichloroethoxycarbonyl (Troc) group, and so on.

As the mercapto-protecting group, there are exemplified benzyl group, methoxybenzyl group, methoxymethyl

(MOM) group, 2-tetrahydropyranyl (THP) group, diphenylmethyl group, acetyl (Ac) group, and so on.

As the carboxyl-protecting group, there are exemplified methyl group, ethyl group, t-butyl group, allyl group, phenacyl group, benzyl group, and so on.

In addition to the above protecting groups for amino, hydroxy, mercapto, or carboxyl groups, there is no particular limitation so long as it can be easily and selectively removed. For example, protecting groups described in Protective Groups in Organic Synthesis (T.W. Greene, John Wiley & Sons Inc., 1999) also can be used.

The deprotection method for the protecting groups of amino, hydroxy, mercapto and carboxyl groups is well known. Examples of such deprotetion are

(1) alkali hydrolysis
(2) deprotection under acidic conditions
(3) deprotection by hydrogenolysis
(4) deprotection using a metal complex
(5) deprotection using a metal, and
(6) deprotection of silyl groups.

Details of these deprotection methods are hereinafter illustrated.

(1) Deprotection by alkali hydrolysis such as deprotection oftrifluoroacetyl group is performed in an organic solvent (*e.g.* methanol, tetrahydrofuran, 1,4-dioxane, *etc.*) at 0 °C to 40 °C, using an alkali hydroxide (*e.g.* sodium hydroxide, potassim hydroxide, lithium hydroxide, *etc.*), an alkaline earth metal hydroxide (*e.g.* barium hydroxide, calcium hydroxide, *etc.*) or a carbonate (*e.g.* sodium carbonate, potassium carbonate, *etc.*) or an aqueous solution thereof or a mixture thereof

(2) The deprotection under acidic conditions such as deprotection of t-butoxycarbonyl, trityl, and so on is carried out at 0 °C to 100 °C with an organic acid (e.g. acetic acid, trifluoroacetic acid, methanesulfonic acid, *etc.)* or an inorganic acid (e.g. hydrochloric acid, sulfuric acid, *etc.)* or a mixture thereof (e.g. hydrogen bromide/acetic acid) in water or an organic solvent *(e.g.* dichloromethane, chloroform, 1,4-dioxane, ethyl acetate, anisole, *etc.).*

(3) The deprotection by hydrogenolysis such as deprotection of benzyl, benzhydryl, benzyloxycarbonyl, allyloxycarbonyl, and so on is carried out at 0 °C to 200 °C in a solvent [ethers (*e.g.* tetrahydrofuran, 1,4-dioxane, dimethoxyethane, diethyl ether, *etc.*), alcohols (*e.g.* methanol, ethanol, *etc.*), benzenes (*e.g.* benzene, toluene, *etc.*), ketones (*e.g.* acetone, methyl ethyl ketone, *etc.*), nitriles (*e.g.* acetonitrile, *etc.*), amides (*e.g.* N,N-dimethylformamide, *etc.*), water, ethyl acetate, acetic acid or a mixture of two or more solvents thereof in the presence of a catalyst (e.g. palladium-carbon, palladium black, palladium hydroxide, platinum oxide, Raney-Ni, *etc.)* under a normal pressure or an increased pressure in a hydrogen stream or in the presence of ammonium formate.

(4) The deprotection using a metal, such as deprotection of allyloxycarbonyl group or the like, is performed at 0 °C to 40 °C in an organic solvent (e.g. dichloromethane, N,N-dimethylformamide, tetrahydrofuran, ethyl acetate, acetonitrile, 1,4-dioxane, ethanol, *etc.),* water or a mixture thereof in the presence of a trapping reagent (e.g. tributyltin hydride, triethylsilane, dimedone, morpholine, diethylamine, pyrrolidine, *etc.),* an organic acid *(e.g.* acetic acid, formic acid, 2-ethylhexanoic acid, *etc.)* and/or an organic acid salt *(e.g.* sodium 2-ethylhexanoate, potassium 2-ethylhexanoate, *etc.)* and in the presence or absence of a phosphine reagent (e.g. triphenylphosphine, *etc.),* using a metal complex [e.g. tetrakistriphenylphosphine palladium(0), bis(triphenylphosphine)palladium(II) dichloride, palladium(II) acetate, tris(triphenylphosphine)rhodium(1)].

(5) The deprotection using a metal is performed in an acidic solvent (e.g. acetic acid, a buffer of pH 4.2 to 7.2, or a mixture of a solvent thereof and an organic solvent such as tetrahydrofuran) in the presence of a zinc dust at 0 to 40 °C while applying ultrasonic waves, if required.

(6) The deprotection of the silyl group is carried out in a water-miscible organic solvent (e.g. tetrahydrofuran, acetonitrile, *etc.*) using tetrabutylammonium fluoride at 0 °C to 40 °C.

Those skilled in the art can easily understand that the desired compounds of the present invention can be easily produced by selectively employing these deprotection methods.

If necessary, conversion into desired non-toxic salts may be followed according to the known method.

(B) Among the compounds represented by general formula (I) of the present invention, a compound wherein T represents O, and  represents a double bond, that is, a compound represented by general formula (IV-A):

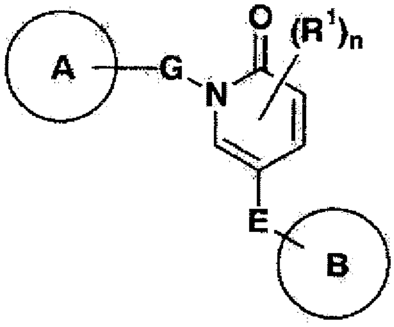

(IV-A)

(wherein all symbols have the same meanings as defined above.) can be prepared by subjecting a compound represented by general formula (II-A) as defined above to a oxidation reaction. Also, a protection/deprotection reaction of functional group may be carried out, if necessary.

The oxidation reaction is well known *per se.* For example, it may be carried out by reacting a compound represented by general formula (II-A) with manganese dioxide in an organic solvent *(e.g.* an aromatic hydrocarbon such as benzene, toluene, xylene, *etc.,* a halogenated hydrocarbon such as dichloromethane, chloroform, *etc.*; these solvents can be used alone, or used as a mixed solvent containing two or more solvents thereof at an optional ratio, for example, about 1:1 to 1:10 (v/v) as necessary) at about 50 °C to 150 °C.

The deprotection reaction of a protective group can be carried out in the same manner as described above.

(C) Among the compounds represented by general formula (I) of the present invention, a compound wherein T represents S, and ---- represents a single bond, that is, a compound represented by general formula (II-B):

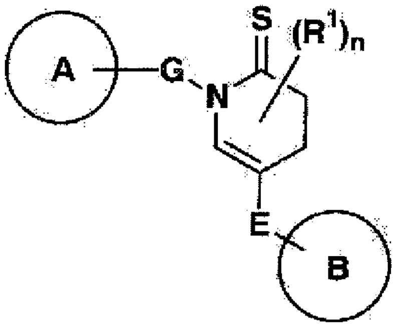

(II-B)

(wherein all symbols have the same meanings as defined above.) can be prepared by subjecting a compound represented by general formula (II-A) as defined above to a thiocarbonylation reaction. Also, a protection/deprotection reaction of functional group may be carried out, if necessary.

The thiocarbonylation reaction is performed by the known method or by the method similar to the known method. For example, the reaction is carried out at 0 °C to 150 °C in an organic solvent *(e.g.* toluene, diethyl ether, dichloromethane, chloroform, 1,4-dioxane, tetrahydrofuran, *etc.*) in the presence of a thionylating reagent (e.g. Lawesson's reagent (2,4-bis(4-methoxyphenyl)-1,3-dithia-2,4-diphosphethane-2,4-disulfide), diphosphorus pentasulfide, *etc.*).

The deprotection reaction of a protective group can be carried out in the same manner as described above.

(D) Among the compounds represented by general formula (I) of the present invention, a compound wherein T represents S, and ---- represents a double bond, that is, a compound represented by general formula (IV-B):

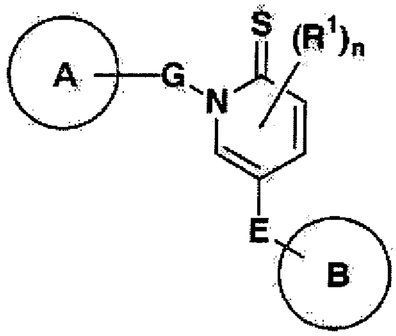

(IV-B)

(wherein all symbols have the same meanings as defined above.) can be prepared by subjecting a compound represented by general formula (IV-A) as defined above to a thiocarbonylation reaction. Also, a protection/deprotection reaction of functional group may be carried out, if necessary.
The thiocarbonylation reaction and the deprotection reaction of a protective group can be carried out in the same manner as described above.
(E) Among the compounds represented by general formula (I) of the present invention, a compound wherein E represents -C(-$OR^v$)-, that is, a compound represented by general formula (V):

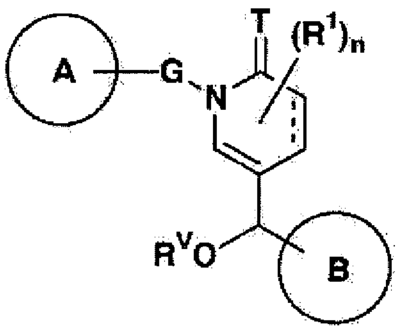

(V)

(wherein $R^V$ represents a C1-8 alkyl group and other symbols have the same meanings as defined above.) can be prepared by subjecting a compound represented by general formula (VI):

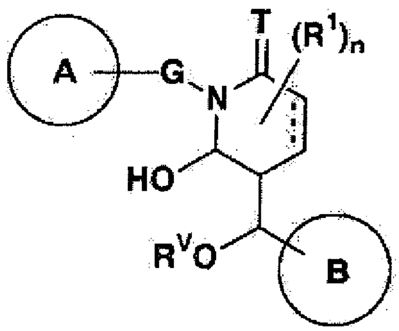

(VI)

(wherein all symbols have the same meanings as defined above.) to a dehydration reaction. Also, a protection/deprotection reaction of functional group may be carried out, if necessary.
The dehydration reaction and the deprotection reaction of a protective group can be carried out in the same manner as described above.
(F) Among the compounds represented by general formula (I) of the present invention, a compound wherein E represents -C(=O)-, that is, a compound represented by general formula (VII):

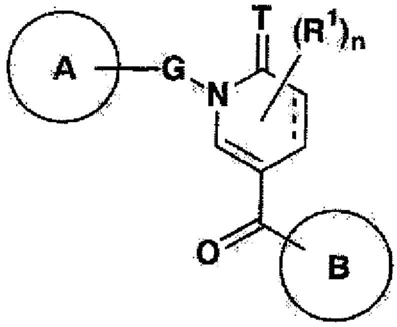

(VII)

(wherein all symbols have the same meanings as defined above.) can be prepared by subjecting a compound represented by general formula (V) as defined above to a oxidation reaction. Also, a protection/deprotection reaction of functional group may be carried out, if necessary.
The oxidation reaction and the deprotection reaction of a protective group can be carried out in the same manner as described above.
(G) Among the compounds represented by general formula (I) of the present invention, a compound wherein E represents -C(=$NR^{VIII}$)-, that is, a compound represented by general formula (VIII):

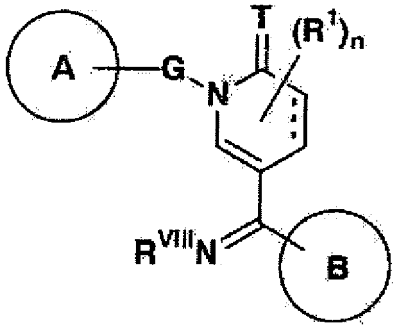

(VIII)

(wherein $R^{VIII}$ represents -$NR^6R^7$, -$OR^2$, or -CN, and other symbols have the same meanings as defined above.) can be prepared by reacting a compound represented by general formula (VII) as defined above and a compound represented by general formula (IX):

**RVIII-NH2** **(IX)**

(wherein $R^{VIII}$ has the same meaning as defined above.)
or a hydrochloride thereof Also, a protection/deprotection reaction of functional group may be carried out, if necessary.

The reaction is well known *per se*. For example, it may be carried out by reacting a compound represented by general formula (VII) with a compound represented by general formula (IX) or a hydrochloride thereof in an organic solvent (e.g. an aromatic hydrocarbon such as benzene, toluene, xylene, *etc.,* a halogenated hydrocarbon such as dichloromethane, chloroform, *etc.;* these solvents can be used alone, or used as a mixed solvent containing two or more solvents thereof at an optional ratio, for example, about 1:1 to 1:10 (v/v) as necessary) or without solvent in the presence or absence of base (*e.g.* an alkylamine such as triethylamine, tributylamine, diisopropylamine, *etc*., an aromatic amine such as N,N-dimethylaniline, pyridine, lutidine, collidine, 4-(dimethylamino)pyridine, *etc.* at about 50 °C to 150 °C.
The deprotection reaction of a protective group can be carried out in the same manner as described above.
The compounds represented by general formula (III-A), (VI) and (IX) as the starting material or the reagent to be used are known per se, or can be easily produced by the method described in the following reaction scheme 1 and 2, or by the known method described in, for example, Comprehensive Organic Transformations: A Guide to Functional Group Preparations, 2nd Edition (Richard C. Larock, John Wiley & Sons Inc, 1999), or a combination method thereof.

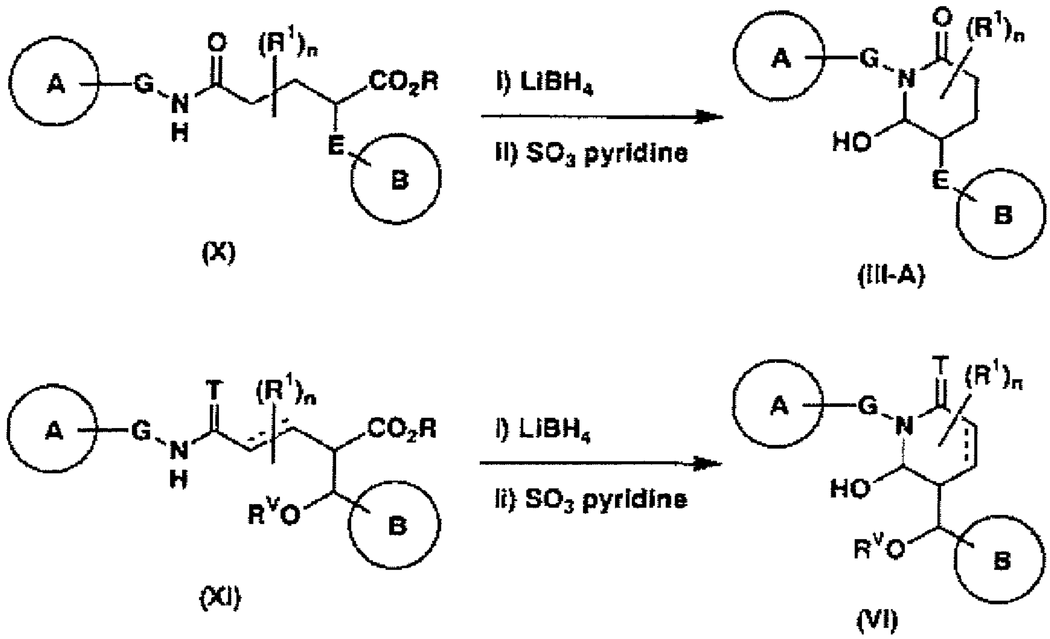


(X) (III-A)

(XI) (VI)

**Reaction Scheme 1**

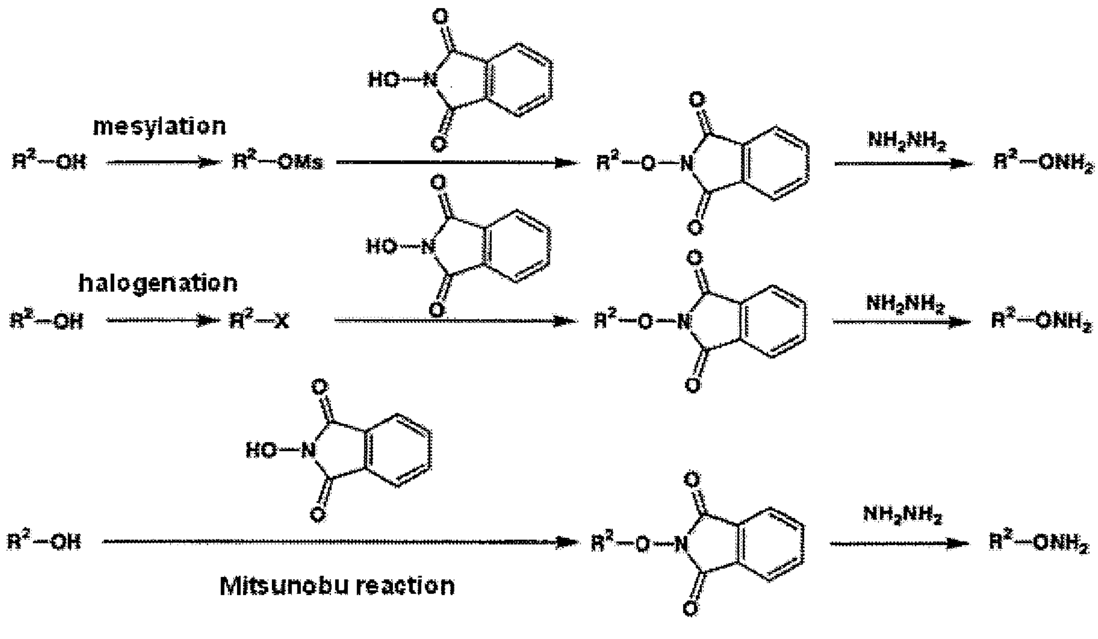


**Reaction Scheme 2**

In each reaction of the present invention, a reagent appropriately carried on a solid carrier of polymers *(e.g.* polystyrene, polyacrylamide, polypropylene, polyethylene glycol, *etc.*) may be used.

The end products of the present invention can be purified by the conventional purification means such as distillation under normal pressure or reduced pressure, high performance liquid chromatography with silica gel or magnesium silicate, thin layer chromatography, or column chromatography, or wasing or recrystallization. Such purification may be carried out in each reaction or may be performed after several reactions.

When the compounds obtained by all reactions described in this specification containing Examples as described below have geometrical isomer (*e.g.* E-form, Z-form, or the like) due to double bond, each isomer can be purified by the conventional purification means such as distillation under normal pressure or reduced pressure, high performance liquid chromatography with silica gel or magnesium silicate, thin layer chromatography, or column chromatography, or wasing or recrystallization.

The heating reaction in each reaction of the present invention may be performed using a water bath, an oil bath, a sand bath or a microwave, though it is apparent to those skilled in the art.

Pharmacological activity of the compounds of the present invention

Except for the pharmacological test described in Examples, there are exemplified the following methods to prove the pharmacological activity of the compounds represented by general formula (I) of the present invention, or its salt, N-oxide or solvate, or a prodrug thereof (hereinafter, abbreviated to the "compound of the present invention"). p38 MAP kinase inhibitory activity of the compounds of the present invention can be proven by these methods.

(a) Study on p38α MAP kinase inhibitory activity

Using activation transcription factor 2 (activating transcription factor 2; ATF-2, Cell Signaling Inc., #9224L) which is a substrate of p38α MAP kinase, the inhibitory effect of the compound of the present invention on the ATF-2 phosphorylation by recombinant human p38α MAP kinase (Upstate Biotechnology Inc., #14-251) was studied by the Western-blotting method using the anti-phosphorylated ATF-2 antibody (Cell Signaling Inc., #9221L). In other words, 10 μL of a solution of the compound of the present invention at a known concentration is added to 10 μL of the kinase buffer (Cell Signaling Inc., #9802) containing recombinant human p38α MAP kinase (100 ng/tube) and pre-incubated for 10 minutes at 30 °C. Then, 20 μL of adenosine triphosphate (ATP) / ATF-2 mixture is added, and after the incubation of 30 minutes at 30 °C, 20 μL of SDS buffer (187.5 mM Tris / 6 % SDS / 30 % glycerol / 150 mM DTT / 0.03 % bromophenol blue) is added to stop the enzyme reaction. After heating at 100 °C for 5 minutes, mixing and centrifugation are performed. After remixing, 20 μL of the sample is subjected to an electrophoresis on SDS-PAGE gel (10 to 20 %, Daiichi Pure Chemicals Co., Ltd.). After the electrophoresis, blotting is performed on PVDF membrane (Sequi-Blot (proprietary name), 0.2 μm, BIO-RAD) by a conventional method. After that, the PVDF membrane is treated with Block Ace (Snow Brand Milk Products Co., Ltd.) (at room temperature, for 1 hour). After reacted with the anti-phosphorylated ATF-2 antibody for 1.5 hours, the membrane is washed with TBS-T solution (0.02 M Tris / 0.137 M NaCl /0.05 % Tween 20, pH 7.6). Furthermore, the reaction with a secondary antibody (anti-rabbit IgG, horseradish peroxide linked whole antibody, Amersham LIFE SCIENCE) is carried out for 1 hour. After washing with TBS-T solution, phosphorylated ATF-2 is detected using Western blotting detection reagent (Amersham Pharmacia Biotech).

(b) Mouse Cytokine-producing Model

By the method shown below, the in vivo effect of the compounds of the present invention can be proven. The vehicle used for administering the compound of the present invention can be any vehicle so long as it is safe and is able to suspend or dissolve into an orally administerable form. For example, such medium includes methylcellulose, carboxymethylcellulose, hydroxypropylcellulose, hydroxypropylmethycellulose, propylene glycol, polyethylene glycol, sugar, sugar alcohol, edible oil, distilled water, physiological saline, and a mixture thereof, all of which have been used for administering a compound to an animal by those skilled in the art.

Experimental Method

The compound of the present invention suspended or dissolved in 0.5 % methylcellulose (MC) is orally administered to a male Balb/c mouse (Charles River Japan, Inc.), and after 0.5 hour, lipopolysaccharide (LPS, 055:B5, Difco) is intraperitoneally administered at the dose of 1 mg/kg (5 animals/group). MC (0.5 %) is orally administered to a control group (5 animals). Ninety minutes after the LPS treatment, heparinized blood collection is performed via the abdominal main vein under anesthesia with ether, and blood plasma is obtained by centrifugation (12,000 rpm, 3 minutes, 4 °C). The obtained blood plasma sample is stored at -80 °C until it is used. TNF-α and IL-6 in the blood plasma are measured using ELISA kits from R&D Inc. (#MTA00) and Endogen Inc. (#EM2IL6), respectively.

Toxicity

Toxicity of the compound of the present invention is sufficiently low, and it was confirmed to be safe enough for use as pharmaceuticals.

Application for Pharmaceuticals

Since the compounds of the present invention suppresse p38 MAP kinase activation in animals including human, particularly in human, they are expected to be useful in the prevention and/or the treatment of cytokine-mediated diseases such as various inflammatory diseases [for example, inflammation, dermatitis, atopic dermatitis, hepatitis, nephritis, glomerulonephritis, pancreatitis, psoriasis, gout, Addison's disease, arthritis (*e.g.* rheumatoid arthritis, osteoarthritis, rhumatoid spondylitis, gouty arthritis, synovitis, etc.), inflammatory ocular diseases, inflammatory pulmonary diseases (e.g. chronic pneumonia, silicosis, pulmonary sarcoidosis, pulmonary tuberculosis, adult respiratory distress syndrome (ARDS), severe acute respiratory syndrome (SARS), etc.), inflammatory bowel diseases *(e.g.* Crohn's disease, ulcerative colitis, *etc.),* allergic diseases (e.g. allergic dermatitis, allergic rhinitis, etc.), autoimmune disease, autoimmune hemolytic anemia, systemic lupus erythematosus, rheumatism, Castleman's disease, immune rejection accompanying transplantation (e.g. graft versus host reaction, *etc.),* and so forth], central nervous system disorders [for example, central neuropathy (e.g. cerebrovascular disease such as cerebral hemorrhage and cerebral infarction, head trauma, spinal cord injury, cerebral edema, multiple sclerosis, *etc.),* neurodegenerative disease *(e.g.* Alzheimer's disease, Parkinson's disease, amyotrophic lateral sclerosis (ALS), AIDS encephalopathy, *etc.*), meningitis, Creutzfeldt-Jakob syndrome, and so forth], respiratory diseases [for example, asthma, chronic obstructive pulmonary disease (COPD), and so forth], cardiovascular diseases [for example, angina, heart failure (e.g. congestive heart failure, acute heart failure, chronic heart failure, *etc.*), myocardial infarction (*e.g.* acute myocardial infarction, myocardial infarction prognosis, *etc.*), atrial myxoma, arteriosclerosis, hypertension, dialysis-induced hypotension, thrombosis, disseminated intravascular coagulation (DIC), reperfusion injury, restenosis after percutaneous transluminal coronary angioplasty (PTCA), and so forth], urinary diseases [for example, renal failure, and so forth], metabolic diseases or endocrine diseases [for example, diabetes, and so forth], bone diseases [for example, osteoporosis, and so forth], cancerous diseases [for example, malignant tumor (e.g. tumor growth and metastasis, *etc.*), multiple myeloma, plasma cell leukemia, carcinemia, and so forth], and infectious diseases [for example, viral infection (e.g. cytomegalovirus infection, influenza virus infection, herpes virus infection, corona virus infection, *etc.*), cachexia associated with infections, cachexia caused by acquired immune deficiency syndrome (AIDS), toxemia (*e.g.* sepsis, septic shock, endotoxin shock, gram negative bacterial sepsis, toxic shock syndrome, severe acute respiratory syndrome (SARS) accompanying virus infection, *etc.),* and so forth], and so on.

Among subtypes ($\alpha$, $\beta$, $\beta_2$,$\gamma$, $\delta$) of p38 MAP kinase, the compounds of the present invention include compounds selectively inhibiting subtype $\alpha$, and compounds inhibiting other subtypes other than subtype $\alpha$.

The compounds of the present invention can be usually administered systemically or topically in the form of oral or parenteral administration.

Since the compounds of the present invention are safe and have low toxicity, they can be administered to a human or a mammal other than humans (e.g. rat, mouse, rabbit, sheep, pig, cow, cat, dog, monkey, etc.).

Although the dose varies depending on age, body weight, symptom, therapeutic effect, administration route and treatment time, the dose for a human adult is generally within a range of 1 mg to 1000 mg per administration that is orally administered up to several times a day, or within a range of 1 mg to 100 mg per administration that is parenterally or preferebaly intravenously administered up to several times a day or intravenously administered over a period of continuous 1 to 24 hours a day.

As mentioned above, the dose to be prescribed depends upon various conditions, and thus there are cases in which doses lower than the range as specified above may be enough or doses greater than the range as specified above may be required.

In the administration of the compounds of the present invention, they are used as solid preparations or liquid preparations for oral administration, or as injections, external preparations or suppositories for parenteral administration.

In the production of these compositions, the compounds of the present invention are not limited to a substantially chemically pure single substance, they may contain impurities (for example, by-products derived from the production process, solvents, starting materials, or decomposition products) so long as such impurities are within an acceptable range as a pharmaceutical bulk.

The solid preparations for oral administration include tablets, pills, capsules, dispersible powders, granules, and so on. The capsules include hard capsules and soft capsules.

In such solid preparations for oral use, one or more of the active compound(s) may be admixed solely or with diluents (e.g. lactose, mannitol, glucose, microcrystalline cellulose, starch, *etc.*), binders (*e.g.* hydroxypropylcellulose, polyvinylpyrrolidone, magnesium aluminometasilicate, *etc.*), disintegrators (*e.g.* cellulose calcium glycolate, *etc.*), lubricants (e.g. magnesium stearate, *etc.*), stabilizers, solubilizers (*e.g.* glutamic acid, aspartic acid, *etc.),* and then formulated into a preparation in the conventional manner. When necessary, such preparations may be coated with a coating agent such as sucrose, gelatin, hydroxypropylcellulose, hydroxypropylmethyl cellulose phthalate, *etc.*) or they may be coated with two or more coating layers. Furthermore, the solid preparations for oral use include capsules of absorbable materials like gelatin.

The liquid preparations for oral administration include pharmaceutically acceptable aqueous solutions, suspensions, emulsions, syrups, elixirs, and so on. In such preparations, one or more of the active compound(s) may be dissolved, suspended or emulsified in a commonly used diluent (*e.g.* purified water, ethanol or a mixture thereof, *etc.*). Besides such diluents, said compositions may also contain wetting agents (*e.g.* glycerin, propylene glycol, *etc.),* suspending agents (*e.g.* Carmellose, agar, gelatin, methylcellulose, *etc.*), emulsifiers *(e.g.* gum acacia, povidone, glyceryl monostearate, *etc.*), sweetening agents *(e.g.* fructose, glucose, *etc.*), flavouring agents (*e.g.* coffee, tea, cocoa, etc.), perfumes (*e.g.* orange oil, thymol, *etc.*), preservatives (*e.g.*benzoic acid, sodium benzoate, ethyl parahydroxybenzoate, propyl parahydroxybenzoate, *etc.),* and buffers (*e.g.* citric acid, disodium hydrogen phosphate, sodium citrate, sodium hydrogen carbonate, acetic acid, lactic acid, *etc.),* and so on.
Injections for parenteral administration include any injection and also include instillation solutions. For example, such injections for parenteral administration include intramuscular injection, subcutaneous injection, intracutaneous injection, intraarterial injection, intravenous injection, intraperitoneal injection, intraspinally injection, and intravenous instillation.
Injections for parenteral administration include solutions, suspensions, emulsions, and solid injection which are dissolved or suspended in a solvent immediately before use. The injections are used by dissolving, suspending or emulsifying one or more of the active compound(s) in a diluent. Said diluents may contain distilled water for injection, physiological saline, vegetable oil, alcohol (*e.g.* propylene glycol, polyethylene glycol, ethanol, *etc.*), and a combination thereof. Further, the injections may contain stabilizers, solubilizers (*e.g.* glutamic acid, aspartic acid, polysorbate 80 (registered trade mark), *etc.*), suspending agents (*e.g.* methylcellulose, carboxymethylcellulose, *etc.*), emulsifiers (*e.g.* polysorbate 80, *etc.*), soothing agents (*e.g.* procaine, lidocaine hydrochloride, *etc.*), buffers (*e.g.* sodium hydrochloride, potassium hydrochloride, disodium hydrogen phosphate, citric acid, sodium citrate, *etc.*), preservatives (*e.g.* benzylalcohol, *etc.*)*, etc.* The injections are sterilized in the final formulation step or prepared by sterile procedure. The injections may also be formulated into sterile solid preparation, for example, by freeze-drying, and may be used after sterilized or dissolved in sterile injectable water or other sterile diluent(s) immediately before use.
Other preparations for parenteral administration may contain one or more active compounds, and as such compositions, there are exemplified conventionally formulated external solutions, ointments, pastes, inhalations, sprays, suppositories, or vaginal pessaries
Sprays may contain stabilizers such as sodium hydrogen sulfite, and buffers capable of imparting isotonicity, including isotonic agents such as sodium chloride, sodium citrate and citric acid. The formulation method for sprays is described in detail in US patents 286891 and 3095355.
The compounds of the present invention may be administered in combination with other drugs for the purpose of

1) complement and/or enhancement of preventing and/or treating effect of the compound,
2) improvement of dynamics and absorption of the compound, and lowering of dose, and/or
3) alleviation of side effect of the compound.

Also, a combination of the compounds of the present invention may be administered as a combination drug for the purpose of:

1) complement and/or enhancement of preventing and/or treating effect of the other drugs,
2) improvement of dynamics and absorption of the other drugs, and lowering of dose, and/or
3) alleviation of side effect of the other drugs.

The compounds of the present invention may be administered in combination with other drugs as a preparation in one drug product comprising these components, or may be administered separately. When they are administered independently, they may be administered simultaneously or with time lag. Administeration with time lag includes the method of administering first the compounds of the present invention and subsequently administering other drugs, and the method of administering first the other drug and subsequently administering the compound of the present invention, and they may be administered in the same route or not.
There is no limitation on the diseases on which the above combination drugs have a preventing and/or treatment effect, so long as the preventing and/or treatment effect of the compound of the present invention is complemented and/or enhanced in the disease.
The weight proportion of the compounds of the present invention and the other drugs is not specifically limited.
Arbitrary two or more of the other drugs may be administered in combination.
Examples of the other drugs for compensating for and/or enhancing the preventive and/or treatment effect of the compounds of the present invention include not only those which have so far been found but also those which will be found on the basis of the aforementioned mechanism.
Other agents to complement and/or enhance a prevention and/or a treatment effect of the compound of the

present invention on rheumatoid arthritis, osteoarthritis, arthritis or the like include a steroidal agent, an elastase inhibitor, a cannabinoid-2 receptor stimulating agent, a prostaglandin, a prostaglandin synthase inhibitor, a phosphodiesterase inhibitor, a metalloproteinase inhibitor, an adhesion molecule inhibitor, an anti-cytokine protein preparation (*e.g.* an anti-TNF-$\alpha$ preparation, an anti-IL-1 preparation, an anti-IL-6 preparation, etc.), a cytokine inhibitor, an immunomodulator, a disease modifying anti-rheumatic drug, a non-steroidal anti-inflammatory agent, c-Jun N-terminal kinase inhibitor, and so on.

Other agents to complement and/or enhance prevention and/or treatment effect of the compound of the present invention on inflammatory bowel disease, Crohn's disease or ulcerative colitis include a steroidal agent, an elastase inhibitor, a cannabinoid-2 receptor stimulating agent, a prostaglandin, a prostaglandin synthase inhibitor, a phosphodiesterase inhibitor, a metalloproteinase inhibitor, an adhesion molecule inhibitor, an anti-cytokine protein preparation, a cytokine inhibitor, an immunomodulator, a leukotoriene receptor antagonist, an anticholinergic agent, a 5-lipoxygenase inhibitor, a nitric monooxide synthase inhibitor, an interleukin-8 antagonist, a poly(ADP)-ribose polymerase inhibitor, a mitochondrial benzodiazepine receptor agonist, an anti-oxidation agent, a local anesthetic, an agent for digestive tract ulcer, a defense factor enhancing agent, mesalazine, salazosulfapyridine and so on.

Other agents to complement and/or enhance prevention and/or treatment effect of the compound of the present invention on asthma, chronic pulmonary inflammatory diseases or adult respiratory distress syndrome (ARDS) include a steroidal agent, an elastase inhibitor, a cannabinoid-2 receptor stimulating agent, a prostaglandin, a prostaglandin synthase inhibitor, a phosphodiesterase inhibitor, a metalloproteinase inhibitor, an adhesion molecule inhibitor, a leukotoriene receptor antagonist, an anticholinergic agent, a thromboxane A2 receptor antagonist, a thromboxane synthase inhibitor, a $\beta_2$ adrenergic receptor stimulating agent, a xanthine derivative, an expectorant agent, an antibiotic, an anti-histamine agent, an anti-cytokine protein preparation, a cytokine inhibitor, a forskolin preparation, a mediator release inhibitor, and so on.

Examples of the steroidal agent include, for example, clobetasol propionate, diflorasone diacetate, fluocinonide, mometasone furcate, betamethasone dipropionate, betamethasone butyrate propionate, betamethasone valerate, difluprednate, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone acetate valerate, fluocinolone acetonide, beclometasone dipropionate, triamcinolone acetonide, flumetasone pivalate, alclometasone dipropionate, clobetasone butyrate, prednisolone, fludroxycortide, cortisone acetate, hydrocortisone, hydrocortisone sodium phosphate, hydrocortisone sodium succinate, fludrocortisone acetate, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, dexamethasone sodium phosphate, dexamethasone palmitate, paramethason acetate, betamethasone, fluticasone propionate, budesonide, flunisolide, ST-126P, ciclesonide, dexamethasone palmitate, mometasone furoate, prasterone sulfonate, deflazacort, methylprednisolone suleptanate, methylprednisolone sodium succinate, and so forth.

Examples of an elastase inhibitor include, for example, ONO-5046, ONO-6818, MR-889, PBI-1101, EPI-HNE-4, R-665, ZD-0892, ZD-8321, GW-311616, DMP-777, L-659286, L-658758, L-680833, L-683845, AE-3763, and so forth.

Examples of a prostaglandin (hereinafter abbreviated to PG) include, for example, PG receptor agonist, PG receptor antagonist, and so forth.

Examples of a PG receptor include, PGE receptor (EP1, EP2, EP3, EP4), PGD receptor (DP, CRTH2), PGF receptor (PGF), PGI receptor (IP), TX receptor (TP), and so forth.

Examples of a prostaglandin synthase inhibitor include, for example, salazosulfapyridine, mesalazine, olsalazine, 4-aminosalicylic acid, JTE-522, auranofin, carprofen, diphenpyramide, flunoxaprofen, flurbiprofen, indometacin, ketoprofen, lornoxicam, loxoprofen, meloxicam, oxaprozin, parsalmide, piproxen, piroxicam, piroxicam betadex, piroxicam cinnamate, tropineindometacinate, zaltoprofen, pranoprofen, and so forth.

Examples of a cannabinoid-2 receptor stimulating agent include, for example, N-arachidonoylethanolamine (anandamide), 2-arachidonoylglycerol (2-AG), and so forth.

Examples of a phosphodiesterase inhibitor include, for example, PDE4 inhibitor such as rolipram, cilomilast (proprietary name:Ariflo), Bay19-8004, NIK-616, roflumilast (BY-217), cipamfylline (BRL-61063), atizoram (CP-80633), SCH-351591, YM-976, V-11294A, PD-168787, D-4396, and IC-485, and a PDE5 inhibitor such as sildenafil, and so forth.

Examples of a metalloproteinase inhibitor include, for example, BB94, ONO-4817, and so forth.

Examples of a adhesion molecule inhibitor include, for example, an antagonist for $\alpha$4-integrin, and so forth.

Examples of an anti-TNF-$\alpha$ preparations include a preparation containing an anti-TNF-$\alpha$ antibody, a soluble TNF-$\alpha$ receptor, an anti-TNF-$\alpha$ receptor antibody or a protein bound to a soluble TNF-$\alpha$, such as a preparation containing infliximab or etanercept, or the like.

Examples of the anti-IL-1 preparations include a preparation containing an anti-IL-1 antibody, a soluble IL-1 receptor, IL-1Ra or an anti-IL-1 receptor antibody, such as a preparation containing anakinra or the like.

Examples of the anti-IL-6 preparations include a preparation containing an anti-IL-6 antibody, a soluble IL-6

receptor or an anti-IL-6 receptor antibody, such as a preparation containing MRA or the like.

Examples of an immunomodulator include, for example, methotrexate, cyclosporine, ascomycin, leflunomide, bucillamine, salazosulfapyridine, azathioprine, tacrolimus, cyclophosphamide, and so on.

Examples of a disease modifying anti-rheumatic drug include, for example, gold thioglucose, sodium aurothiomalate, auranofin, chloroquine, actarit, D-penicillamine preparation, lobenzarit disodium, bucillamine, hydroxychloroquine, salazosulfapyridine, and so on.

Examples of a non-steroidal anti-inflammatory agents include, for example, sasapyrine, sodium salicylate, aspirin, aspirin·di-aluminate composition, diflunisal, indometacin, suprofen, ufenamate, dimethyl isopropylazulene, bufexamac, felbinac, diclofenac, tolmetin sodium, Clinoril, fenbufen, nabumetone, proglumetacin, indometacin farnesil, acemetacin, proglumetacin maleate, amfenac sodium, mofezolac, etodolac, ibuprofen, ibuprofen piconol, naproxen, flurbiprofen, flurbiprofenaxetil, ketoprofen, fenoprofen calcium, thiaprofen, oxaprozin, pranoprofen, loxoprofen sodium, alminoprofen, zaltoprofen, mefenamic acid, aluminum mefenamate, tolfenamic acid, floctafenine, ketophenylbutazon, oxyphenbutasone, piroxicam, tenoxicam, ampiroxicam, Napageln ointment, epirizole, tiaramide hydrochloride, tinoridine hydrochloride, emorfazone, sulpyrine, migrenin, Saridon, Sedes G, Amipylo-N, Sorbon, pyrine preparation for cold syndrome, acetaminophen, phenacetin, dimetotiazine mesilate, simetride combinations, a non-pyrine cough and cold preparation, and so on.

Examples of a leukotoriene receptor antagonist include, for example, pranlukast hydrate, montelukast, zafirlukast, seratrodast, MCC-847, KCA-757, CS-615, YM-158, L-740515, CP-195494, LM-1484, RS-635, A-93178, S-36496, BIIL-284, ONO-4057, and so on.

Examples of an anti-cholinergic agents include, for example, ipratropium bromide, oxitropium bromide, flutropium bromide, cimetropium bromide, temiverine, tiotropium bromide, revatropate (UK-112166), and so on.

Examples of a topical anesthetics include, for example, cocaine hydrochloride, procaine hydrochloride, lidocaine, dibucaine hydrochloride, tetracaine hydrochloride, and so on.

Examples of a defence factor enhancing agents include sucralfate, aldioxa, teprenone, cetraxate hydrochloride, ornoprostil, and so on.

Examples of a thromboxane A2 receptor antagonist include, for example, seratrodast, ramatroban, domitroban calcium hydrate, KT-2-962, and so on.

Examples of a thromboxane synthase inhibitor include ozagrel hydrochloride, imitrodast sodium, and so on.

Examples of a $\beta_2$ adrenergic receptor stimulating agent include, for example, fenoterol hydrobromide, salbutamol sulfate, terbutaline sulfate, formoterol fumarate, salmeterol xinafoate, isoproterenol sulfate, orciprenaline sulfate, chlorprenaline sulfate, epinephrine, trimetoquinol hydrochloride, hexoprenalinemesyl sulfate, procaterol hydrochloride, tulobuterol hydrochloride, tulobuterol, pirbuterol hydrochloride, clenbuterol hydrochloride, mabuterol hydrochloride, ritodrine hydrochloride, bambuterol, dopexamine hydrochloride, meluadrine tartrate, AR-C68397, levosalbutamol, R,R-formoterol, KUR-1246, KUL-7211, AR-C89855, S-1319, and so on.

Examples of a xanthine derivatives include, for example, aminophylline, theophylline, doxophylline, cipamfylline, diprophylline, and so on.

Examples of a expectorant agents include, for example, foeniculated ammonia spirit, sodium hydrogen carbonate, bromhexine hydrochloride, carbocysteine, ambroxol hydrochloride, ambroxol hydrochloride sustained preparation, methylcysteine hydrochloride, acetylcysteine, L-ethylcysteine hydrochloride, tyloxapol, and so on.

Examples of the antibiotics include, for example, cefuroxime sodium, meropenem trihydrate, netilmicin sulfate, sisomicin sulfate, ceftibuten, PA-1806, IB-367, tobramycin, PA-1420, doxorubicin, astromicin sulfate, cefetamet pivoxil hydrochloride, and so on. Examples of the antibiotics as an inhalation include, for example, PA-1806, IB-367, tobramycin, PA-1420, doxorubicin, astromicin sulfate, cefetamet pivoxil hydrochloride.

Examples of an anti-histamine agents include, for example, ketotifen fumarate, mequitazine, azelastine hydrochloride, oxatomide, terfenadine, emedastine difumarate, epinastine hydrochloride, astemizole, ebastine, cetirizine hydrochloride, bepotastine, fexofenadine, loratadine, desloratadine, olopatadine hydrochloride, TAK-427, ZCR-2060, NIP-530, mometasone furoate, mizolastine, BP-294, andolast, auranofin, acrivastine, and so on.

Examples of the cytokine inhibitors include any one of non-protein preparations which can block the action of cytokines, containing a MAP kinase inhibitor, a gene regulating agent, a cytokine production inhibitor, a TNF-$\alpha$ converting enzyme inhibitor, an IL-1$\beta$ converting enzyme inhibitor, an IL-6 antagonist, an IL-8 antagonist, a chemokine antagonist, a gene therapy agent, and an anti-sense compound, and so on. The MAP kinase inhibitor includes, for example, PD-98059 and so on. The gene regulating agent includes an inhibitor to molecules involved in signal transmission, such as NF-$\kappa$B, IKK-1, IKK-2, and AP-1, and so on. The cytokine production inhibitor includes, for example, suplatast tosilate (proprietary name: IPD), T-614, SR-31747, sonatimod, and so on. The chemokine antgonist includes, for example, ONO-4128 and so on. The gene therapy agent includes, for example, a gene therapy agent for accelerating expression of genes having antiinflammatory action, such as interleukin-4, interleukin-10, a soluble IL-1 receptor and a soluble TNF-$\alpha$ receptor, and so on.

Examples of a mediator release inhibitor include, for example, tranilast, sodium cromoglicate, amlexanox,

repirinast, ibudilast, tazanolast, pemirolast potassium, and so on.

Examples of the c-Jun N-terminal kinase inhibitors include compounds described in WO 00/35906, WO 00/35909, WO 00/35921, WO 00/64872, and WO 00/75118, and so on.

Examples of an antioxidant, for example, vitamin E, edaravone, and so on.

## Examples

The present invention is explained below in detail based on Examples, but the present invention is not limited thereto.

In chromatographic separations and TLC, the solvents in parenthesis show the eluting and developing solvents and the ratios of the solvents used are by volume. As ammonia water, a commercially available 28 % ammonia water was used.

In $^1$H-NMR, the solvents in parenthesis show the solvents used in measurement. Unless otherwise specified, $^1$H-NMR data was measured with heavy chloroform ($CDCl_3$).

### Example 1 : ethyl 5-[(2,6-dichlorophenyl)amino]-2-[(2,4-difluorophenyl)(methoxymethoxy)methyl]-5-oxopentanoate

Under an atmosphere of argon, to a solution of the compound prepared in Reference Example 20 of WO03/043988 (3.4 g) in dichloromethane (20 mL) were added diisopropylethylamine (1.66 mL) and methoxymethyl chloride (1.73 mL) at 0 °C and the solution was stirred at 50 °C for 1 hour. 0.5 N hydrochloric acid was added to the reaction solution, which was extracted with ethyl acetate. The organic layer was washed with an aqueous saturated sodium bicarbonate solution and brine sequentially, dried and concentrated. The obtained residue was purified by column chromatography on silica gel (hexane : ethyl acetate = 3 : 1) to give the title compound (2.7 g) having the following physical data.
TLC : Rf 0.47 (hexane : ethyl acetate = 3 : 2);
$^1$H-NMR : $\delta$7.50-7.30 (m, 3H), 7.25-6.95 (m, 2H), 6.95-6.75 (m, 2H), 5.15 (m, 1H), 4.60-4.40 (m, 2H), 4.35-4.00 (m, 2H), 3.33-3.25 (m, 3H), 3.05 (m, 1H), 2.70-1.65 (m, 4H), 1.40-1.00 (m, 3H).

### Example 2 : N-(2,6-dichloroophenyl)-5-(2,4-difluorophenyl)-4-(hydroxymethyl)-5-(methoxymethoxy)pentanamide

Under an atmosphere of argon, to a solution of the compound prepared in Example 1 (2.6 g) in tetrahydrofuran (20 mL) was added lithium borohydride (2.32 g) at room temperature and the solution was stirred at 40 °C overnight. 1N hydrochloric acid cooled with ice was added to the reaction solution, which was extracted with ethyl acetate. The organic layer was washed with brine, dried and concentrated. The obtained residue was purified by column chromatography on silica gel (hexane : ethyl actete =1 : 1) to give the title compound (870 mg) having the following physical data.
TLC : Rf 0.25 (hexane : ethyl actete = 1 : 1);
$^1$H-NMR: $\delta$ 7.50-7.30 (m, 3H), 7.25-7.05 (m, 2H), 6.95-6.70 (m, 2H), 5.03 (m, 1H), 4.60-4.45 (m, 2H), 3.95-3.20 (m, 5H), 2.90-1.70 (m, 6H).

### Example 3 : 1-(2,6-dichlorophenyl)-5-[(2,4-difluorophenyl)(methoxymethoxy)methyl]-6-hydroxypiperidin-2-one

To a solution of the compound prepared in Example 2 (840 mg) in ethyl acetate (4 mL) were added dimethylsulfoxied (4 mL), triethylamine (1.57 mL) and sulfur trioxidepyridine complex (898 mg) and the solution was stirred at room temperature for 1 hour. Iced water was added to the reaction solution, which was extracted with ethyl acetate. The organic layer was washed with 1 N hydrochloric acid, an aqueous saturated sodium bicarbonate solution and brine sequentially, dried and concentrated. The obtained residue was purified by column chromatography on silica gel (hexane : ethyl acetate = 1 : 1) to give the title compound (less polar diastereomer : 345 mg and more polar diastereomer : 357mg) having the following physical data.
less polar diastereomer : TLC : Rf 0.31 (hexane : ethyl acetate = 1 : 1);
more polar diastereomer : TLC : Rf 0.22 (hexane : ethyl acetate = 1 : 1).

### Example 4 : 1-(2,6-dichlorophenyl)-5-[(2,4-difluorophenyl)(methoxymethoxy)methyl]-3,4-dihydropyridin-2(1H)-one

Under an atmosphere of argon, to a solution of the compound prepared in Example 3 (less polar diastereomer: 335 mg) in dichloromethane (3 mL) were added triethylamine (0.32 mL) and methanesulfonyl chloride (175 $\mu$L) at 0 °C and the solution was stirred at room temperature for 1 hour. Iced water was added to the reaction solution, which was extracted with ethyl acetate. The organic layer was washed with 1N hydrochloric acid, an aqueous saturated sodium bicarbonate solution and brine sequentially, dried and concentrated to the title compound (268 mg) having the following

physical data.
TLC : Rf 0.68 (hexane : ethyl acetate = 1:1);
$^1$H-NMR: δ 7.50 (m, 1H), 7.43-7.38 (m, 2H), 7.26 (m, 1H), 6.92 (m, 1H), 6.81 (m, 1H), 6.10 (s, 1H), 5.46 (s, 1H), 4.75 (d, J=6.6Hz, 1H), 4.61 (d, J=6.6Hz, 1H), 3.35 (s, 3H), 2.70-2.63 (m, 2H), 2.41-2.21 (m, 2H).

Example 5 : 1-(2,6-dichlorophenyl)-5-[(2,4-difluorophenyl)(methoxy)methyl]-3,4-dihydropyridin-2(1H)-one

To a solution of the compound prepared in Example 4 (260 mg) in methanol (3 mL) was added a catalytic amount of concentrated hydrochloric acid and the solution was stirred at room temperature for 15 minutes and then at 50 °C for 1 hour. An aqueous saturated sodium bicarbonate solution cooled with ice was added to the reaction solution, which was extracted with ethyl acetate. The organic layer was washed with brine, dried and concentrated. The obtained residue was purified by column chromatography on silica gel (hexane : ethyl acetate = 4 : 1) to give the compound of the present invention (180 mg) having the following physical data.
TLC : Rf 0.34 (hexane : ethyl acetate =3 : 1);
$^1$H-NMR : δ 7.50-7.40 (m, 2H), 7.30-7.20 (m, 2H), 6.90 (m, 1H), 6.80 (m, 1H), 6.08 (s, 1H), 5.00 (s, 1H), 3.35 (s, 3H), 2.69-2.63 (m, 2H), 2.40-2.20 (m, 2H).

Example 6(1) and 6(2) : 1-(2,6-dichlorophenyl)-5-(2,4-difluorobenzoyl)pyridin-2(1H)-one [Example 6(1)] and 1-(2,6-dichlorophenyl)-5-[(2,4-difluorophenyl)(methoxy)methyl]pyridin-2(1H)-one [Example 6(2)]

To a solution of the compound prepared in Example 5 (175 mg) in benzene (5 mL) was added manganese dioxide (870 mg) and the solution was heated under reflux for 7 hours. The catalyst was removed by filtration through Celite (brand name) and washed with ethyl acetate. The filtrated and the washings were combined. The solution was washed with brine, dried and concentrated. The obtained residue was purified by column chromatography on silica gel (hexane : ethyl acetate = 2 : 1) to give the compound of the present invention [Example 6(1):86 mg and Example 6(2): 31 mg] having the following physical data.

Example 6(1):

TLC : Rf 0.30 (hexane : ethyl acetate = 2:1);
$^1$H-NMR: δ 8.02 (m, 1H), 7.65 (m, 1H), 7.59 (m, 1H), 7.51 (m, 1H), 7.49 (m, 1H), 7.39 (m, 1H), 7.01 (m, 1H), 6.91 (m, 1H), 6.76 (m, 1H).

Example 6(2):

TLC : Rf 0.16 (hexane : ethyl acetate = 2 : 1);
$^1$H-NMR : δ 7.52-7.29 (m, 5H), 7.10 (m, 1H), 6.94 (m, 1H), 6.82 (m, 1H), 6.66 (m, 1H), 5.32 (s, 1H), 3.37 (s, 3H).

Example 7 : 1-(2,6-dichlorophenyl)-5-[(2,4-difluorophenyl)(hydroxyimino)methyl]pyridin-2(1H)-one (the mixture of E-form and Z-form)

To a solution of 1-(2,6-dichlorophenyl)-5-(2,4-difluorobenzoyl)pyridin-2(1H)-one (79 mg : It is the compound prepared in Example 6(1)) in pyridine (2 mL) was added hydroxylamine hydrochloride (115 mg) and the solution was stirred at 90 °C for 1 hour. Iced water was added to the reaction solution, which was extracted with ethyl acetate. The organic layer was washed with 1N hydrochloric acid cooed with ice and brine sequentially, dried and concentrated. The obtained residue was purified by column chromatography on silica gel (hexane:ethyl acetate = 3 : 1 → 3 : 2) to give the compound of the present invention (60 mg) having the following physical data.
TLC : Rf 0.24 (hexane : ethyl acetate = 3 : 2);
$^1$H-NMR ($CD_3OD$) : δ 8.20 - 6.60 (m,9H).

Example 8 : 5-(2,4-difluorobenxyl)-1 -(2,6-dimethylphenyl)-3,4-ddihydropyridin-2(1H)-one

By the same procedure as a series of reactions of Example 2 → Example 3 → Example 4 using ethyl 2-(2,4-difluorobenzyl)-5-[(2,6-dimethylphenyl)amino]-5-oxopentanozte instead of the compound prepared in Example 1, the compound of the present invention having the following physical data was obtained.
TLC : Rf 0.59 (hexane : ethyl acetate = 1 : 1 );
$^1$H-NMR : δ 7.20-7.07 (m, 4H), 6.87-6.75 (m, 2H), 5.73 (s, 1H), 3.38 (s, 2H), 2.70-2.63 (m, 2H), 2.36 (t, J=7.5Hz, 2H), 2.16 (s, 6H).

Example 9 : 5-(2,4-difluorobenzyl)-1-(2,6-dimethylphenyl))pyridin-2(1H)-one

By the same procedure as a series of reactions of Example 6 using the compound prepared in Example 8 instead of the compound prepared in Example 5, the compound of the present invention having the following physical data was obtained.
TLC : Rf 0.33 (hexane : ethyl acetate =3:2);
$^1$H-NMR:δ 2.08 (s, 6H), 3.71 (s, 2H), 6.68 (dd, J=9.43, 0.64Hz, 1H), 6.85 (m, 3H), 7.21 (m, 5H).

Example 10 : 1-(2,6-dichlorophenyl)-5-(2,4-difluorobenzyl)-2(1H)-pyridinone

By the same procedure as a series of reactions of Example 2 → Example 3 → Example 4 → Example 6 using ethyl 2-(2,4-difluorobenzyl)-5-[(2,6-dichloroophenyl)amino]-5-oxopentanoate instead of the compound prepared in Example 1, the compound of the present invention having the following physical data was obtained.
TLC : Rf 0.11 (ethyl acetate : hexane = 3:7);
$^1$H-NNIR : δ 3.73 (s, 2H), 6.59-6.71 (m, 1H), 6.76-6.93 (m, 3H), 7.06-7.23 (m, 1H), 7.27-7.39 (m, 2H), 7.43-7.53 (m, 2H).

Example 11 : 1-(2,6-dichlorophenyl)-5-[(2,4-difluorophenyl)(methoxyimino)methyl]-2(1H)-pyridinone (the mixture of E-form and Z-form)

By the same procedure as a series of reactions of Example 7 using O-methyl hydroxylamine hydrochloride instead of hydroxylamine hydrochloride, the compound of the present invention having the following physical data was obtained.
TLC : Rf 0.43 (ethyl acetate:toluene = 1 : 4);
$^1$H-NMR : δ 3.96&4.04 (s&s, 3H), 6.66&6.74 (d&d, J=9.79Hz&9.79Hz, 1H), 6.77-7.03 (m, 3H), 7.09-7.56 (m, 4H), 7.66&8.05 (dd&dd, J=9.79, 2.65Hz&9.79, 2.65Hz, 1H).

Example 11(1)-11(59)

By the same procedure as a series of reactions of Example 7 using a corresponding amine or amine hydrochloride instead of hydroxylamine hydrochloride, the compounds of the present invention having the following physical data were obtained. When the geometric isomers due to double bond were isolated respectively, they weren't defined E-form or Z-form.

Example 11(1) : 1-(2,6-dichlorophenyl)-5-[(2,4-didifluorophenyl)(ethoxyimino)methyl]-2(1H)-pyridinone (the mixture of E-form and Z-form)

TLC : Rf 0.36 (ethyl acetate : hexane = 3:7);
$^1$H-NMR: δ 1.27&1.34 (t&t, J=6.96Hz&J=7.14Hz, 3H), 4.21&4.29 (q&q, J=6.96Hz, &J=7.14Hz, 2H), 6.63-7.54 (m, 8H), 7.67&8.07 (dd&dd, J=9.79, 2.65Hz&J=9.89, 2.56Hz, 1H).

Example 11(2) : 1-(2,6-dichlorophenyl)-5-[(2,4-difluorophenyl)(isobutoxyimino)methyl]-2(1H)-pyridinone (the mixture of E-form and Z-form)

TLC : Rf 0.45 (ethyl acetate : hexane = 3 : 7);
$^1$H-NMR: δ 0:87&0.93 (d&d, J=6.59Hz&3=6.77Hz, 6H), 1.86-2.16 (m, 1H), 3.93&4.01 (d&d, J=6.77Hz&J=6.77Hz, 2H), 6.54-7.59 (m, 8H), 7.63&8.07 (dd&dd, J=9.70, 2.75Hz&J=9.89, 2.56Hz, 1H).

Example 11(3) : 1-(2,6-dichlorophenyl)-5-{(2,4-difluorophenyl)[(2-hydroxyethoxy)imino]methyl}-2(1H)-pyridinone (E form or Z-form : geometrical isomer of a compound of Example 11 (4))

TLC : Rf 0.28 (ethyl acetate : hexane = 3 : 2);
$^1$H-NMR: δ 1.93-2.07 (m, 1H), 3.77-4.00 (m, 2H), 4.23-4.36 (m, 2H), 6.71-6.78 (m, 1H), 6.81-6.84 (m, 1H), 6.87-7.02 (m, 2H), 7.17-7.27 (m, 1H), 7.30-7.38 (m, 1H), 7.42-7.49 (m, 2H), 8.00 (dd, J=9.89, 2.56Hz, 1H).

Example 11 (4) : 1-(2,6-dichlorophenyl)-5-{(2,4-difluorophenyl)[(2-hydroxyethoxy)imino]methyl}-2(1H)-pyridinone (E form or Z-form : geometrical isomer of a compound of Example 11(3))

TLC : Rf 0.20 (ethyl acetate : hexane = 3 : 2);

$^1$H-NMR: δ 2.22 (t, J=5.95Hz, 1H), 3.83-4.10 (m, 2H), 4.31-4.42 (m, 2H), 6.65-6.70 (m, 1H), 6.79-7.06 (m, 2H), 7.32-7.40 (m, 1H), 7.41-7.56 (m, 4H), 7.68 (dd, J=9.89, 2.56Hz, 1H).

Example 11(5):1-(2,6-dichlorophenyl)-5-{(2,4-difluorophenyl)[(2-methoxyethoxy)imino]methyl}-2(1H)-pyridinone (E form or Z-form)

TLC : Rf 0.37 (ethyl acetate : hexane = 2 : 3);
$^1$H-NMR : δ 3.34 (s, 3H), 3.57-3.70 (m, 2H), 4.22-4.39 (m, 2H), 6.70-6.77 (m, 1H), 6.80 (d, J=2.65Hz, 1H), 6.84-7.02 (m, 2H), 7.16-7.28 (m, 1H), 7.29-7.38 (m, 1H), 7.41-7.51 (m, 2H), 8.04 (dd, J=9.79, 2.65Hz, 1H).

Example 11(6) : 1-(2,6-dichlorophenyl)-5-((2,4-difluorophenyl){[2-(dimethylamino)ethoxy]imino}methyl)-2(1H)-pyridinone (E form or Z-form : geometrical isomer of a compound of Example 11(7))

TLC : Rf 0.3 7 (dichloromethane : methanol : ammonia water = 90 : 10 : 1);
$^1$H-NNM: δ 2.24 (s, 6H), 2.64 (t, J=6.04Hz, 2H), 4.29 (t, J=6.04Hz, 2H), 6.74 (d, J=9.89Hz, 1H), 6.78 (d, J=2.56Hz, 1H), 6.83-7.04 (m, 2H), 7.14-7.28 (m, 1H), 7.28-7.38 (m, 1H), 7.41-7.51 (m, 2H), 8.06 (dd, J=9.89, 2.56Hz, 1H).

Example 11(7): 1-(2,6-dichlorophenyl)-5-((2,4-difluarophenyl){[2_ (dimethylamino)ethoxy]imino}methyl)-2(1H)-pyridinone (E form or Z-form : geometrical isomer of a compound of Example 11(6))

TLC : Rf 0.39 (dichloromethane : methanol : ammonia water = 90: 10:1);
$^1$H-NMR : δ 2.23 (s, 6H), 2.67 (t, J=5.68Hz, 2H), 4.34 (t, J=5.68Hz, 2H), 6.56-6.70 (m, 1H), 6.77-7.04 (m, 2H), 7.30-7.41 (m, 1H), 7.41-7.54 (m, 3H), 7.55-7.64 (m, 1H), 7.75-7.85 (m, 1H).

Example 11(8) : 1-(2,6-dichlorophenyl)-5-((2,4-difluorophenyl){[2-(1-pyrrolidinyl)ethoxy]imino}methyl)-2(1H)-pyridimone (E form or Z-form : geometrical isomer of a compound of Example 11 (9))

TLC : Rf 0.37 (dichloromethane : methanol = 9 : 1);
$^1$H-NMR: δ 1.69-1.84 (m, 4H), 2.37-2.66 (m, 4H), 2.80 (t, J=6.22Hz, 2H), 4.32 (t, J=6.22Hz, 2H), 6.70-6.77 (m, 1H), 6.78 (d, J=2.65Hz, 1H), 6.83-7.01 (m, 2H), 7.15-7.26 (m, 1H), 7.29-7.38 (m, 1H), 7.40-7.50 (m, 2H), 8.06 (dd, J=9.79, 2.65Hz, 1H).

Example 11(9): 1-(2,6-dichlorophenyl)-5-((2,4-difluoraphenyl){[2-(1-pyrrolidinyl)ethoxy]imino}methyl)-2(1H)-pyridinone (E form or Z-form : geometrical isomer of a compound of Example 11(8))

TLC : Rf 0.40 (dichloromethane : methanol =9:1);
$^1$H-NMR: δ 1.60-1.76 (m, 4H), 2.39-2.63 (m, 4H), 2.82 (t, J=5.77Hz, 2H), 4.37 (t, J=5.77Hz, 2H), 6.60-6.69 (m, 1H), 6.79-7.04 (m, 2H), 7.31-7.41 (m, 1H), 7.41-7.53 (m, 3H), 7.56-7.66 (m, 1H), 7.67-7.77 (m, 1H).

Example 11(10) : 1-[2-({[[1-(2,6-dichlorophenyl)-6-oxo-1,6-dihydro-3-pyridinyl](2,4-difluorophenyl)methylene]amino}oxy)ethyl]-2,2,6,6-tetramethyl-4-piperidinyl methanesulfonate (the mixture of E-form and Z-form)

TLC : Rf 0.28 (ethyl acetate : hexane =1:1);
$^1$H-NMR: δ 8.06-6.66 (m, 9H), 4.97 (m, 1H), 4.15-4.02 (m, 2H), 3.01 (s, 3H), 2.84-2.73 (m, 2H), 2.02-1.97 (m, 2H), 1.66.-1.57 (m, 2H), 1.16 (s, 6H), 1.10 (s, 6H).

Example 11(11):1-(2,6-dichlorophenyl)-5-((2,4-difluorophenyl){[2-(2,2,6,6-tetramethyl-3,6-dihydro-1(2H)-pyridinyl)ethoxy]imino}methyl)-2(1H)-pyridinone (the mixture of E-form and Z-form)

TLC : Rf 0.53 (ethyl acetate : hexane =1:1);
$^1$H-NMR : δ 8.09-6.65 (m, 9H), 5.55-5.39 (m, 2H), 4,21-4.07 (m, 2H), 2.95-2.85 (m, 2H), 2.00-1.90 (m, 2H),1.12 (s, 6H), 1.10 (s, 6H).

Example 11(12) : 2-({[[1-(2,6-dichlorophenyl)-6-oxo-1,6-dihydro-3-pyridinyl](2,4-difluorophenyl)methylene]amino}oxy)acetamide (the mixture af E-form and Z-form)

TLC : Rf 0.59 (ethyl acetate : methanol = 9 : 1);
$^1$H-NMR : δ 4.60-4.74 (m, 2H), 5.44-5.61 (m, 1H), 6.13-6.27 (m, 1H), 6.67-7.24 (m, 4H), 7.30-7.68 (m, 4H), 7.96-8.05

(m, 1H).

Example 11(13) : 2-({[[1-(2,6-dichlorophenyl)-6-oxo-1,6-dihydro-3-pyridinyl](2,4-difluorophenyl)methylene]amino} oxy)-N-methylacetamide (the mixture of E-form and Z-form)

TLC : Rf 0.60 (ethyl acetate : methanol = 9 : 1);
$^1$H-NMR : δ 2.85-2.91 (m, 3H), 4.60-4.73 (m, 2H), 6.09-7.24 (m, 5H), 7.30-7.66 (m, 4H), 7.97-8.03 (m, 1H).

Example 11(14) : 2-({[[1-(2,6-dichlorophenyl)-6-oxo-1,6-dihydro-3-pyridinyl](2,4-difluorophenyl)methylene]amino} oxy)-N,N-dimethylacetamide (the mixture of E-form and Z-form)

TLC:Rf 0.48 (ethyl acetate : methanol = 19 : 1);
$^1$H-NMR: δ 2,96&2.97 (s&s, 6H), 4.80&4.90 (s&s, 2H), 6.61-6.76 (m, 1H), 6.78-7.04 (m, 3H), 7.28-7.38 (m, 1H), 7.38-7.57 (m, 3H), 7.89-8.10 (m, 1H).

Example 11(15) : 2-({[[1-(2,6-dichlorophenyl)-6-oxo-1,6-dihydro-3-pyridinyl](2,4-difluorophenyl)methylene]amino} oxy)-N',N'-dimethylacethydrazide (the mixture of E-form and Z-form)

TLC : Rf 0.50 (ethyl acetate : methanol = 9 : 1);
$^1$H-NMR: δ 2.50-2.62 (m, 6H), 4.62-4.73 (m, 2H), 6.64-7.25 (m, 5H), 7.31-7.68 (m, 4H), 7.93-8.02 (m, 1H).

Example 11(16) : 1-(2,6-dichlorophenyl)-5-((2,4-difluorophenyl){[2-oxo-2-(1-pyrrolidinyl)ethoxy]imino}methyl)-2(1H)-pyridinone (the mixture of E-form and Z-form)

TLC : Rf0.58 (ethyl acetate : methanol = 9 : 1);
$^1$H-NMR : δ 1.79-2.01 (m, 4H), 3.33-3.56 (m, 4H), 4.70-4.82 (m, 2H), 6.66-7.01 (m, 4H), 7.28-7.58 (m, 4H), 7.96-8.03 (m, 1H).

Example 11(17) : 1-(2,6-dichlorophenyl)-5-((2,4-difluorophenyl){[2-oxo-2-(1-piperidinyl)ethoxy]imino}methyl)-2(1H)-pyridinone (the mixture ofE-form and Z-form)

TLC : Rf 0.69 (ethyl acetate : methanol =9:1);
$^1$H-NMR : δ 1.45-1.70 (m, 6H), 3.26-3.37 (m, 2H), 3.50-3.60 (m, 2H), 4.78-4.92 (m, 2H), 6.66-7.02 (m, 4H), 7.29-7.53 (m, 4H), 7.97-8.04 (m, 1H).

Example 11(18) : 1-(2,6-dichlorophenyl)-5-((2,4-difluorophenyl){[2-(4-morpholinyl)-2-oxoethoxy]imino}methyl)-2 (1H)-pyridinone (the mixture of E-form and Z-form)

TLC : Rf 0.61 (ethyl acetate : methanol = 9 : 1);
$^1$H-NMR : δ 3.37-3.72 (m, 8H), 4.78-4.90 (m, 2H), 6.66-7.03 (m, 4H), 7.29-7.50 (m, 4H), 7.91-8.04 (m, 1H).

Example 11(19) : 1-(2,6-dichlorophenyl)-5-((2,4-difluorophenyl){[2-(4-methyl-1-piperazinyl)-2-oxoethoxy]imino}methyl)-2(1H)-pyridinone (the mixture of E-form and Z-form)

TLC : Rf 0.40 (ethyl acetate: methanol: triethylamine =8 : 1 : 1);
$^1$H-NMR: δ 2.23-2.42 (m, 7H), 3.36-3.69 (m, 4H), 4.78-4.92 (m, 2H), 6.66-7.02 (m, 4H), 7.29-7.51 (m, 4H), 7.93-8.04 (m, 1H).

Example 11(20): 5-[[(benzyloxy)imino](2,4-difluorophenyl)methyl]-1-(2,6-dichlorophenyl)-2(1H)-pyridinone (the mixture of E-form and Z-form)

TLC : Rf 0.36 (ethyl acetate : hexane = 3 : 7);
$^1$H-NMR : δ 5.19&5.26 (s&s, 2H), 6.61-6.68&6.69-6.74 (m&m, 1H), 6.75-6.77&7.52-7.56 (m&m, 1H), 6.80-7.02 (m, 2H), 7.14-7.39 (m, 6H), 7.40-7.51 (m, 3H), 7.61&8.03 (dd&dd, J=9.89, 2.56Hz&J=9.89, 2.56Hz, 1H).

Example 11(21) : 1-(2,6-diehlorophenyl)-5-[(2,4-.dilluorophenyl)(([3_ (methylamino)benzyl]oxy)imino)methyl]-2(1H)-pyridinone (the mixture of E-form and Z-form)

TLC : Rf 0.42 (ethyl acetate : hexane = 1:1);
$^1$H-NMR : δ 8.06-6.52 (m, 13H), 5.20-5.13 (m, 2H), 3.75 (brs, 1H), 2.82-2.81 (m, 3H).

Example 11(22) : 1-(2,6-dichlorophenyl)-5-[(2,4-difluorophenyl)({[3-(dimethylamino)benzyl]oxy}imino)methyl]-2(1H)-pyridinone (the mixture of E-form and Z-form)

TLC : Rf 0.56 (ethyl acetate : hexane = 1 : 1);
$^1$H-NMR : δ 8.06-6.62 (m, 13H), 5.23-5.16 (m, 2H), 2.93-2.92 (m, 6H).

Example 11(23) : 1-(2,6-dichlorophenyl)-5-{(2,4-difluorophenyl)[({3-[(dimethylamino)methyl]benzyl}oxy)imino]methyl}-2(1H)-pyridinone (the mixture of E-form and Z-form)

TLC : Rf 0.59 (ethyl acetate : methanol : triethylamine = 8 : 1 : 1);
$^1$H-NMR : δ 2.18-2.27 (m, 6H), 3.37-3.47 (m, 2H), 5.16-5.27 (m, 2H), 6.59-7.24 (m, 6H), 7.27-7.66 (m, 6H), 7.98-8.08 (m, 1H).

Example 11(24):1-(2,6-dichlorophenyl)-5-{(2,4-difluorophenyl)[(2-pyridinylmethoxy)imino]methyl}-2(1H)-pyridinone (the mixture of E-form and Z-form)

TLC : Rf 0.44 (ethyl acetate : hexane = 3 : 1);
$^1$H-NMR : δ 5.31-5.42 (m, 2H), 6.62-8.59 (m, 13H).

Example 11(25): 1-(2,6-dichlorophenyl)-5-{(2,4-difluorophenyl)[(3-pyridinylmethoxy)imino]methyl}-2(1H)-pyridinone (the mixture of E-form and Z-form)

TLC : Rf 0.33 (ethyl acetate : hexane = 3 : 1);
$^1$H-NMR : δ 5.15-5.31 (m, 2H), 6.59-8.66 (m, 13H).

Example 11(26) : 1-(2,6-dichlorophenyl)-5-{(2,4-difluorophenyl)[(4-pyridinylmethoxy)imino]methyl}-2(1H)-pyridinone (the mixture of E-form and Z-form)

TLC : Rf 0.23 (ethyl acetate : hexane = 3 : 1);
$^1$H-NMR : δ 5.17-5.29 (m, 2H), 6.63-7.26 (m, 6H), 7.28-7.68 (m, 4H), 7.94-8.04 (m, 1H), 8.54-8.63 (m, 2H).

Example 11(27) : 1-(2,6-dichlorophenyl)-5-((2,4-difluorophenyl){[(6-methyl-2-pyridinyl)methoxy]imino}methyl)-2(1H)-pyridinone (E form or Z-form : geometrical isomer of a compound of Example 11(28))

TLC : Rf0.60 (ethyl acetate);
$^1$H-NMR : δ 2.51 (s, 3H), 5.20 (s, 2H), 6.70-6.75 (m, 1H), 6.76 (d, J=2.56Hz, 1H), 6.86-6.99 (m, 2H), 7.09-7.22 (m, 2H), 7.29-7.36 (m, 1H), 7.42-7.46 (m, 2H), 7.54 (dd, J=7.69, 1.74Hz, 1H), 8.01 (dd, J=9.89, 2.56Hz, 1H), 8.44 (dd, J=5.03, 1.74Hz, 1H).

Example 11(28) : 1-(2,6-dichlorophenyl)-5-((2,4-difluorophenyl){[(6-methyl-2-pyridinyl)methoxy]imino}methyl)-2(1H)-pyridinone (E form or Z-form: geometrical isomer of a compound of Example 11(27))

TLC : Rf 0.51 (ethyl acetate);
$^1$H-NMR : δ 2.55 (s, 3H), 5.28 (s, 2H), 6.65 (dd, J=9.70, 0.73Hz, 1H), 6.81-6.98 (m, 2H), 7.13 (dd, J=7.69, 4.85Hz, 1H), 7.31-7.63 (m, 7H), 8.46 (dd, J=4.85, 1.74Hz, 1H).

Example 11(29): 5-[{[(6-chloro-3-pyridinyl)methoxy]imino}(2,4-difluorophenyl)methyl]-1-(2,6-dichlorophenyl)-2(1H)-pyridinone (the mixture of E-form and Z-form)

TLC : Rf 0.44 (ethyl acetate : hexane =1:1);
$^1$H-NMR : δ 5.16&5.23 (s&s, 2H), 6.65&6.73 (dd&dd, J=9.89, 0.73Hz&9.79, 0.64Hz, 1H), 6.77 (d, J=2.56Hz, 1H), 6.81-7.01 (m, 2H), 7.10-7.22 (m, 1H), 7.28-7.39 (m, 2H), 7.40-7.50 (m, 2H), 7.55-7.64 (m, 1H), 7.67&7.98 (dd&dd,

J=9.79, 2.38Hz&9.89, 2.56Hz, 1H), 8.28-8.46 (m, 1H).

Example 11(30) : 1-(2,6-dichlorophenyl)-5-[(2,4-difluorophenyl)({[6-(dimethylamino)-2-pyridinyl]methoxy}imino)methyl]-2(1H)-pyridinone (the mixture of E-form and Z-form)

TLC : Rf 0.52 (ethyl acetate : hexane = 1 : 1);
$^1$H-NMR : δ 8.06-6.38 (m, 12H), 5.23-5.16 (m, 2H), 3.06-3.04 (m, 6H).

Example 11(31) : N-{6-[({[[1-(2,6-dichlorophenyl)-6-oxo-1,6-dihydro-3-pyridinyl](2,4-difluorophenyl)methylene]amino}oxy)methyl]-2-pyridinyl }-2, 2-dimethylpropanamide (the mixture of E-form and Z-form)

TLC : Rf 0.21 (ethyl acetate : hexane = 1 : 1);
$^1$H-NMR : δ 8.20-6.65 (m, 12H), 7.98 (brs, 1H), 5.25-5.17 (m, 2H), 1.57 (s, 9H).

Example 11(32) : 1-(2,6-dichlorophenyl)-5-((2,4-difluorophenyl){[(5-methyl-3-isoxazolyl)methoxy]imino}methyl)-2(1H)-pyridinone (the mixture of E-form and Z-form) TLC : Rf 0.50 (ethyl acetate : hexane = 1 : 1);

$^1$H-NMR : δ 2.36-2.49 (m, 3H), 5.18&5.26 (s&s, 2H), 5.99&6.04 (s&s, 1H), 6.66&6.73 (d&d, J=9.89Hz&9.89Hz, 1H), 6.79-7.05 (m, 3H), 7.14-7.24 (m, 1H), 7.28-7.39 (m, 1H), 7.40-7.52 (m, 2H), 7.63&8.03 (dd&dd, J=9.70, 2.38Hz&9.79, 2.47Hz, 1H).

Example 11(33) : 1-(2,6-dichlorophenyl)-5-((2,4-difluorophenyl){[(1,5-dimethyl-1H-pyrazole-3-yl)methoxy]imino}methyl)-2(1H)-pyridinone (E form or Z-form : geometrical isomer of a compound of Example 11(34))

TLC : Rf 0.37 (ethyl acetate : hexane = 2 : 1);
$^1$H-NMR : δ 2.25 (s, 3H), 3.74 (s, 3H), 5.11 (s, 2H), 6.01 (s, 1H), 6.73 (d, J=9.79Hz, 1H), 6.78 (d, J=2.47Hz, 1H), 6.82-6.97 (m, 2H), 7.15-7.26 (m, 1H), 7.28-7.37 (m, 1H), 7.39-7.48 (m, 2H), 8.07 (dd, J=9.79, 2.47Hz, 1H).

Example 11(34) : 1-(2,6-dichlorophenyl)-5-((2,4-difluorophenyl){[(1,5-dimethyl-1H-pyrazole-3-yl)methoxy]imino}methyl)-2(1H)-pyridinone (E form or Z-form : geometrical isomer of a compound of Example 11(33))

TLC : Rf 0.34 (ethyl acetate : hexane = 2 : 1);
$^1$H-NMR: δ 2.25 (s, 3H), 3.74 (s, 3H), 5.18 (s, 2H), 6.06 (s, 1H), 6.63 (dd, J=9.79, 0.73Hz, 1H), 6.80-6.89 (m, 1H), 6.89-6.98 (m, 1H), 7.30-7.38 (m, 1H), 7.42-7.48 (m, 2H), 7.48-7.56 (m, 2H), 7.67 (dd, J=9.79, 2.11Hz, 1H).

Example 11(35): 1-(2,6-dichlorophenyl)-5-((2,4-difluorophenyl){[(1-methyl-1H-imidazole-2-yl)methoxy]imino}methyl)-2(1H)-pyridinone (the mixture of E-form and Z-form)

TLC : Rf 0.11 (ethyl acetate : hexane = 5 : 1);
$^1$H-NMR : δ 3.63&3.69 (s&s, 3H), 5.25&5.29 (s, 2H), 6.63&6.72 (d&d, J=10.25Hz&9.70Hz, 1H), 6.78 (d, J=2.20Hz, 1H), 6.82-6.97 (m, 3H), 6.97-7.04 (m, 1H), 7.10-7.22 (m, 1H), 7.28-7.38 (m, 1H), 7.40-7.49 (m, 2H), 7.54-7.62&8.01 (m&dd, J=9.79, 2.20Hz, 1H).

Example 11(36) : 1-(2,6-dichlorophenyl)-5-{(2,4-difluorophenyl)[(1,3-thiazole-4-ylmethoxy)imino]methyl}-2(1H)-pyridinone (the mixture ofE-form and Z-form)

TLC : Rf 0.38 (ethyl acetate : hexane = 3:2);
$^1$H-NMR : δ 5.37-5.45 (m, 2H), 6.62-7.00 (m, 3H), 7.18-8.81 (m, 8H).

Example 11(37) : 1-(2,6-dichlorophenyl)-5-{(2,4-difluorophenyl)[(tetrahydro-2H-pyran-4-yloxy)imino]methyl}-2(1H)-pyridinone (the mixture of E-form and Z-form)

TLC : Rf 0.42 (ethyl acetate : hexane =1:1);
$^1$H-NMR : δ 1.61-1.87 (m, 2H), 1.89-2.20 (m, 2H), 3.28-3.66 (m, 2H), 3.69-4.01 (m, 2H), 4.31-4.54 (m, 1H), 6.62-7.02 (m, 3H), 7.15-8.13 (m, 6H).

Example 11(38) : 1-(2,6-dichlorophenyl)-5-((2,4-difluorophenyl){[(1-methyl-4-piperidinyl)oxy]imino}methyl)-2(1H)-pyridinone (the mixture of E-form and Z-form)

TLC : Rf 0.34 (dichloromethane : methanol : ammonia water = 90 : 10 : 1);
$^1$H-NMR : δ 1.70-1.92 (m, 2H), 1.92-2.14 (m, 2H), 2.19-2.40 (m, 5H), 2.42-2.74 (m, 2H), 4.18-4.38 (m, 1H), 6.66&6.74 (d&d, J=9.79Hz&J=9.89Hz, 1H), 6.79&7.68 (d&d, J=2.56Hz&J=2:65Hz, 1H), 6.82-7.01 (m, 2H), 7.13-7.52 (m, 4H), 7.56&8.06 (dd&dd, J=9.79, 2.65Hz&J=9.89, 2.56Hz, 1H).

Example 11(39) : 1-(2,6-dichlorophenyl)-5-((2,4-difluorophenyl){[(1-ethyl-4-piperidinyl)oxy]imino}methyl)-2(1H)-pyridinone (E form or Z-form : geometrical isomer of a compound of Example 11(40))

TLC : Rf 0.34 (dichloromethane : methanol = 9 : 1);
$^1$H-NMR : δ 1.10 (t, J=7.14Hz, 3H), 1.77-1.94 (m, 2H), 2.01-2.15 (m, 2H), 2.28-2.49 (m, 4H), 2.58-2.74 (m, 2H), 4.25-4.39 (m, 1H), 6.65 (d, J=9.79Hz, 1H), 6.83-6.99 (m, 2H), 7.33-7.40 (m, 1H), 7.42-7.51 (m, 3H), 7.55 (dd, J=9.79, 2.65Hz, 1H), 7.67 (d, J=2.65Hz, 1H).

Example 11(40) : 1-(2,6-dichlorophenyl)-5-((2,4-difluorophenyl){[(1-ethyl-4-piperidinyl)oxy]imino}methyl)-2(1H)-pyridinone (E form or Z-form : geometrical isomer of a compound of Example 11(39))

TLC : Rf 0.32 (dichloromethane : methanol = 9 : 1 );
$^1$H-NMR: δ 1.13 (t, J=7.14Hz, 3H), 1.77-2.15 (m, 4H), 2.36-2.70 (m, 6H), 4.26-4.36 (m, 1H), 6.74 (d, J=9.89Hz, 1H), 6.79 (d, J=2.56Hz, 1H), 6.84-7.00 (m, 2H), 7.15-7.25 (m, 1H), 7.29-7.37 (m, 1H), 7.42-7.48 (m, 2H), 8.06 (dd, J=9.89, 2.56Hz, 1H).

Example 11(41) : 1-(2,6-dichlorophenyl)-5-((2,4-difluorophenyl){[(1-isopropyl-4-piperidinyl)oxy]imino}methyl)-2(1H)-pyridinone (the mixture of E-form and Z-form)

TLC : Rf 0.37 (dichloromethane : methanol: ammonia water = 90 : 10 : 1);
$^1$H-NMR : δ 1.00&1.03 (d&d, J=6.59Hz&J=6.59Hz, 6H), 1.69-1.89 (m, 2H), 1.92-2.15 (m, 2H), 2.29-2.46 (m, 2H), 2.53-2.83 (m, 3H), 4.18-4.36 (m, 1H), 6.64&6.74 (d&d, J=9.43Hz&J=9.79Hz, 1H), 6.79&7.70 (d&d, J=2.65Hz&J=2.29Hz, 1H), 6.81-7.00 (m, 2H), 7.12-7.26 (m, 1H), 7.27-7.40 (m, 1H), 7.41-7.52 (m, 2H), 7.56&8.07 (dd&dd, J=9.43, 2.29Hz&J=9.79, 2.65Hz, 1H).

Example 11(42):5-[{[(1-tert-butyl-4-piperidinyl)oxy]imino}(2,4-difluorophenyl)methyl]-1-(2,6-dichlorophenyl)-2(1H)-pyridinone (E form or Z-form : geometrical isomer of a compound of Example 11 (43))

TLC : Rf 0.32 (dichloromethane : methanol : ammonia water = 90 :10 : 1);
$^1$H-NMR : δ 1.05 (s, 9H), 1.70-1.91 (m, 2H), 1.94-2.16 (m, 2H), 2.26-2.57 (m, 2H), 2.63-2,94 (m, 2H), 4.17-4.37 (m, 1H), 6.64 (d, J=9.70Hz, 1H), 6.79-7.01 (m, 2H), 7.31-7.40 (m, 1H), 7.42-7.52 (m, 3H), 7.53-7.61 (m, 1H), 7.64-7.74 (m, 1H).

Example 11(43) : 5-[{[(1-tert-butyl-4-piperidinyl)oxy]imino }(2,4-difluorophenyl)methyl]-1-(2,6-dichlorophenyl)-2(1H)-pyridinone (E form or Z-form : geometrical isomer of a compound of Example 11 (42))

TLC : Rf 0.30 (dichloromethane : methanol : ammonia water = 90 : 10 : 1);
$^1$H-NMR : δ 1.08 (s, 9H), 1.52-1.85 (m, 2H), 1.86-2.10 (m, 2H), 2.25-2.51 (m, 2H), 2.62-2.83 (m, 2H), 4.19-4.34 (m, 1H), 6.74 (d, J=9.89Hz, 1H), 6.79 (d, J=2.56Hz, 1H), 6.82-7.01 (m, 2H), 7.10-7.25 (m, 1H), 7.28-7.38 (m, 1H), 7.41-7.48 (m, 2H), 8.07 (dd, J=9.89, 2.56Hz, 1H).

Example 11(44) ; 5-[({[1-(cyclopropylmethyl)-4-piperidinyl]oxy}imino)(2,4-difluorophenyl)methyl]-1-(2,6-dichlorophenyl)-2(1H)-pyridinone (the mixture of E-form and Z-form)

TLC:Rf 0.33 (dichloromethane : methanol : ammonia water = 90 : 10 : 1);
$^1$H-NMR : δ 0.03-0.23 (m, 2H), 0.37-0.62 (m, 2H), 0.72-0. 97 (m, 1H), 1.71-1.91 (m, 2H), 1.92-2.11 (m, 2H), 2.14-2.43 (m, 4H), 2.60-2.78 (m, 2H), 4.08-4.42 (m, 1H), 6.64&6.74 (d&d, J=9.98Hz&J=9.89Hz, 1H), 6.79&7.73 (d&d, J=2.56Hz&J=2.65Hz, 1H), 6.81-7.00 (m, 2H), 7.14-7.50 (m, 4H), 7.54&8.07 (dd&dd, J=9.98, 2.65Hz&J=9.89, 2.56Hz, 1H).

Example 11(45): 1-(2,6-dichlorophenyl)-5-[(2,4-difluorophenyl)({[1-(2-hydroxyethyl)-4-piperidinyl]oxy}imino)methyl]-2(1H)-pyridinone (the mixture of E-form and Z-form)

TLC : Rf0.29 (dichloromethane : methanol : ammonia water = 90 : 10 : 1);
$^1$H-NMR: δ 1.68-1.89 (m, 2H), 1.90-2.10 (m, 2H), 2.31-2.44 (m, 2H), 2.47-2.56 (m, 2H), 2.57-2.76 (m, 3H), 3.56-3.65 (m, 2H), 4.21-4.39 (m, 1H), 6.66&6.72-6.76 (dd&m, J=9.61, 0.82Hz, 1H), 6.79&7.62-7.64 (d&m, J=2.56Hz, 1H), 6.82-6.99 (m, 2H), 7.16-7.52 (m, 4H), 7.55-7.62&8.07 (m&dd, J=9.70, 2.56Hz, 1H).

Example 11(46):1-(2,6-dichlorophenyl)-5-[(2,4-difluorophenyl)({[1-(2-methoxyethyl)-4-piperidinyl]oxy}imino)methyl]-2(1H)-pyridinone (the mixture of E-form and Z-form)

TLC : Rf 0.35 (dichloromethane : methanol = 9 : 1);
$^1$H-NMR : δ 1.73-1.90 (m, 2H), 1.91-2.11 (m, 2H), 2.25-2.41 (m, 2H), 2.49-2.76 (m, 4H), 3.34&3.35 (s&s, 3H), 3.45-3.55 (m, 2H), 4.19-4.35 (m, 1H), 6.65&6.73 (d&d, J=9.70Hz&J=9.89Hz, 1H), 6.79&7.68 (d&d, J=2.75Hz&2.56Hz, 1H), 6.82-6.98 (m, 2H), 7.13-7.52 (m, 4H), 7.56&8.06 (dd&dd, J=9.70, 2.56Hz&9.89, 2.75Hz, 1H).

Example 11(47):1-(2,6-dichlorophenyl)-5-[(2,4-difluorophenyl)({[1-(2-hydroxy-2-methylpropyl)-4-piperidinyl]oxy}immo) methyl]-2(1H)-pyridinone (the mixture of E-form and Z-form)

TLC : Rf 0.38 (dichloromethane : methanol = 9 : 1);
$^1$H-NMR : δ 1.15&1.16 (s&s, 6H), 1.66-2.09 (m, 4H), 2.28&2.29 (s&s, 2H), 2.42-2.58 (m, 2H), 2.65-2.84 (m, 2H), 4.17-4.36 (m, 1H), 6.63-8.11 (m, 9H).

Example 11(48) : 1-(2,6-dichlorophenyl)-5-((2,4-difluorophenyl){[(1-ethanimidoyl-4-piperidinyl)oxy]imino}methyl)-2(1H)-pyridinone hydrochloride (the mixture of E-form and Z-form)

TLC : Rf 0.35 (dichloromethane : methanol : acetic acid = 10 : 2 : 1);
$^1$H-NMR($CD_3OD$) : δ 1.86-2.23 (m, 4H), 2.30&2.33 (s&s, 3H), 3.43-3.79 (m, 4H), 4.54-4.66 (m, 1H), 6.69&6.75 (d&d, J=9.70Hz&J=9.89Hz, 1H), 6.99-7.12 (m, 2H), 7.15&7.74 (d&d, J=2.56Hz&J=2.56Hz, 1H), 7.25-7.64 (m, 4H), 7.83&8.17 (dd&dd, J=9.70, 2.56Hz&J=9.89, 2.56Hz, 1H), 8.37-8.49 (m, 1H), 8.96-9.10 (m, 1H).

Example 11(49) : 1-(2,6-dichlorophenyl)-5-{(2,4-difluorophenyl)[({1-[2-hydroxy-1-(hydroxymethyl)ethyl]-4-piperidinyl} oxy)imino]methyl}-2(1H)-pyridinone (the mixture ofE-form and Z-form)

TLC : Rf 0.40 (ethyl acetate : methanol : triethylamine = 50 : 10 : 1);
$^1$H-NMR: δ 1.64-1.88 (m, 2H), 1.91-2.02 (m, 2H), 2.53-3.00 (m, 7H), 3.51-3.67 (m, 4H), 4.29 (m, 1H), 6.66&6.73 (d&d, J=9.70Hz&9.89Hz, 1H), 6.78&7.56 (d&d, J=2.38Hz&2.56Hz, 1H), 6.80-7.01 (m, 2H), 7.12-7.52 (m, 4H), 7.64&8.06 (dd&dd, J=9.70, 2.38Hz&9.89, 2.56Hz, 1H).

Example 11(50) : 2-(dimethylamino)ethyl 4-({[[1-(2,6-dichlorophenyl)-6-oxo-1,6-dihydro-3-pyridinyl](2,4-difluorophenyl) methylene]amino}oxy)-1-piperidinecarboxylate (the mixture of E-form and Z-form)

TLC : Rf 0.53 (dichloromethane : methanol : ammonia water = 90 : 10 : 1);
$^1$H-NMR : δ 1.54-2.10 (m, 4H), 2.35 (s, 6H), 2.60-2.74 (m, 2H), 3.22-3.42 (m, 2H), 3.44-3.57 (m, 1H), 3.61-3.83 (m, 1H), 4.15-4.32 (m, 2H), 4.34-4.49 (m, 1H), 6.66&6.75 (dd&dd, J=9.6, 0.6Hz&J=9.9, 0.6Hz, 1H), 6.80-7.00 (m, 2H), 7.15-7.50 (m, 4H), 6.79&7.54 (br.d&dd, J=2.7Hz&J=2.7, 0.6Hz, 1H), 7.60&8.06 (dd&dd, J=9.6, 2.7Hz&J=9.9, 2.7Hz, 1H).

Example 11(51) : 1-(2,6-dichlorophenyl)-5-{(2,4-difluorophenyl)[({1-[2-(dimethylamino)ethyl]-4-piperidinyl}oxy)imino] methyl}-2(1H)-pyridinone (the mixture of E-form and Z-form)

TLC : Rf 0.46 (dichloromethane : methanol: ammonia water = 90 : 10 : 1);
$^1$H-NMR: δ 1.66-1.89 (m, 2H), 1.88-2.11 (m, 2H), 2.16-2.53 (m, 12H), 2.52-2.74 (m, 2H), 4.04-4.49 (m, 1H), 6.65&6.73 (dd&dd, J=9.9, 0.6Hz&J=9.9, 0.6Hz, 1H), 6.82-7.00 (m, 2H), 7.15-7.55 (m, 4H), 6.78&7.66 (br.d&dd, J=2.7Hz&J=2.7, 0.6Hz, 1H), 7.58&8.06 (dd&dd, J=9.9, 2.7Hz&J=9.9, 2.7Hz, 1H).

Example 11(52) : 1-(2,6-dichlorophenyl)-5-{(2,4-difluorophenyl)[({1-[2-(4-morpholinyl)ethyl]-4-piperidinyl}oxy)imino]methyl}-2(1H)-pyridinone (the mixture of E-form and Z-form)

TLC : Rf 0.50 (dichloromethane : methanol : ammonia water = 90 : 10 : 1);
$^1$H-NMR : δ 1.66-1.85 (m, 2H), 1.85-2.14 (m, 2H), 2.18-2.38 (m, 2H), 2.40-2.82 (m, 10H), 3.64-3.78 (m, 4H), 4.15-4.38 (m, 1H), 6.65&6.73 (d&d, J=9.9Hz&9.9Hz, 1H), 6.80-7.00 (m, 2H), 7.15-7.55 (m, 4H), 6.78&7.63 (d&d, J=2.7Hz&2.7Hz, 1H), 7.58&8.06 (dd&dd, J=9.9, 2.7Hz, &9.9, 2.7Hz1H).

Example 11(53): 1-(2,6-dichlorophenyl)-5-[(2,4-difluorophenyl)({[1-(2-oxopropyl)-4-piperidinyl]oxy}imino)methyl]-2(1H)-pyridinone (the mixture of E-form and Z-form)

TLC : Rf 0.45 (dichloromethane : methanol = 9 : 1);
$^1$H-NMR: δ 1.73-2.10 (m, 4H), 2.14 (s, 3H), 2.25-2.39 (m, 2H), 2.49-2.69 (m, 2H), 3.14&3.14 (s&s, 2H), 4.21-4.35 (m, 1H), 6.66&6.74 (d&d, J=9.89Hz&J=9.70Hz, 1H), 6.78&7.65 (d&d, J=2.56Hz&J=2.56Hz, 1H), 6.82-6.99 (m, 2H), 7.15-7.51 (m, 4H), 7.58&8.06 (dd&dd, J=9.89, 2.56Hz&J=9.70, 2.56Hz, 1H).

Example 11(54) : 2-[4-({[[1-(2,6-dichlorophenyl)-6-oxo-1,6-dihydro-3-pyridinyl](2,4-difluorophenyl)methylene]amino } oxy)-1-piperidinyl]-N,N-dimethylacetamide (the mixture of E-form and Z-form)

TLC : Rf 0.30 (dichloromethane : methanol : ammonia water = 90 : 10 : 1);
$^1$H-NMR: δ 1.66-1.88 (m, 2H), 1.89-2.10 (m, 2H), 2.28-2.43 (m, 2H), 2.53-2.74 (m, 2H), 2.92-2.96 (m, 3H), 3.04-3.08 (m, 3H), 3.12 (s, 2H), 4.19-4.33 (m, 1H), 6.65&6.73 (dd&m, J=9.15, 1.28Hz&J=9.89Hz, 1H), 6,77-6.99 (m, 2H), 7.15-8.10 (m, 6H).

Example 11(55): diethyl 2-[4-({[[1-(2,6-dichlorophenyl)-6-oxo-1,6-dihydro-3-pyridinyl](2,4-difluorophenyl)methylene] amino}oxy)-1-piperidinyl]malonate (the mixture of E-form and Z-form)

TLC : Rf 0.46 (ethyl acetate : hexane = 1 : 1);
$^1$H-NMR : δ 1.19-1.34 (m, 6H), 1.72-2.14 (m, 4H), 2.52-3.02 (m, 4H), 4.02&4.03 (s&s, 1H), 4.17-4.33 (m, 5H), 6.66&6.73 (d&d, J=9.70Hz&9.89Hz, 1H), 6.78&7.55 (d&d, J=2.56Hz&2.56Hz, 1H), 6.81-6.98 (m, 2H), 7.15-7.51 (m, 4H), 7.64&8.05 (dd&dd, J=9.89, 2.56Hz&9.70, 2.56Hz, 1H).

Example 11(56) : 5-[{[(1-acetyl-4-piperidinyl)oxy]imino}(2,4-difluorophenyl)methyl]-1-(2,6-dichlorophenyl)-2(1H)-pyridinone (the mixture ofE-form and Z-form)

TLC : Rf 0.39 (dichloromethane : methanol = 9 : 1);
$^1$H-NMR : δ 1.63-2.04 (m, 4H), 2.08&2.10 (s&s, 3H), 3.22-3.96 (m, 4H), 4.33-4.60 (m, 1H), 6.64&6.74 (m&d, J=9.89Hz, 1H), 6.80&7.54 (d&m, J=2.56Hz, 1H), 6.82-7.00 (m, 2H), 7.14-7.51 (m, 4H), 7.60&8.06 (m&dd, J=9.89, 2.56Hz, 1H).

Example 11(57): 1-(2,6-dichlorophenyl)-5-[(2,4-difluorophenyl)({[1-(3,4-dihydro-2H-pyrrole-5-yl)-4-piperidinyl]oxy}imino)methyl]-2(1H)-pyridinone (the mixture of E-form and Z-form)

TLC : Rf 0.1 (dichloromethane: methanol : triethylamine = 90 : 10 : 1);
$^1$H-NMR : δ 1.74-2.15 (m, 6H), 2.39-2.70 (m, 2H), 3.21-3.80 (m, 6H), 4.40-4.50 (m, 1H), 6.66&6.74 (d&d, J=9.70Hz&9.79Hz, 1H), 6.80&7.51 (d&d, J=2.56Hz&2.56Hz, 1H), 6.83-7.01 (m, 2H), 7.13-7.49 (m, 4H), 7.60&8.04 (dd&dd, J=9.70, 2.56Hz&9.79, 2.56Hz, 1H).

Example 11(58) : 1-(2,6-dichlorophenyl)-5-[(2,4-difluorophenyl)({[1-(2-pyridinyl)-4-piperidinyl]oxy}imino)methyl]-2(1H)-pyridinone (the mixture of E-form and Z-form)

TLC : Rf 0.53 (ethyl acetate : hexane = 2 : 1);
$^1$H-NMR.: δ 1.70-1.92 (m, 2H), 1.93-2.20 (m, 2H), 3.27-3.47 (m, 2H), 3.64-3.95 (m, 2H), 4.39-4.57 (m, 1H), 6.54-7.01 (m, 5H), 7.15-7.66 (m, 7H), 8.04-8.22 (m, 1H).

Example 11(59) : 3-({[[1-(2,6-dichlorophenyl)-6-oxo-1,6-dihydro-3-pyridinyl](2,4-difluorophenyl)methylene]amino}oxy)-6-azoniaspiro[5.5]undecane chloride (the mixture of E-form and Z-form)

TLC : Rf 0.29 (n-propanol : acetic acid : water = 4 : 2 : 1);

$^1$H-NMR($CD_3OD$) : δ 1.71 (m, 2H), 2.02-2.38 (m, 8H), 3.24-3.60 (m, 8H), 4.47-4.57 (m, 1H), 6.65-6.78 (m, 1H), 7.00-7.17 (m, 2H), 7.20&7.77 (d&d, J=2.65Hz&2.20Hz, 1H), 7.28-7.65 (m, 4H), 7.86&8.16 (dd&dd, J=9.52, 2.20Hz&9.79, 2.65Hz, 1H).

Example 12 : tert-butyl 4-({[[1-(2,6-dichlorophenyl)-6-oxo-1,6-dihydro-3-pyridinyl](2,4-difluorophenyl)methylene]amino} oxy)-1-piperidinecarboxylate (mixture of E-form and Z-form)

By the same procedure as a series of reactions of Example 7 using tert-butyl 4-(aminooxy)-1-piperidinecarboxylate hydrochloride instead of hydroxylamine hydrochloride, the compound of the present invention having the following physical data was obtained.
TLC : Rf 0.21 (ethyl acetate:hexane =3:7);
$^1$H-NMR : δ 1.45&1.46 (s&s, 9H), 1.60-2.04 (m, 4H), 2.85-3.93 (m, 4H), 4.28-4.53 (m, 1H), 6.65&6.74 (m&m, 1H), 6.79 (d, J=2.65Hz, 1H), 6.82-7.03 (m, 2H), 7.14-7.53 (m, 4H), 7.60&8.06 (dd&dd, J=10.07, 2.38Hz&, J=9.79, 2.65Hz, 1H).

Example 13 : 1-(2,6-dichlorophenyl)-5-{(2,4-difluorophenyl)[(4-piperidinyloxy)imino]methyl}-2(1H)-pyridinone (the mixture of E-form and Z-form)

To a solution of the compound prepared in Example 12 (130 mg) in ethyl acetate (1 mL) was added 4N hydrogen chloride/ethyl acetate solution (1 mL) at 0 °C. After stirring at room temperature for 2 hours, ethyl acetate was added thereto. The organic layer was washed with an aqueous saturated sodium bicarbonate solution and brine sequentially, dried and concentrated to give the compound of the present invention (85 mg) having the following physical data.
TLC : Rf 0.29 (dichloromethane : methanol : ammonia water = 90 : 10 : 1);
$^1$H-NMR : δ 1.53-1.71 (m, 2H), 1.89-2.12 (m, 2H), 2.54-2.80 (m, 2H), 2.90-3.16 (m, 2H), 4.22-4.44 (m, 1H), 6.56-6.77 (m, 1H), 6.77-7.01 (m, 2H), 7.09-7.54 (m, 4H), 7.55-7.68 (m, 1H), 8.06 (dd, J=9.70, 2.56Hz, 1H).

Example 14 : 5-[[(3-azetidinyloxy)imino](2,4-difluorophenyl)methyl]-1-(2,6-dichlorophenyl)-2(1H)-pyridinone (the mixture of E-form and Z-form)

By the same procedure as a series of reactions of Example 7 → Example 13 using tert-butyl 3-(aminooxy)-1-azetidinecarboxylate hydrochloride instead of hydroxylamine hydrochloride, the compound of the present invention having the following physical data was obtained.
TLC : Rf 0.33 (ethyl acetate : methanol : triethylamine =8:1:1);
$^1$H-NMR: δ 3.74-3.96 (m, 4H), 5.07-5.20 (m, 1H), 6.65-7.02 (m, 4H), 7.16-8.10 (m, 6H).

Example 14(1) - 14(10)

By the same procedure as a series of reactions of Example 7 → Example 13 using a corresponding amine or amine hydrochloride instead of hydroxylamine hydrochloride, the compounds of the present invention having the following physical data were obtained.

Example 14(1) : 1-(2,6-dichlorophenyl)-5-((2,4-difluorophenyl){[(35)-3-pyrrolidinyloxy]imino}melhyl)-2(1H)-pyridinone (the mixture of E-form and Z-form)

TLC : Rf 0.35 (dichloromethane : methanol : ammonia water = 90: 10 : 1);
$^1$H-NMR : δ 1.90-2.20 (m, 2H), 2.84-3.37 (m, 4H), 4.84-4.98 (m, 1H), 6.65&6.74 (m&m, 1H), 6.80&7.52 (d&m, J=2.56Hz, 1H), 6.82-7.01 (m, 2H), 7.13-7.50 (m, 4H), 7.60&8.05 (dd&dd, J=9.98, 2.84Hz&J=9.89, 2.56Hz, 1H).

Example 14(2) : 1-(2,6-dichlorophenyl)-5-((2,4-difluorophenyl){[(3R)-3-pyrrolidinyloxy]imino}methyl)-2(1H)-pyridinone (the mixture of E-form and Z-form)

TLC : Rf 0.35 (dichloromethane : methanol : ammonia water = 90 : 10 : 1);
$^1$H-NMR : δ 1.85-2.12 (m, 2H), 2.78-3.13 (m, 3H), 3.15-3.32 (m, 1H), 4.83-4.96 (m, 1H), 6.65&6.72-6.77 (dd&m, J=9.70, 0.73Hz, 1H), 6.79&7.52-7.54 (d&m, 1H), 6.82-6.99 (m, 2H), 7.13-7.51 (m, 4H), 7.59&8.05 (dd&dd, J=9.70, 2.56Hz&J=9.89, 2.56Hz, 1H).

Example 14(3) : 1-(2,6-dichlorophenyl)-5-((2,4-difluorophenyl){[(3 5)-3-piperidinyloxy]imino}methyl)-2(1H)-pyridinone (the mixture of E-form and Z-form)

TLC : Rf 0.37 (dichloromethane : methanol: ammonia water = 90 : 10 : 1);
$^1$H-NMR: δ 1.37-1.54 (m, 1H), 1.55-2.07 (m, 3H), 2.64-2.76 (m, 1H), 2.76-2.95 (m, 2H), 3.06-3.20 (m, 1H), 4.11-4.30 (m, 1H), 6.64-6.68&6.74 (m&d, J=9.89Hz, 1H), 6.79&7.66-7.69 (d&m, J=2.56Hz, 1H), 6.82-7.00 (m, 2H), 7.I8-7.26 (m, 1H), 7.29-7.39 (m, 1H), 7.43-7.52 (m, 2H), 7.60-7.65&8.05 (m&dd, J=9.89, 2.56Hz, 1H).

Example 14(4) : 1-(2,6-dichlorophenyl)-5-((2,4-difluorophenyl){[(3R)-3-piperidinyloxy]imino}methyl)-2(1H)-pyridinone (the mixture of E-form and Z-form)

TLC : Rf 0.37 (dichloromethane : methanol : ammonia water = 90 : 10 : 1);
$^1$H-NNIR : δ 1.36-1.54 (m, 1H), 1.54-1.82 (m, 2H), 1.85-2.08 (m, 1H), 2.64-2.74 (m, 1H), 2.74-2.93 (m, 2H), 3.06-3.21 (m, 1H), 4.08-4.30 (m, 1H), 6.64-6.68&6.74 (m&d, J=9.89Hz, 1H), 6.79&7.67-7.69 (d&m, J=2.56Hz, 1H), 6.82-7.00 (m, 2H), 7.16-7.26 (m, 1H), 7.29-7.39 (m, 1H), 7.41-7.53 (m, 2H), 7.63&8.06 (dd&dd, J=9.61, 2.65Hz&J=9.89, 2.56Hz, 1H).

Example 14(5) : 5-[[(4-azepanyloxy)imino](2,4-difluorophenyl)methyl]-1-(2,6-dichlorophenyl)-2(1H)-pyridinone (the mixture of E-form and Z-form)

TLC : Rf 0.48 (chloroform : methanol = 3 : 1);
$^1$H-NMR : δ 1.44-2.26 (m, 6H), 2.64-3.04 (m, 4H), 4.40-4.57 (m, 1H), 6.53-8.26 (m, 9H).

Example 14(6) : 1-(2,6-dichlorophenyl)-5-{(2,4-difluorophenyl)[(4-piperidinylmethoxy)imino]methyl}-2(1H)-pyridinone (the mixture of E-form and Z-form)

TLC : Rf 0.28 (dichloromethane : methanol : ammonia water = 90 : 10 : 1);
$^1$H-NMR: δ 1.13-1.37 (m, 2H), 1.61-1.78 (m, 2H), 1.84-2.19 (m, 1H), 2.54-2.72 (m, 2H), 3.10-3.21 (m, 2H), 4.01&4.08 (d&d, 3=6.59Hz&J=6.77Hz, 2H), 6.62-7.08 (m, 3H), 7.09-8.16 (m, 6H).

Example 14(7) : 5-[{[(2-aminobenzyl)oxy]imino}(2,4-difluorophenyl)methyl]-1-(2,6-dichlorophenyl)-2(1H)-pyridinone (the mixture of E-form and Z-form)

TLC : Rf 0.43 (ethyl acetate : hexane = 1 : 1);
$^1$H-NMR : δ 3.97 (br.s, 2H), 5.15&5.21 (s&s, 2H), 6.56-7.05 (m, 6H), 7.07-7.52 (m, 6H), 7.59&8.01 (dd&dd, J=9.70, 2.56Hz&J=9.89, 2.56Hz, 1H).

Example 14(8) : 5-[{[(3-aminobenzyl)oxy]imino}(2,4-difluorophenyl)methyl]-1-(2,6-dichlorophenyl)-2(1H)-pyridinone (the mixture of E-form and Z-form)

TLC : Rf 0.30 (ethyl acetate : hexane = 1 : 1);
$^1$H-NMR : δ 3.65 (br.s, 2H), 5.10&5.17 (s&s, 2H), 6.54-7.03 (m, 6H), 7.06-7.57 (m, 6H), 7.63&8.04 (dd&dd, J=9.43, 2.29Hz&J=9.79, 2.65Hz, 1H).

Example 14(9) : 5-[{[(6-amino-2-pyridinyl)methoxy]imino}(2,4-difluorophenyl)methyl]-1-(2,6-dichlorophenyl)-2(1H)-pyridinone (the mixture of E-form and Z-form)

TLC : Rf 0.30 (ethyl acetate : hexane = 2 : 1);
$^1$H-NMR: δ 8.05-6.38 (m, 12H), 5.19-5.13 (m, 2H), 4.40 (s, 2H).

Example 14(10): 1-(2,6-dichlorophenyl)-5-((2,4-difluorophenyl){[2-(1-piperazinyl)ethoxy]imino}methyl)-2(1H)-pyridinone (the mixture of E-form and Z-form)

TLC : Rf 0.19 (dichloromethane: methanol = 10 : 1);
$^1$H-NMR : δ 8.07-6.64 (m, 9H), 4.37-4.28 (m, 2H), 2.94-2.82 (m, 4H), 2.73-2.67 (m, 2H), 2.56-2.42 (m, 4H), 1.77 (brs, 1H).

Example 15 : 1-(2,6-dichlorophenyl)-5-((2,4-diflurophenyl){[(1-oxide-2-pyridinyl)methoxy]imino} methyl)-2(1H)-pyridinone (the mixture of E-form and Z-form)

To a solution of the compound prepared in Example 11 (24) (30 mg) in dichloromethane (2.2 mL) was added metachloro perbenzoic acid (18 mg) and the solution was stirred at room temperature for 2 hours. The reaction solution was poured into an aqueous saturated sodium bicarbonate solution and extracted with ethyl acetate. The organic layer was washed water and brine sequentially, dried and concentrated. The obtained residue was purified by column chromatography on silica gel (acetone : hexane : methanol = 1 : 1 : 0 → 2 : 1 : 0 → 1 : 0 : 0 → 9 : 0 : 1) to give the title compound (26 mg) having the following physical data.
TLC : Rf 0.36 (ethyl acetate:methanol = 9 : 1);
$^1$H-NMR : δ 5.42-5.57 (m, 2H), 6.67-7.08 (m, 3H), 7.19-8.28 (m, 10H).

Example 15(1)- Example 15(2)

By the same procedure as a series of reactions of Example 15 using the compound prepared in Example 11 (31) instead of the compound prepared in Example 11 (24), the compounds of the present invention having the following physical data were obtained.

Example 15(1) : N-{6-[({[[1-(2,6-dichlorophenyl)-6-oxo-1,6-dihydro-3-pyridinyl](2,4-difluorophenyl)methylene]amino} oxy)methyl]-1-oxide-2-pyridinyl}-2,2-dimethylpropanamide (E form or Z-form : geometrical isomer of a compound of Example 15(2))

TLC : Rf 0.37 (ethyl acetate : hexane = 2 : 1);
$^1$H-NMR: δ 10.4 (brs, 1H), 8.39 (m, 1H), 7.98 (dd, J=9.9, 2.4Hz, 1H), 7.47-7.43 (m, 2H), 7.38-7.24 (m, 3H), 7.06-6.93 (m, 3H), 6.82 (d, J=2.4Hz, 1H), 6.71 (m, 1H), 5.47 (s, 2H), 1.37 (s, 9H).

Example 15(2) : N-{6-[({[[1-(2,6-dichlorophenyl)-6-oxo-1,6-dihydro-3-pyridinyl] (2,4-difluorophenyl)methylene] amino } oxy)methyl]-1-oxide-2-pyridinyl}-2,2-dimethylpropanamide (E form or Z-form : geometrical isomer of a compound of Example 15(1))

TLC : Rf 0.28 (ethyl acetate : hexane = 2:1);
$^1$H-NMR : δ 10.4 (brs, 1H), 8.41 (m, 1H), 7.71 (dd, J=9.9, 2.4Hz, 1H), 7.60 (d, J=2.4Hz, 1H), 7.52-7.30 (m, 5H), 7.05 (m, 1H), 6.98-6.82 (m, 2H), 6.71 (d, J=9.9Hz, 1H), 5.54 (s, 2H), 1.36 (s, 9H).

Example 16 : 1-(2,6-dichlorophenyl)-5-[(2,4-difluorophenyl)(hydrazono)methyl]-2(1H)-pyridinone (E-form or Z-form)

To a solution of the compound prepared in Example 6(1) (33 mg) in ethanol (1.0 mL) were added hydrazine monohydrate (54 mg) and acetic acid (50 μL) and the solution was heated under reflux for 5 hours. Ethyl acetate was added thereto. The organic layer was washed with an aqueous saturated sodium bicarbonate solution and brine sequentially, dried and concentrated. The obtained residue was purified by preparative thin-layer chromatography (ethyl acetate : hexane = 1 : 1) to give the title compound (7.2 mg) having the following physical data.
TLC : Rf 0.34 (ethyl acetate : hexane = 1 : 1);
$^1$H-NMR : δ 5.79 (brs, 2H), 6.65-7.01 (m, 3H), 7.20-7.29 (m, 1H), 7.31-7.41 (m, 1H), 7.42-7.67 (m, 4H).

Example 17 : 5-(2-chloro-4-fluorobenzoyl)-1-(2,6-dichlorophenyl)pyridin-2(1H)-one

By the same procedure as a series of reactions of Example 1 → Example 2 → Example 3 → Example 4 → Example 6 using ethyl 5-[(2,6-dichlorophenyl)amino]-2-[(2-chloro-4-fluorophenyl)(hydroxy)methyl]-5-oxopentanoate instead of the compound prepared in Reference Example 20 of WO03/043988, the compounds of the present invention having the following physical data were obtained.
TLC : Rf 0.29 (ethyl acetate : hexane = 1 : 2);
$^1$H-NMR : δ 8.03 (dd, J=9.6, 2.4Hz, 1H), 7.52-7.36 (m, 5H), 7.20 (dd, J=8.4, 2.4Hz, 1H), 7.10 (m, 1H), 6.76 (d, J=9.6Hz, 1H).

Example 18 : 5-[(2-chloro-4-fluorophenyl)(hydroxyimino)methyl]-1-(2,6-dichlorophenyl)-2(1H)-pyridinone (the mixture of E-form and Z-form)

By the same procedure as a series of reactions of Example 7 using the compound prepared in Example 17 instead of the compound prepared in Example 6(1), the compound of the present invention having the following physical

data was obtained.
TLC : Rf 0.20 (ethyl acetate: hexane =2:3);
$^1$H-NMR($CD_3OD$): δ 8.20-6.64 (m, 9H).

Example 18(1) - Example 18(19)

By the same procedure as a series of reactions of Example 7 using the compound prepared in Example 17 instead of the compound prepared in Example 6(1) and a corresponding amine or amine hydrochloride instead of hydroxylamine hydrochloride, the compounds of the present invention having the following physical data were obtained.

Example 18(1): 5-[(2-chloro-4-fluorophenyl)(methoxyimino)methyl]-1-(2,6-dichlorophenyl)-2(1H)-pyridinone (the mixture of E-form and Z-form)

TLC : Rf 0.30 (ethyl acetate : toluene =1:9);
$^1$H-NMR: δ 3,94&4.04 (s&s, 3H), 6.61-6.78 (m, 2H), 6.97-7.52 (m, 6H), 7.64&8.07 (dd&dd, J=9.89, 2.56Hz&j=9.70, 2.56Hz, 1H).

Example 18(2) : 5-[(2-chloro-4-fluorophenyl)(propoxyimino)methyl]-1-(2,6-dichlorophenyl)-2(1H)-pyridinone (the mixture of E-form and Z-form)

TLC : Rf 0.45 (ethyl acetate : hexane = 1 : 3);
$^1$H-NMR: δ 8.11-6.63 (m, 9H), 4.21-4.07 (m, 2H), 1.81-1.61 (m, 2H), 0.98-0.85 (m, 3H).

Example 18(3) : 5-{(2-chloro-4-fluorophenyl)[(2-hydroxyethoxy)imino]methyl}-1-(2,6-dichlorophenyl)-2(1H)-pyridinone (E form or Z-form : geometrical isomer of a compound of Example 18(4))

TLC : Rf 0.27 (ethyl acetate : hexane =3:2);
$^1$H-NMR : δ 8.04 (dd, J=9.9, 2.4Hz, 1H), 7.50-7.05 (m, 6H), 6.75 (dd, J=9.9, 0.6Hz, 1H), 6.67 (dd, J=2.4, 0.6Hz, 1H), 4.30-4.20 (m, 2H), 3.90-3.80 (m, 2H), 1.98 (m, 1H).

Example 18(4) : 5-{(2-chloro-4-fluorophenyl)[(2-hydroxyethoxy)imino]methyl}-1-(2,6-dichlorophenyl)-2(1H)-pyridinone (E form or Z-form : geometrical isomer of a compound of Example 18(3))

TLC : Rf 0.18 (ethyl acetate : hexane = 3 : 2);
$^1$H-NMR : δ 7.66 (dd, J=9.9, 2.4Hz, 1H), 7.52 (dd, J=2.4, 0.6Hz, 1H), 7.50-7.32 (m, 4H), 7.19 (m, 1H), 7.07 (m, 1H), 6.66 (dd, J=9.9, 0.6Hz, 1H), 4.38-4.33 (m, 2H), 3.98-3.92 (m, 2H), 2.13 (m, 1H).

Example 18(5) : 5-{(2-chloro-4-fluorophenyl)[(3-hydroxypropoxy)imino]methyl}-1-(2,6-dichlorophenyl)-2(1H)-pyridinone (E form or Z-form : geometrical isomer of a compound of Example 18(6))

TLC : Rf 0.48 (ethyl acetate : hexane = 3 : 2);
$^1$H-NMR : δ 8.07 (dd, J=9.6, 2.7Hz, 1H), 7.47-7.03 (m, 6H), 6.75 (dd, J=9.6, 0.6Hz, 1H), 6.65 (dd, J=2.7, 0.6Hz, 1H), 4.30 (t, J=6.0Hz, 2H), 3.70 (q, J=6.OHz, 2H), 1.92 (quint, J=6.0Hz, 2H), 1.50 (t, J=6.0Hz, 1H).

Example 18(6) : 5-{(2-chloro-4-fluorophenyl)[(3-hydroxypropoxy)imino]methyl}-1-(2,6-dichlorophenyl)-2(1H)-pyridinone (E form or Z-form : geometrical isomer of a compound of Example 18(5))

TLC : Rf 0.34 (ethyl acetate : hexane = 3 : 2);
$^1$H-NMR : δ 7.61 (dd, J=9.6, 2.7Hz, 1H), 7.54-7.03 (m, 7H), 6.65 (dd, J=9.6, 0.6Hz, 1H), 4.38 (t, J=6.0Hz, 2H), 3.77 (q, J=6.0Hz, 2H), 1.99 (quint, J=6.0Hz, 2H), 1.62 (t, J=6.0Hz, 1H).

Example 18(7) : 5-{(2-chloro-4-fluorophenyl)[(4-hydroxybutoxy)imino]methyl}-1-(2,6-dichlorophenyl)-2(1H)-pyridinone (E form or Z-form : geometrical isomer of a compound of Example 18(8))

TLC : Rf 0.59 (ethyl acetate : hexane = 3 : 2);
$^1$H-NMR : δ 1.50-1.66 (m, 2H), 1.67-1.82 (m, 2H), 3.62 (t, J=6.41Hz, 2H), 4.18 (t, J=6.41Hz, 2H), 6.64 (d, J=2.56Hz, 1H), 6.75 (d, J=9.89Hz, 1H), 7.00-7.12 (m, 1H), 7.13-7.24 (m, 2H), 7.28-7.37 (m, 1H), 7.40-7.50 (m, 2H), 8.08 (dd, J=9.89, 2.56Hz, 1H).

Example 18(8) : 5-{(2-chloro-4-fluorophenyl)[(4-hydroxybutoxy)imino]methyl}-1-(2,6-dichlorophenyl)-2(1H)-pyridinone (E form or Z-form : geometrical isomer of a compound of Example 18(7))

TLC : Rf 0.50 (ethyl acetate : hexane = 3 : 2);
$^1$H-NMR : δ 1-55-1.73 (m, 2H), 1.75-1.91 (m, 2H), 3.67 (t, J=6.41Hz, 2H); 4.27 (t, J=6.41Hz, 2H), 6.64 (d, J=9.70Hz, 1H), 7.01-7.11 (m, 1H), 7.18 (dd, J=8.42, 2.56Hz, 1H), 7.31-7.40 (m, 1H), 7.41-7.52 (m, 3H), 7.53-7.65 (m, 2H).

Example 18(9) : 5-((2-chloro-4-fluorophenyl){[3-(dimethylamino)propoxy]imino}methyl)-1-(2,6-dichlorophenyl)-2(1H)-pyridinone (E form or Z-form : geometrical isomer of a compound of Example 18(10))

TLC : Rf 0.44 (dichloromethane : methanol = 10 : 1);
$^1$H-NMR : δ 8.08 (dd, J=9.6, 2.7Hz, 1H), 7.46-7.02 (m, 6H), 6.74 (dd, J=9.6, 0.6Hz, 1H), 6.64 (dd, J=2.7, 0.6Hz, 1H), 4.18 (t, J=6.3Hz, 2H), 2.31 (t, J=6.3Hz, 2H), 2.22 (s, 6H), 1.84 (quint, J=6.3Hz, 2H).

Example 18(10) : 5-((2-chloro-4-fluorophenyl){[3-(dimethylamino)propoxy]imino}methyl)-1-(2,6-dichlorophenyl)-2(1H)-pyridinone (E form or Z-form : geometrical isomer of a compound of Example 18(9))

TLC : Rf 0.35 (dichloromethane : methanol = 10 : 1);
$^1$H-NMR : δ 7.62 (dd, J=2.7, 0.6Hz, 1H), 7.57 (dd, J=9.6, 2.7Hz, 1H), 7.50-7.03 (m, 6H), 6.64 (dd, J=9.6, 0.6Hz, 1H), 4.28 (t, J=6.3Hz, 2H), 2.39 (t, J=6.3Hz, 2H), 2.22 (s, 6H), 1.92 (quint, J=6.3Hz, 2H).

Example 18(11) : 5-((2-chloro-4-fluorophenyl){[4-(dimethylamino)butoxy]imino}methyl)-1-(2,6-dichlorophenyl)-2(1H)-pyridinone (E form or Z-form : geometrical isomer of a compound of Example 18(12))

TLC : Rf 0.40 (chloroform : methanol = 6 : 1);
$^1$H-NMR : δ 1.40-1.54 (m, 2H), 1.59-1.74 (m, 2H), 2.19 (s, 6H), 2.24 (t, J=6.50Hz, 2H), 4.15 (t, J=6.50Hz, 2H), 6.63 (dd, J=2.56, 0.64Hz, 1H), 6.74 (dd, J=9.89, 0.64Hz, 1H), 6.98-7.11 (m, 1H), 7.12-7.24 (m, 2H), 7.28-7.38 (m, 1H), 7.39-7.49 (m, 2H), 8.08 (dd, J=9.89, 2.56Hz, 1H).

Example 18(12) : 5-((2-chloro-4-fluorophenyl){[4-(dimethylamino)butoxy]imino}methyl)-1-(2,6-dichlorophenyl)-2(1H)-pyridinone (E form or Z-form : geometrical isomer of a compound of Example 18(11))

TLC: Rf 0.30 (chloroform : methanol = 6:1);
$^1$H-NMR : δ 1.47-1.64 (m, 2H), 1.69-1.83 (m, 2H), 2.21 (s, 6H), 2.31 (t, J=6.59Hz, 2H), 4.25 (t, J=6.59Hz, 2H), 6.64 (d, J=9.89Hz, 1H), 7.00-7.11 (m, 1H), 7.17 (dd, J=8.42, 2.56Hz, 1H), 7.30-7.39 (m, 1H), 7.41-7.54 (m, 4H), 7.63 (dd, J=9.89, 2.56Hz, 1H).

Example 18(13) : 5-[(2-chloro-4-fluorophenyl)({[6-(hydroxymethyl)-2-pyridinyl]methoxy}imino)methyl]-1-(2,6-dichlorophenyl)-2(1H)-pyridinone (the mixture of E-form and Z-form)

TLC : Rf 0.31 (ethyl acetate : hexane =3:1);
$^1$H-NMR: δ 3.60-3.82 (m, 1H), 4.70-4.79 (m, 2H), 5.26-5.44 (m, 2H), 6.62-8.08 (m, 12H).

Example 18(14) : 5-{(2-chloro-4-fluorophenyl)[({6-[(dimethylamino)methyl]-2-pyridinyl}methoxy}imino]methyl}-1-(2,6-dichlorophenyl)-2(1H)-pyridinone (the mixture of E-form and Z-form)

TLC : Rf 0.57 (ethyl acetate : methanol : triethylamine = 8 : 1 : 1);
$^1$H-NMR : δ 2.24-2.33 (m, 6H), 3.52-3.60 (m, 2H), 5.26-5.44 (m, 2H), 6.62-8.08 (m, 12H).

Example 18(15) : 5-{(2-chloro-4-fluorophenyl)[(2-pyrazinylmethoxy)imino]methyl}-1-(2,6-dichlorophenyl)-2(1H)-pyridinone (E form or Z-form : geometrical isomer of a compound of Example 18(16))

TLC : Rf 0.48 (ethyl acetate : hexane = 3 : 1);
$^1$H-NMR : δ 5.34 (s, 2H), 6.62-6.76 (m, 2H), 7.03-7.49 (m, 6H), 7.96-8.05 (m, 1H), 8.49-8.65 (m, 3H).

Example 18(16) : 5-{(2-chloro-4-fluorophenyl)[(2-pyrazinylmethoxy)imino]methyl}-1-(2,6-dichlorophenyl)-2(1H)-pyridinone (E form or Z-form : geometrical isomer of a compound of Example 18(15))

TLC : Rf 0.42 (ethyl acetate : hexane =3:1);
$^1$H-NMR : δ 5.42 (s, 2H), 6.63-6.72 (m, 1H), 7.00-7.72 (m, 9H), 8.50-8.58 (m, 2H).

Example 18(17) : 5-{(2-chloro-4-fluorophenyl)[(2-pyrimidinylmethoxy)imino]methyl}-1-(2,6-dichlorophenyl)-2(1H)-pyridinone (the mixture of E-form and Z-form)

TLC : Rf 0.34 (ethyl acetate : hexane =3:1);
$^1$H-NMR : δ 5.32-5.53 (m, 2H), 6.60-6.74 (m, 2H), 7.00-7.51 (m, 7H), 7.77-8.02 (m, 1H), 8.63-8.80 (m, 2H).

Example 18(18) : 5-{(2-chloro-4-fluorophenyl)[(1,2,4-oxadiazole-3-ylmethoxy)imino]methyl}-1-(2,6-dichlorophenyl)-2 (1H)-pyridinone (E form or Z-form)

TLC : Rf 0.51 (ethyl acetate : hexane = 2 : 1);
$^1$H-NMR: δ 5.42 (s, 2H), 6.62-6.70 (m, 1H), 7.00-7.20 (m, 2H), 7.30-7.50 (m, 5H), 7.71-7.78 (m, 1H), 8.73 (s, 1H).

Example 18(19) : 5-((2-chloro-4-fluorophenyl){[(1-ethyl-4-piperidinyl)oxy]imino}methyl)-1-(2,6-dichlorophenyl)-2 (1H)-pyridinone (the mixture of E-form and Z-form)

TLC : Rf0.39 (dichloromethane : methanol: ammonia water = 90 : 10 : 1);
$^1$H-NMR : δ 1.07 (t, J=7.14Hz, 3H), 1.65-1.87 (m, 2H), 1.91-2.09 (m, 2H), 2.20-2.33 (m, 2H), 2.33-2.45 (m, 2H), 2.51-2.68 (m, 2H), 4.18-4.34 (m, 1H), 6.60-8.13 (m, 9H).

Example 19 : 5-{(2-chloro-4-fluorophenyl)[(4-piperidinyloxy)imino]methyl}-1-(2,6-dichlorophenyl)-2(1H)-pyridinone (the mixture of E-form and Z-form)

By the same procedure as a series of reactions of Example 7 → Example 13 using the compound prepared in Example 17 instead of the compound prepared in Example 6(1) and tert-butyl 4-(aminooxy)-1-piperidinecarboxylate hydrochloride instead of hydroxylamine hydrochloride, the compound of the present invention having the following physical data was obtained.
TLC : Rf 0.25 (dichloromethane : methanol : ammonia water = 90 : 10 : 1);
$^1$H-NMR : δ 1.52-1.73 (m, 2H), 1.87-2.11 (m, 2H), 2.56-2.76 (m, 2H), 2.90-3.11 (m, 2H), 4.23-4.39 (m, 1H), 6.62-8.14 (m, 9H).

Example 20 : 5-(2,4-difluorobenzoyl)-1-(2,6-dimethylphenyl)pyridin-2(1H)-one

By the same procedure as a series of reactions of Example 6 using the compound prepared in Example 8 instead of the compound prepared in Example 5, the compound of the present invention having the following physical data was obtained.
TLC : Rf 0.35 (ethyl acetate : hexane = 35 : 65);
$^1$H-NMR: δ 2.10 (s, 6H), 6.76 (d, J=9.70Hz, 1H), 6.89 (m, 1H), 7.01 (m, 1H)7.15-7.23 (m, 2H), 7.28 (m, 1H), 7.59 (m, 1H), 7.68 (m, 1H), 8.01 (m, 1H).

Example 21 : 5-[(2,4-difluorophenyl)(hydroxyimino)methyl]-1-(2,6-dimethylphenyl)-2(1H)-pyridinone (the mixture of E-form and Z-form)

By the same procedure as a series of reactions of Example 7 using the compound prepared in Example 20 instead of the compound prepared in Example 6(1), the compound of the present invention having the following physical data was obtained.
TLC : Rf 0.26 (ethyl acetate : hexane = 1 : 1);
$^1$H-NMR : δ 2.06&2.11 (s&s, 6H), 6.63-6.80 (m, 1H), 6.81-7.04 (m, 2H), 7.06-7.34 (m, 3H), 7.39-7.78 (m, 2H), 7.98 (dd, J=9.70, 2.56Hz, 1H).

Example 21 (1) - Example 21 (7)

By the same procedure as a series of reactions of Example 7 using the compound prepared in Example 20

instead of the compound prepared in Example 6(1) and a corresponding amine or amine hydrochloride instead of hydroxylamine hydrochloride, the compounds of the present invention having the following physical data were obtained.

Example 21(1) ; 5-{(2,4-difluorophenyl)[(2-pyridinylmethoxy)imino]methyl}-1-(2,6-dimethylphenyl)-2(1H)-pyridinone (the mixture of E-form and Z-form)

TLC : Rf 0.34 (ethyl acetate : hexane = 3 : 1);
$^1$H-NMR : δ 1.99-2.10 (m, 6H), 5.27-5.40 (m, 2H), 6.63-8.60 (m, 13H).

Example 21(2) : 5-((2,4-difluorophenyl){[(1-ethyl-4-piperidinyl)oxy]imino}methyl)-1-(2,6-dimethylphenyl)-2(1H)-pyridinone (the mixture of E-form and Z-form)

TLC : Rf 0.47 (ethyl acetate : methanol : triethylamine = 8 : 1 : 1);
$^1$H-NMR : δ 1.06 (t, J=7.23Hz, 3H), 1.68-1.83 (m, 2H), 1.89-2.02 (m, 2H), 2.03-2.13 (m, 6H), 2.15-2.29 (m, 2H), 2.30-2.43 (m, 2H), 2.48-2.70 (m, 2H), 4.18-4.33 (m, 1H), 6.62-8.08 (m, 9H).

Example 21(3) : 5-((E)-(2,4-difluorophenyl){[(1-isopropylpiperidine-4-yl)oxy]imino}methyl)-1-(2,6-dimethylphenyl)pyridin-2(1H)-one (E-form : geometrical isomer of a compound of Example 21(4))

TLC : Rf 0.41 (dichloromethane : methanol : ammonia water = 90 : 10 : 1);
$^1$H-NMR : δ 1.00 (d, J=6.59Hz, 6H), 1.71-2.14 (m, 10H), 2.31-2.42 (m, 2H), 2.62-2.77 (m, 3H), 4.20-4.32 (m, 1H), (d, J=9.70Hz, 1H), 6.80-6.89 (m, 1H), 6.90-6.98 (m, 1H), 7.14-7.28 (m, 3H), 7.43-7.53 (m, 1H), 7.57 (dd, J=9.70, 2.38Hz, 1H), 7.68 (d, J=2.38Hz, 1H).

Example 21(4) : 5-((Z)-(2,4-difluorophenyl){[(1-isopropylpiperidine-4-yl)oxy]imino}methyl)-1-(2,6-dimethylphenyl)pyridin-2(1H)-one (Z-form : geometrical isomer of a compound of Example 21(3))

TLC : Rf 0.38 (dichloromethane : methanol : ammonia water = 90 : 10 : 1);
$^1$H-NMR : δ 1.05 (d, J=6.59Hz, 6H), 1.69-1.90 (m, 2H), 1.93-2.17 (m, 8H), 2.34-2.50 (m, 2H), 2.58-2.70 (m, 2H), 2.72-2.86 (m, 1H), 4.21-4.31 (m, 1H), 6.75 (d, J=9.79Hz, 1H), 6.81-6.97 (m, 3H), 7.11-7.28 (m, 4H), 8.04 (dd, J=9.79, 2.65Hz, 1H).

Example 21 (5) : 5-((2,4-difluorophenyl){[(1-pyridine-2-ylpiperidine-4-yl)oxy]imino}methyl)-1-(2,6-dimethylphenyl)pyridin-2(1H)-one (the mixture of E-form and Z-form)

TLC : Rf 0.41 (ethyl acetate : hexane = 2 : 1);
$^1$H-NMR : δ 1.70-1.88 (m, 2H), 1.91-2.15 (m, 8H), 3.25-3.47 (m, 2H), 3.65-3.99 (m, 2H), 4.37-4.55 (m, 1H), 6.54-8.23 (m, 13H).

Example 21(6) : 5-((2,4-difluorophenyl){[(1-methyl-4-piperidinyl)oxy]imino}methyl)-1-(2,6-dimethylphenyl)-2(1H)-pyridinone (the mixture of E-form and Z-form) white amorphous

TLC : Rf 0.33 (dichloromethane : methanol: ammonia water = 90 : 10: 1);
$^1$H-NMR : δ 1.43-1.74 (m, 2H), 1.76-1.99-(m, 2H), 2.02-2.72 (m, 13H), 4.24-4.38 (m, 1H), 6.67&6.75 (d&d, J=9.70Hz&J=9.70Hz, 1H), 6.81-8.05 (m, 8H).

Example 21(7) : 5-{(2,4-difluorophenyl)[({1-[2-(dimethylamino)ethyl]-4-piperidinyl}oxy)imino]methyl}-1-(2,6-dimethylphenyl)-2(1H)-pyridinone (the mixture ofE-form and Z-form)

TLC : Rf0.29 (dichloromethane : methanol : ammonia water = 90 : 10 : 1);
$^1$H-NMR : δ 1.70-1.87 (m, 2H), 1.91-2.15 (m, 8H), 2.16-2.84 (m, 14H), 4.21-4.33 (m, 1H), 6.66&6.75 (d&d, J=9.70Hz&J=9.70Hz, 1H), 6.81-8.06 (m, 8H).

Example 22 : 5-{(2,4-difluorophenyl)[(4-piperidinyloxy)imino]methyl}-1-(2,6-dimethylphenyl)-2(1H)-pyridinone (the mixture of E-form and Z-form)

By the same procedure as a series of reactions of Example 7 → Example 13 using the compound prepared in Example 20 instead of the compound prepared in Example 6(1) and tert-butyl 4-(aminooxy)-1-piperidinecarboxylate hydrochloride instead of hydroxylamine hydrochloride, the compound of the present invention having the following

physical data was obtained.
TLC : Rf 0.33 (ethyl acetate : methanol : tirethyl amine = 7 : 2 : 1);
$^1$H-NMR : δ 1.52-1.70 (m, 2H), 1.89-2.01 (m, 2H), 2.03-2.12 (m, 6H), 2.62-2.76 (m, 2H), 2.91-3.11 (m, 2H), 4.25-4.37 (m, 1H), 6.64-8.06 (m, 9H).

Example 23 : 1-(4-bromo-2,6-dichlorophenyl)-5-(2,4-difluorobenzoyl)pyridin-2(1H)-one

By the same procedure as a series of reactions of Example 2 → Example 3 → Example 4 → Example 6 using ethyl 5-[(2,6-dichloro-4-bromophenyl)amino]-2-[(2,4-difluorophenyl)(hydroxy)methyl]-5-oxopentanoate instead of the compound prepared in Example 1, the compound of the present invention having the following physical data was obtained.
TLC : Rf 0.48 (ethyl acetate : hexane = 3 : 7);
$^1$H-NMR : δ 8.00 (m, 1H), 7.66 (s, 2H), 7.64-7.54 (m, 2H), 7.01 (m, 1H), 6.91 (m, 1H), 6.75 (d, J=9.7Hz, 1H).

Example 24:1-(4-bromo-2,6-dichlorophenyl)-5-{(2,4-difluorophenyl)[(4-piperidinyloxy)imino]methyl}-2(1H)-pyridinone (the mixture of E-form and Z-form)

By the same procedure as a series of reactions of Example 7 → Example 13 using the compound prepared in Example 23 instead of the compound prepared in Example 6(1) and tert-butyl 4-(aminooxy)-1-piperidinecarboxylate hydrochloride instead of hydroxylamine hydrochloride, the compound of the present invention having the following physical data was obtained.
TLC : Rf 0.22 (dichloromethane : methanol : ammonia water = 90 : 10 : 1);
$^1$H-NMR : δ 1.71-1.92 (m, 2H), 2.01-2.20 (m, 2H), 2.76-3.18 (m, 4H), 4.32-4.47 (m, 1H), 6.62-8.08 (m, 8H).

Example 25 : methyl 3,5-dichloro-4-[5-(2,4-difluorobenzoyl)-2-oxopyridin-1(2H)-yl]benzoate

To a solution of the compound prepared in Example 23 (1.1 g) in a mixture solution of methanol (5 mL) and N, N-dimethylformamide (5 mL) was added triethylamine (1.66 mL) and the solution was deaerated with argon. 1,1'-Bis (diphenylphosphino)ferrocene (137 mg) and palladium acetate (54 mg) were added thereto and the solution was stirred at 90 °C for 3 hours under an atmosphere of carbon monoxide gas. Ethyl acetate was added thereto. The organic layer was washed with 1N hydrochloric acid, an aqueous saturated sodium bicarbonate solution and brine sequentially, dried and concentrated. The obtained residue was purified by column chromatography on silica gel (ethyl acetate : hexane = 1 : 4 → 3 : 7 → 2 : 3) to give the compound of the present invention (666 mg) having the following physical data.
TLC : Rf 0.22 (ethyl acetate : hexane = 1 : 4);
$^1$H-NMR : δ 8.14 (s, 2H), 8.01 (m, 1H), 7.67-7.55 (m, 2H), 7.01 (m, 1H), 6.91 (m, 1H), 6.75 (d, J=9.9Hz, 1H), 3.97 (s, 3H).

Example 26 : 4-[5-[[(benzyloxy)imino](2,4-difluorophenyl)methyl]-2-oxo-1 (2H)-pyridinyl]-3,5-dichlorobenzoic acid (the mixture of E-form and Z-form)

To a solution of the compound prepared in Example 25 (55 mg) in pyridine (1.5 mL) was added O-benzylhydroxylamine hydrochloride (100 mg) and the solution was stirred at 100°C for 6 hours. Ethyl acetate was added thereto. The organic solution was washed with 1N hydrochloric acid, an aqueous saturated sodium bicarbonate solution and brine sequentially, dried and concentrated. The obtained residue was purified by preparative thin-layer chromatography (ethyl acetate : hexane = 1 : 4) to give ester (55 mg). To a solution of the obtained ester (52 mg) in a mixture solution of tetrahydrofuran (1 mL) and methanol (0.5 mL) was added 2N aqueous sodium hydroxide solution (143 μL) and the solution was stirred at room temperature for 1 hour. The solution was neutralized with 2N hydrochloric acid and extracted with ethyl acetate. The organic layer was washed with brine, dried and concentrated to give the compound of the present invention (50 mg) having the following physical data.
TLC : Rf 0.13 (dichloromethane : methanol = 9 : 1);
$^1$H-NMR: δ 5.20&5.26 (s&s, 2H), 6.67-7.03 (m, 4H), 7.09-7.58 (m, 6H), 8.01&8.02 (s&s, 2H), 7.67&8.11 (dd&dd, J=9.70, 2.5 6Hz&J=9.8 9, 2.56Hz, 1H).

Example 27: 5-[[(benzyloxy)imino](2,4-difluorophenyl)methyl]-1-[2,6-dichloro-4-(hydroxymethyl)phenyl]-2(1H)-pyridinone (the mixture of E-form and Z-form)

To a solution of the compound prepared in Example 26 (325 mg) in N,N-dimethylformamide (5 mL) were added N-hydroxysuccinimide (106 mg) and I-ethyl-3-[3-(dimethylamino)propyl]carbodiimide (EDC) hydrochloride (340 mg) and the solution was stirred at room temperature for 1 hour. Ethyl acetate was added thereto. The organic layer was washed with water and brine sequentially, dried and concentrated. To a solution of the obtained residue in tetrahydrofuran (5

mL) was added a solution of sodium borohydride (70 mg) in water (0.5 mL) at 0 °C. The solution was stirred at 0 °C for 1 hour. Water was added thereto and the solution was extracted with ethyl acetate. The organic layer was washed with 1N hydrochloric acid, an aqueous saturated sodium bicarbonate solution and brine sequentially, dried and concentrated. The obtained residue was purified by column chromatography on silica gel (ethyl acetate : hexane = 1 : 4 → 3 : 7 → 2 : 3) to give the compound of the present invention (3 06 mg) having the following physical data.
TLC : Rf 0.60 (ethyl acetate : hexane = 4:1);
$^1$H-NMR: δ 2.20-2.42 (m, 1H), 4.65&4.67 (s&s, 2H), 5.19&5.26 (s&s, 2H), 6.59-6.99 (m, 3H), 7.10-7.55 (m, 9H), 7.64&8.04 (m&m, 1H).

Example 28 : 5-[[(benzyloxy)imino](2,4-difluorophenyl)methyl]-1-{2,6-dichloro-4-[(dimethylamino)methyl]phenyl}-2(1H)-pyridinone (the mixture ofE-form and Z-form)

To a solution of the compound prepared in Example 27 (61 mg) in dichloromethane (1 mL) were added triethylamine (33 μL) and methanesulfonyl chloride (18 μL) at 0 °C. After stirring for 1 hour, ethyl acetate was added thereto. The organic layer was washed with 1N hydrochloric acid, an aqueous saturated sodium bicarbonate solution and brine sequentially, dried and concentrated. To a solution of the obtained residue in tetrahydrofuran (0.5 mL) was added 50 % aqueous dimethylamine solution (531 μL) at room temperature and the solution was stirred for 30 minutes. Ethyl acetate was added thereto. The organic layer was washed with water and brine sequentially, dried and concentrated. The obtained residue was purified by preparative thin-layer chromatography (ethyl acetate : hexane = 19 : 1) to give the compound of the present invention (55 mg) having the following physical data.
TLC : Rf 0.33 (ethyl acetate);
$^1$H-NMR : δ 2.26&2.27 (s&s, 6H), 3.39&3.41 (s&s, 2H), 5.19&5.26 (s&s, 2H), 6.63&6.71 (d&d, J=9.70Hz&J=9.89Hz, 1H), 6.76&7.54 (d&d, J=2.56Hz&J=2.56Hz, 1H), 6.80-7.02 (m, 2H), 7.12-7.39 (m, 6H), 7.39-7.51 (m, 2H), 7.61&8.02 (dd&dd, J=9.70, 2.56Hz&J=9.89, 2.56Hz, 1H).

Example 28(1) : 5-[[(benzyloxy)imino](2,4-difluorophenyl)methyl]-1-{2,6-dichloro-4-[(4-methyl-1-piperadinyl)methyl]phenyl}-2(1H)-pyridinone (the mixture of E-form and Z-form)

By the same procedure as a series of reactions of Example 28 using N-methylpiperadine instead of 50 % aqueous dimethylamine solution, the compound of the present invention having the following physical data was obtained.
TLC : Rf 0.28 (dichloromethane : methanol = 9 : 1);
$^1$H-NX4R : δ 2.30&2.31 (s&s, 3H), 2.39-2.59 (m, 8H), 3.47&3.48 (s&s, 2H), 5.19&5.26 (s&s, 2H), 6.63&6.71 (d&d, J=9.79Hz&J=9.79Hz, 1H), 6.75&7.52 (d&d, J=2.65Hz&J=2.47Hz, 1H), 6.79-7.01 (m, 3H), 7.12-7.50 (m, 7H), 7.62&8.03 (dd&dd, J=9.79, 2.47Hz&J=9.79, 2.65Hz, 1H).

Example 29 : tert-butyl 4-({[{1-[2,6-dichloro-4-(methoxycarbonyl)phenyl]-6-oxo-1,6-dihydropyridine-3-yl}(2,4-difluorophenyl)methylene]amino}oxy)piperidine-1 - carboxylate

By the same procedure as a series of reactions of Example 7 using the compound prepared in Example 25 instead of the compound prepared in Example 6(1) and tert-butyl 4-(aminooxy)-1-piperidinecarboxylate hydrochloride instead of hydroxylamine hydrochloride, the compound of the present invention having the following physical data was obtained.
TLC : Rf 0.31 (ethyl acetate : hexane = 3 : 7);
$^1$H-NMR: δ 8.15-6.64 (m, 8H), 4.39 (m, 1H), 3.97&3.95 (s&s, 3H), 3.75-3.15 (m, 4H), 2.04-1.80 (m, 2H), 1.78-1.60 (m, 2H), 1.46&1.45 (s&s, 9H).

Example 30 : methyl 3,5-dichloro-4-[5-{(2,4-difluorophenyl)[(4-piperidinyloxy)imino]methyl}-2-oxo-1(2H)-pyridinyl]benzoate (the mixture of E-form and Z-form)

By the same procedure as a series of reactions of Example 13 using the compound prepared in Example 29 instead of the compound prepared in Example 12, the compound of the present invention having the following physical data was obtained.
TLC : Rf 0.25 (dichloromethane : methanol: ammonia water = 90 : 10 : 1);
$^1$H-NMR : δ 1.61-1.76 (m, 2H), 1.94-2.12 (m, 2H), 2.68-2.83 (m, 2H), 2.93-3.13 (m, 2H), 3.95&3.97 (s&s, 3H), 4.28-4.40 (m, 1H), 6.63-8.14 (m, 8H).

Example 31 : 4-[5-[({[1-(tert-butoxycarbonyl)piperidin-4-yl]oxo}imino)(2,4-difluorophenyl)methyl]-2-oxopyridin-1(2H)-yl]-3,5-dichlorobenzoic acid

To a solution of the compound prepared in Example 29 (258 mg) in a mixture solution of tetrahydrofuran (2 mL) and methanol (1 mL) was added 2N aqueous sodium hydroxide solution (609 μL) and the solution was stirred at room temperature for 1 hour. The solution was neutralized with 2N hydrochloric acid and extracted with ethyl acetate the organic layer was washed with brine, dried and concentrated to give the compound of the present invention (252 mg) having the following physical data.
TLC : Rf 0.13 (dichloromethane : methanol =9:1);
$^1$H-NMR: δ 8.13&7.64 (dd&dd, J=9;9, 2.4Hz&9.5, 2.4Hz, 1H), 8.07&8.04 (s&s, 2H), 7.61-6.71 (m, 5H), 4.40 (m, 1H), 3.73-3.17 (m, 4H), 2.05-1.80 (m, 2H), 1.76-1.60 (m, 2H), 1.46& 1.45 (s&s, 9H).

Example 32 : tert-butyl 4-({[{1-[2,6-dichloro-4-(hydroxynethyl)phenyl]-6-oxo-1,6-dihydropyridin-3-yl}(2,4-difluorophenyl) methylene]amino}oxy)piperidine-1-carboxylate

By the same procedure as a series of reactions of Example 27 using the compound prepared in Example 31 instead of the compound prepared in Example 26, the compound of the present invention having the following physical data was obtained.
TLC: Rf0.31 (dichloromethane : methanol = 9 : 1);
$^1$H-NMR: δ 8.10-6.63 (m, 8H), 4.72&4.69 (s&s, 2H), 4.40 (m, 1H), 3.69-3.17 (m, 4H), 2.03-1.80 (m, 2H), 1.79-1.60 (m, 2H), 1.46&1.45 (s&s, 9H).

Example 33 : 1-[2,6-dichloro-4-(hydroxymethyl)phenyl]-5-{(2,4-difluorophenyl)[(4-piperidinyloxy)imino]methyl}-2 (1H)-pyridinone (the mixture of E-form and Z-form)

By the same procedure as a series of reactions of Example 13 using the compound prepared in Example 32 instead of the compound prepared in Example 12, the compound of the present invention having the following physical data was obtained.
TLC : Rf 0.17 (dichloromethane : methanol: ammonia water = 90 : 10 : 1);
$^1$H-NMR : δ 1.63-1.82 (m, 2H), 1.93-2.33 (m, 2H), 2.71-2.85 (m, 2H), 2.92-3.11 (m, 2H), 4.30-4.40 (m, 1H), 4.69&4.71 (s&s, 2H), 6.65&6.74 (d&d, J=9.79Hz&J=9.79Hz, 1H), 6.77&7.66 (d&d, J=2.47Hz&J=2.65Hz, 1H), 6.82-6.99 (m, 2H), 7.15-7.52 (m, 3H), 7.56&8.07 (dd&dd, J=9.79, 2.65Hz&J=9.79, 2.47Hz, 1H).

Example 34 : 1-{2,6-dichloro-4-[(dimethylamino)methyl]phenyl}-5-{(2,4-difluorophenyl)[(4-piperidinyloxy)imino]methyl}-2(1H)-pyridinone dihydrochloric acid (the mixture of E-form and Z-form)

By the same procedure as a series of reactions of Example 28 → Example 13 using the compound prepared in Example 32 instead of the compound prepared in Example 27, the compound of the present invention having the following physical data was obtained.
TLC : Rf 0.41 (dichloromethane : methanol: ammonia water = 40 : 10 : 1);
$^1$H-MMR($CD_3OD$) : δ 1.93-2.30 (m, 4H), 2.89&2.91 (s&s, 6H), 3.04-3.34 (m, 4H), 4.36&4.38 (s&s, 2H), 4.50-4.59 (m, 1H), 6.73&6.78 (d&d, J=9.70Hz&J=9.70Hz, 1H), 7.00-7.15 (m, 2H), 7.23&7.70 (d&d, J=2.56Hz&J=2.56Hz, 1H), 7.29-7.39&7.52-7.63 (m&m, 1H), 7.77&7.81 (s&s, 2H), 7.95&8.23 (dd&dd, J=9.70, 2.56Hz&J=9.70, 2.55Hz, 1H).

Example 35 : 1-[2,6-dichloro-4-(methoxymethyl)phenyl]-5-{(2,4-difluorophenyl)[(4-piperidinyloxy)imino]methyl}-2 (1H)-pyridinone (the mixture of E-form and Z-form)

To a solution of the compound prepared in Example 32 (53 mg) in acetonitrile (1 mL) were added methyl iodide (605 μL) and silver oxide (300 mg) and the solution was light-shielded and stirred for 3 days. The insoluble matter was filtrated and the filtrate was concentrated. The obtained residue was purified by preparative thin-layer chromatography (ethyl acetate : hexane = 2 : 3) to give ether (49 mg). To a solution of the obtained ether in ethyl acetate (1 mL) was added 4N hydrogen chloride/ethyl acetate solution (1 mL) at room temperature and the solution was stirred at room temperature for 2 hours. Ethyl acetate was added thereto. The organic layer was washed with an aqueous saturated sodium bicarbonate solution and brine sequentially, dried and concentrated to give the compound of the present invention (33 mg) having the following physical data.
TLC : Rf 0.42 (dichloromethane : methanol : ammonia water = 90 : 10 : 1);
$^1$H-NMR: δ 1.62-1.81 (m, 2H), 1.94-2.13 (m, 2H), 2.68-2.84 (m, 2H), 2.94-3.14 (m, 2H), 3.43&3.45 (s&s, 3H), 4.28-4.40 (m, 1H), 4.43&4.46 (s&s, 2H), 6.63-8.10 (m, 8H).

Example 36 : 1-(2-chloro-4-fluorophenyl)butane-1,3-dione

To a emulsion of sodium hydride (60 % in oil, 600 mg) in ethyl acetate (1.47 mL) and tetrahydrofuran (16 mL) were added 2-chloro-4-fluoroacetophenone (1.73 g) in tetrahydrofuran (8 mL) and then dibenzo-18-crown-6 (50 mg) in tetrahydrofuran (8 mL) and the solution was heated under reflux for 4 hours. The reaction solution was poured into 1N hydrochloric acid-ice and the solution was extracted with ethyl acetate. The organic layer was washed with water and brine sequentially, dried and concentrated. The obtained residue was purified by preparative medium pressure liquid chromatograph : W-prep 2XY (column : main column 3L, inject column L ; auto condition setting : hexane : ethyl acetate = 19 : 1, Rf 0.40, separation mode GR) to give the title compound (431 mg) having the following physical data.
TLC : Rf 0.26 (ethyl acetate hexane = 1: 19);
$^1$H-NMR : δ 7.62 (dd, J=8.7, 6.0Hz, 1H), 7.18 (dd, J=8.7, 2.4Hz, 1H), 7.05 (ddd, J=8.7, 7.5, 2.4Hz, 1H), 6.04 (s, 1H), 2.19 (s, 3H).

Example 37 : 1-(2-chloro-4-fluorophenyl)-3-[(2,6-dichlorophenyl)amino]but-2-en-1-one

To a solution of the compound prepared in Example 36 (431 mg) in toluene (5 mL) were added 2,6-dichloroaniline (326 mg) and p-toluenesulfonic acid monohydrate (4 mg) at room temperature and the solution was heated under reflux for 7 hours. The reaction solution was concentrated. The obtained residue was purified by preparative medium pressure liquid chromatograph : W-prep 2XY (column : main column 3L, inject column L ; auto condition setting : hexane : ethyl acetate = 19 : 1, Rf 0.20, separation mode GR) to give the title compound (440 mg) having the following physical data.
TLC : Rf 0.18 (ethyl acetate : hexane = 1 : 19);
$^1$H-NMR: δ 12.35 (s, 1H), 7.59 (dd, J=8.7, 6.0Hz, 1H), 7.43 (d, J=8.1Hz, 2H), 7.28-7.20 (m, 1H), 7.15 (dd, J=8.7, 2.4Hz, 1H), 7.02 (ddd, J=8.7, 7.8, 2.4Hz, 1H), 5.69 (s, 1H), 1.87 (s, 3H).

Example 38 : 5-(2-chloro-4-fluorobenzoyl)-1-(2,6-dichlorophenyl)-6-methyllpyridin-2(1H)-one

To a solution of the compound prepared in Example 37 (200 mg) in tetrahydrofuran (3 mL) was added sodium hydroxide (220 mg) and the solution was stirred at 50 °C for 2 hours. Methyl propiolic acid (75 μL) was added thereto and the solution was stirred 50 °C for 4 hours. To the reaction solution was added ethyl acetate. The organic layer was washed with 1N hydrochloric acid and brine sequentially, dried and concentrated. The obtained residue was purified by preparative thin-layer chromatography (hexane : ethyl acetate = 2 : 1) to give the compound of the present invention (23 mg) having the following physical data.
TLC : Rf 0.32 (ethyl acetate : hexane = 1:2);
$^1$H-NMR : δ 7.57-7.48 (m, 3H), 7.46-7.38 (m, 2H), 7.21 (dd, J=8.4, 2.4Hz, 1H), 7.17-7.08 (m, 1H), 6.51 (d, J=9.6Hz, 1H), 2.29 (s, 3H).

Example 39 : 5-[(2-chloro-4-fluorophenyl)(hydraxyimino)methyl]-1-(2,6-dichlorophenyl)-6-methyl-2(1H)-pyridinone (the mixture of E-form and Z-form)

By the same procedure as a series of reactions of Example 7 using the compound prepared in Example 38 instead of the compound prepared in Example 6(1), the compound of the present invention having the following physical data was obtained.
TLC : Rf 0.33 (ethyl acetate : haxane =1:1);
$^1$H-NMR($CD_3OD$) : δ 1.92&2.11 (s&s, 3H), 6.44-6.57 (m, 1H), 7.13-7.72 (m, 7H).

Example 39(1) - Example 39(2)

By the same procedure as a series of reactions of Example 7 using the compound prepared in Example 38 instead of the compound prepared in Example 6(1) and a corresponding amine or amine hydrochloride instead of hydroxylamine hydrochloride, the compounds of the present invention having the following physical data were obtained.

Example 39(1) : 5-{(2-chloro-4-fluorophenyl)[(2-hydroxyethoxy)imino]methyl}-1-(2,6-dichlorophenyl)-6-methyl-2 (1H)-pyridinone (E form or Z-form : geometrical isomer of a compound of Example 39(2))

TLC : Rf 0.40 (ethyl acetate : hexane =2:1);
$^1$H-NMR($CD_3OD$) : δ 2.13 (s, 3H), 3.72-3.80 (m, 2H), 4.18-4.25 (m, 2H), 6.51 (d, J=9.52Hz, 1H), 7.15-7.25 (m, 1H), 7.33 (dd, J=8.70, 2.47Hz, 1H), 7.38 (d, J=9.52Hz, 1H), 7.48 (dd, J=8.70, 6.13Hz, 1H), 7.53 (dd, J=9.15, 7.14Hz, 1H), 7.61-7.69 (m, 2H).

Example 39(2) : 5-{(2-chloro-4-fluorophenyl)[(2-hydroxyethoxy)imino]methyl}-1-(2,6-dichlorophenyl)-6-methyl-2 (1H)-pyridinone (E form or Z-form : geometrical isomer of a compound of Example 39(1))

TLC : Rf 0.33 (ethyl acetate : hexane = 2 : 1);
$^1$H-NMR($CD_3OD$) : δ 1.93 (s, 3H), 3.77-3.87 (m, 2H), 4.24-4.34 (m, 2H), 6.51 (d, J=9.52Hz, 1H), 7.16-7.25 (m, 1H), 7.24-7.33 (m, 2H), 7.53 (dd, J=9.06, 7.23Hz, 1H), 7.61-7.71 (m, 3H).

Example 40 : 5-{(2-chloro-4-fluorophenyl)[(4-piperidinyloxy)imino]methyl}-1-(2,6-dichlorophenyl)-6-methyl-2(1H)-pyridinone (the mixture of E-form and Z-form)

By the same procedure as a series of reactions of Example 7 → Example 13 using the compound prepared in Example 38 instead of the compound prepared in Example 6(1) and tert-butyl 4-(aminooxy)-1-piperidine carboxylate hydrochloride instead of hydroxylamine hydrochloride, the compound of the present invention having the following physical data was obtained.
TLC: Rf 0.51 (dichloropmethane: methanol: ammonia water = 90 : 10: 1);
$^1$H-NMR: δ 1.75-1.95 (m, 2H), 1.90&2.05 (s&s, 3H), 2.00-2.25 (m, 2H), 2.80-2.99 (m, 2H), 2.99-3.18 (m, 2H), 4.30-4.42 (m, 1H), 6.45-7.60 (m, 8H).

Example 41: methyl (4S)-5-[(2,6-dichlorophenyl)amino]-4-methyl-5-oxopentanoate

To a solution of (R)-(-)-4-methylglutaric acid 1-mono-methyl ester (10.0 g) in dichloromethane (200 mL) was added oxalyl chloride (5.99 mL) and a catalytic amount of N,N-dimethylformamide at room temperature and the solution was stirred for 3 hours. The reaction solution was concentrated to give a desired acid chloride. To a solution of the obtained acid chloride in N,N-dimethylformamide (200 mL) was added 2,6-dichloroaniline (21.2 g) at room temperature and the solution was stirred for 2 hours. The reaction solution was poured into ice water and the solution was extracted with ethyl acetate. The organic layer was washed with brine, dried and concentrated. The obtained residue was purified by preparative medium pressure liquid chromatograph: W-prep 2XY (column : main column 3L, inject column L ; auto condition setting : hexane : ethyl acetate = 2 : 1, Rf 0.35, separation mode GR) to give the title compound (6.62 g) having the following physical data.
TLC : Rf 0.28 (ethyl acetate : hexane =1:2);
$^1$H-NMR : δ 7.37 (d, J=8.1Hz, 2H), 7.25-7.10 (m, 3H), 3.70 (s, 3H), 2.75-2.55 (m, 1H), 2.51 (t, J=7.1Hz, 2H), 2.20-2.00 (m, 1H), 1.90-1.75 (m, 1H), 1.31 (d, J=6.9Hz, 3H).

Example 42 : methyl (4S)-2-(2-chloro-4-fluorobenzyl)-5-[(2,6-dichlorophenyl)amino]-4-methyl-5-oxopentanoate

To a solution of the compound prepared in Example 41 (4.56 g)in tetrahydrofuran (70 mL) was added dropwise 1M lithium hexamethyl disilazide / tetrahydrofuran solution (31.5 mL) at -78 °C and the solution was stirred for 20 minutes. 2-Chloro-4-fluorobenzyl bromide (4.02 g) in tetrahydrofuran (5 mL) was added dropwise thereto and the solution was stirred at -78 °C for 30 minutes. The reaction solution was risen to 0 °C gradually. An aqueous saturated ammonium chloride solution was added to the reaction solution and the solution was extracted with ethyl acetate. The organic layer was washed with brine, dried and concentrated. The obtained residue was purified by preparative medium pressure liquid chromatograph : W-prep 2XY (column : main column 4L, inject column L ; auto condition setting : hexane : ethyl acetate = 2:1, Rf 0.65, separation mode GR) to give the title compound (5.75 g) having the following physical data.
TLC : Rf 0.20 (ethyl acetate: hexane =1:4);
$^1$H-NMR : δ 7.37 (d, J=8.1Hz, 2H), 7.25-7.03 (m, 4H), 6.90 (td, J=8.4, 2.7Hz, 1H), 3.62 (s, 3H), 3.10-2.90 (m, 3H), 2.60-2.42 (m, 1H), 2.40-2.22 (m, 1H), 1.80-1.60 (m, 1H), 1.27 (d, J=7.2Hz, 3H).

Example 43(1) and 43(2) : 5-[(2-chloro-4-fluorophenyl)(hydroxyimino)methyl]-1-(2,6-dichlorophenyl)-3-methyl-2 (1H)-pyridinone (E-form or Z-form)

By the same procedure as a series of reactions of Example 2 → Example 3 → Example 4 → Example 6 → Example 7 using the compound prepared in Example 42 instead of the compound prepared in Example 1, the compounds of the present invention having the following physical data were obtained.

Example 43(1):

TLC : Rf 0.39 (ethyl acetate : hexane = 1 : 2);
$^1$H-NMR($CD_3OD$) : δ 2.17-2.24 (m, 3H), 6.65-6.72 (m, 1H), 7.08-7.20 (m, 1H), 7.21-7.34 (m, 2H), 7.43 (dd, J=9.15,

6.96Hz, 1H), 7.50-7.58 (m, 2H), 8.02-8.09 (m, 1H).

Example 43(2) :

TLC : Rf 0.33 (ethyl acetate : hexane = 1 : 2);
$^1$H-NMR($CD_3OD$):δ 2.10-2.16 (m,3H),7.10-7.21 (m,1H), 7.29 (dd,J= 8.60,2.56Hz,1H),7.43-7.54 (m,2H),7.54-7.63 (m, 3H),7.67-7.72 (m,1H).

Example 44 : 1-(2,6-dichlorophenyl)-5-{(4-fluorophenyl)[(4-piperidinyloxy)imino]methyl}-2(1H)-pyridinone (the mixture of E-form and Z-form)

By the same procedure as a series of reactions of Example 2 → Example 3 → Example 4 → Example 6 → Example 7 → Example 13 using ethyl 2-(4-fluorobenzyl)-5-[(2,6-dimethylphenyl)amino]-5-oxopentanoate instead of the compound prepared in Example 1 and tert-butyl 4-(aminooxy)-I-piperidinecarboxylate instead of hydroxylamine hydrochloride, the compounds of the present invention having the following physical data were obtained.
TLC : Rf 0.50 (dichloromethane : methanol : ammonia water = 90 : 10 : 1);
$^1$H-NMR : δ 1.67-1.88 (m,2H),1.92-2.24 (m,2H),2.73-2.92 (m,2H),2.92-3.18 (m,2H),4.23-4.44 (m, 1H), 6.74 & 6.70 (d & d,J = 6.0Hz & 5.7Hz, 1H), 6.62-8.09 (m,9H).

Example 44(1) : 1-(2,6-dichlorophenyl)-5-{(2-fluorophenyl)[(4-piperidinyloxy)imino]methyl}-2(1H)-pyridinone (the mixture of E-form and Z-form)

By the same procedure as a series of reactions of Example 2 → Example 3 → Example 4 → Example 6 → Example 7 → Example 13 using ethyl 2-(2-fluorobenzyl)-5-[(2,6-dimethylphenyl)amino]-5-oxopentanoate instead of the compound prepared in Example 1 and tert-butyl 4-(aminooxy)-1-piperidinecarboxylate instead of hydroxylamine hydrochloride, the compounds of the present invention having the following physical data were obtained.
TLC : Rf 0.33 (dichloromethane : methanol : ammonia water = 90 : 10 : 1);
$^1$H-NMR : δ 1.58-1.82 (m, 2H), 1.85-2.26 (m, 2H), 2.65-2.88 (m, 2H), 2.89-3.16 (m, 2H), 4.29-4.43 (m, 1H), 6.65&6.74 (dd&dd, J=9.9, 0.6Hz&J=9.3, 1.2Hz, 1H), 6.77-8.16 (m, 9H).

Example 45 : 5-(2,4-difluorobenzoyl)-2H-pyran-2-one

To coumaric acid (24.2 g) was added thionyl chloride (50 mL) and the solution was heated under reflux for 4 hours. The solvent was distilled under a reduced pressure, dried to give an acid chloride (27.0 g). To a mixture of the acid chloride (25.8 g) and 1,3-difluorobenzene (160 mL) was added aluminum chloride (42.7 g) with stirring and the solution was heated under reflux for 3.5 hours. After cooling, the reaction solution was poured into cooled dilute hydrochloric acid and the solution was extracted with ethyl acetate. The organic layer was washed with water, an aqueous saturated sodium bicarbonate solution, water and brine sequentially, dried over anhydrous sodium sulfate and concentrated. The obtained residue was washed with tert-butyl methyl ether : hexane = 1 : 1 and dried to give the title compound (15.4 g) having the following physical data.
TLC : Rf 0.34 (ethyl acetate : hexane = 1 : 2);
$^1$H-NMR: δ 6.43 (d, J=9,7Hz, 1H), 6.89-7.10 (m,2H), 7.54-7.67 (m, 1H), 7.83-7.91 (m, 1H), 7.98-8.06 (m, 1H).

Example 46 : 5-(2,4-difluorobenzoyl)-1-(4-fluoro-2,6-dimethylphenyl)pyridin-2(1H)-one

To a solution of the compound prepared in Example 45 (2.50 g) in N,N-dimethylformamide (21 mL) was added 4-fluoro-2,6-dimethylaniline (3,68 g) and the solution was stirred at 80 °C for 30 minutes. After cooling, the reaction solution was poured into cooled dilute hydrochloric acid and the solution was extracted with a mixture solvent of ethyl acetate and hexane (about 19 : 1). The organic layer was washed with water and brine, dried over anhydrous magnesium sulfate and concentrated. The obtained residue was washed with a mixture solvent of diisopropyl ether and hexane (1 : 3) and dried to give the compound of the present invention (3.12 g) having the following physical data.
TLC : Rf 0.58 (ethyl acetate : hexane = 3 : 2);
$^1$H-NMR : δ 2.09 (s, 6H), 6.75 (d, J=9.9Hz, 1H), 6.84-6.94 (m, 3H), 6.98-7.07 (m, 1H), 7.55-7.69 (m, 2H), 7.96-8.05 (m, 1H).

Example 47 : (Z-form) 5-{(Z)-(2,4-difluorophenyl)[(4-piperidinyloxy)imino]methyl}-1-(4-fluoro-2,6-dimethylphenyl)-2 (1H)-pyridinone; (E-form) 5-{(E)-(2,4-difluorophenyl)[(4-piperidinyloxy)imino]methyl}-1-(4-fluoro-2,6-dimethylphenyl)-2 (1H)-pyridinone

To a solution of the compound prepared in Example 46 (2.17 g) in pyridine (24 mL) were added tert-butyl 4-(aminooxy)-1-piperidineearboxylate (1.71 g) and 4N hydrogen chloride /1,4-dioxane solution (1.5 mL) and the solution was stirred at 100 °C for 8 hours. After cooling, the reaction solution was poured into cooled dilute hydrochloric acid and the solution was extracted with ethyl acetate. The organic layer was washed with water and brine, dried over anhydrous magnesium sulfate and concentrated. The obtained residue was purified by column chromatography on silica gel (ethyl acetate : hexane = 1: 6 - 1 : 2) to give the N-Boc derivative of the present invention (Z-form: 1.25 g, E-form : 0.34 g) having the following physical data.

N-Boc derivative (Z-form):

TLC : Rf 0.32 (ethyl acetate : hexane = 1 : 1);
$^1$H-NMR : δ 1.45 (s, 9H), 1.62-1.75 (m, 2H), 1.79-1.94 (m, 2H), 2.05 (s, 6H), 3.20-3.33 (m, 2H), 3.40-3.54 (m, 2H), 4.31-4.46 (m, 1H), 6.75 (d, J=9.70Hz, 1H), 6.79-6.99 (m, 5H), 7.13-7.23 (m, 1H), 8.03 (dd, J=9.70, 2.74Hz, 1H).

N-Boc derivative (E-form) :

TLC : Rf 0.36 (ethyl acetate : hexane = 1 : 1);
$^1$H-NMR : δ 1.46 (s, 9H), 1.61-1.77 (m, 2H), 1.93-2.03 (m, 2H), 2.06-2.11 (m, 6H), 3.08-3.29 (m, 2H), 3.63-3.81 (m, 2H), 4.30-4.45 (m, 1H), 6.67 (d, J=9.70Hz, 1H), 6.81-7.00 (m, 4H), 7.16-7.23 (m, 1H), 7.47-7.54 (m, 1H), 7.56-7.66 (m, J=9.33, 2.38Hz, 1H).

Then, to a solutioin of the obtained N-Boc derivative (Z-form : 1.25 g and E-form : 0.34 g) in ethyl acetate (4.5 mL) was added 4N hydrogen chloride / ethyl acetate solution (4.5 mL) under ice bath and the solution was stirred at room temperature for 1.5 hours. The reaction solution was poured into cooled sodium hydrogen carbonate solution and the solution was extracted with ethyl acetate. The organic layer was washed with water and brine, dried over anhydrous magnesium sulfate and concentrated to give the compound of the present invention (Z-form : 0.97g, E-form : 0.25g) having the following physical data.

(Z-form) :

TLC : Rf 0.11 (dichloromethan : methanol : ammonia water = 90 : 10 : 1);
$^1$H-NMR : δ 1.38-1.84 (m, 3H), 1.87-2.01 (m, 2H), 2.05 (s, 6H), 2.63-2.77 (m, 2H), 2.91-3.05 (m, 2H), 4.22-4.38 (m, 1H), 6.74 (d, J=9.79Hz, 1H), 6.80 (d, J=2.65Hz, 1H), 6.82-7.00 (m, 4H), 7.13-7.25 (m, 1H), 8.03 (dd, J=9.79, 2.65Hz, 1H).

(E-form) :

TLC : Rf 0.26 (ethyl acetate : methanol: triethylamine = 6 : 3 : 1);
$^1$H-NMR: δ 1.59-1.78 (m, 2H), 1.89-2.22 (m, 8H), 2.69-2.86 (m, 2H), 2.90-3.19 (m, 3H), 4.23-4.44 (m, 1H), 6.67 (d, J=9.70Hz, 1H), 6.79-7.02 (m, 4H), 7.35-7.66 (m, 3H).

Example 48 : 5-(Z)-(2,4-difluorophenyl){[(1-methyl-4-piperidinyl)oxy]imino}methyl)-1-(4-fluoro-2,6-dimethylphenyl)-2 (1H)-pyridinone

To a solution of the compound (Z-form) prepared in Example 47 (410 mg) in methanol (9.0 mL) was added 37 % formaldehyde solution (0.13 mL) and the solution was stirred under water bath. Sodium borohydride (68 mg) was added to the solution, which was stirred for 10 minutes. The reaction solution was poured into ice water. The solution was neutralized with 1N hydrochloric acid and extracted with ethyl acetate. The organic layer was washed with water and brine, dried over anhydrous magnesium sulfate and concentrated. The organic residue was purified by column chromatography on silica gel (ethyl acetate : methanol: triethylamine = 1 : 0 : 0 - 20 : 1 : 1) to give the compound of the present invention (82 mg) having the following physical data.
white crystal (melting point : 172 - 173 °C)
TLC : Rf 0.35 (dichloromethane : methanol : ammonia water = 90 : 10 : 1),
$^1$H-NMR : δ 1.70-1.86 (m, 2H), 1.89-2.10 (m, 8H), 2.18-2.38 (m, 5H), 2.44-2.62 (m, 2H), 4.17-4.31 (m, 1H), 6.74 (d, J=9.70Hz, 1H), 6.80 (d, J=2.56Hz, 1H), 6.82-6.99 (m, 4H), 7.13-7.26 (m, 1H), 8.03 (dd, J=9.70, 2.56Hz, 1H).

Example 49 : 5-((Z)-(2,4-difluorophenyl){[(1-ethyl-4-piperidinyl)oxy]imino}methyl)-1-(4-fluoro-2,6-dimethylphenyl)-2 (1H)-pyridinone

To a solution of the compound (Z-form) prepared in Example 47 (105 mg) in N,N-dimethylformamide (2 mL) were added ethyl bromide (21 μL) and potassium carbonate (159 mg) and the solution was stirred for 4 hours. The reaction mixture was poured into ice water and extracted with ethyl acetate. The organic solution was washed with water and brine, dried over anhydrous sodium sulfate and concentrated. The obtained residue was purified by preparative thin-layer chromatography (dichloromethane : methanol : ammonia water = 1 : 4) to give the compound of the present invention (58 mg) having the following physical data. TLC : Rf 0.35 (dichloromethane : methanol : ammonia water = 90 : 10 : 1);
$^1$H-NMR : δ 1.08 (t, J=7.14Hz, 3H), 1.68-1.84 (m, 2H), 1.90-2.08 (m, 8H), 2,13-2.31 (m, 2H), 2.39 (q, J=7.14Hz, 2H), 2.51-2.66 (m, 2H), 4.19-4.31 (m, 1H), 6.74 (d, J= 9.70Hz, 1H), 6.80 (d, J=2.56Hz, 1H), 6.82-6.98 (m, 4H), 7.12-7.25 (m, 1H), 8.03 (dd, J=9.70, 2.56Hz, 1H).

Example 50 : 5-((E)-(2,4-difluorophenyl){[(1-methyl-4-piperidinyl)oxy]imino} methyl)-1-(4-fluoro-2,6-dimethylphenyl)-2 (1H)-pyridinone

By the same procedure as series of reactions of Example 48 using the compound prepared in Example 47 (E-form) instead of the compound prepared in Example 47 (Z-form), the compound of the present invention having the following physical data was obtained.
TLC : Rf 0.53 (ethyl acetate: methanol : triethylamine =8:1:1);
$^1$H-NMR: δ 1.72-1.89 (m, 2H), 1.97-2.15 (m, 8H), 2.17-2.34 (m, 5H), 2.50-2.67 (m, 2H), 4.20-4.34 (m, 1H), 6.65 (d, J=8.97Hz, 1H), 6.78-6.98 (m, 4H), 7.41-7.57 (m, 2H), 7.66 (d, J=2.38Hz, 1H).

Example 51(1) - Example 51(16)

By the same procedure as a series of reactions of Example 46 → Example 47 using 4-fluoro-2,6-dimethylaniline or a corresponding amine instead of 4-fluoro-2,6-dimethylaniline, and tert-butyl 4-(aminooxy)-1-piperidinecarboxylate or a corresponding amine instead oftert-butyl 4-(aminooxy)-1-piperidinecarboxylate, the compounds of the present invention having the following physical data were obtained. The geometrical isomer due to double bond (E/Z) has not been decided.

Example 51(1) : 1-(2-chlorophenyl)-5-{(2,4-difluorophenyl)[(4-piperidinyloxy)imino]methyl}-2(1H)-pyridinone (the mixture of E-form and Z-form)

TLC : Rf 0.30 (dichloromethane : methanol : ammonia water = 90 : 10 : 1);
$^1$H-NMR : δ 1.77-2.01 (m, 2H), 2.06-2.26 (m, 2H), 2.73-3.26 (m, 4H), 4.27-4.53 (m, 1H), 6.65&6.72 (dd&dd, J=9.6, 0.9Hz&9.9, 0.6Hz, 1H), 6.78-8.11 (m, 9H).

Example 51(2) : 1-(2,6-difluorophenyl)-5-{(2,4-difluorophenyl)[(4-piperidinyloxy)imino]methyl}-2(1H)-pyridinone (the mixture of E-form and Z-form)

TLC : Rf 0.36 (dichloromethane : methanol : acetic acid = 10 : 2 : 1);
$^1$H-NMR : δ 8.02-6.84 (m, 8H), 6.68 (m, 1H), 4.35 (m, 1H), 3.15-2.70 (m, 4H), 2.40-1.40 (m, 4H), 1.80 (brs, 1H).

Example 51(3) : 5-{(2,4-difluorophenyl)[(4-piperidinyloxy)imino]methyl}-1-(2-methoxy-6-methylphenyl)-2(1H)-pyridinone (the mixture of E-form and Z-form)

TLC : Rf 0.10 (dichloromethane : methanol : ammonia water = 90 : 10 : 1);
$^1$H-NMR: δ 1.53-2.06 (m, 4H), 2.08&2.12 (s&s, 3H), 2.64-2.79 (m, 2H), 2.91-3.15 (m, 2H), 3.74&3.77 (s&s, 3H), 4.23-4.37 (m, 1H), 6.64&6.71 (dd&dd, J=9.70, 0,73Hz&J=9.70, 0.55Hz, 1H), 6.80-8.04 (m, 8H).

Example 51(4): 1-(2,6-diethylphenyl)-5-{(2,4-difluorophenyl)[(4-piperidinyloxy)imino]methyl}-2(1H)-pyridinone (the mixture of E-form and Z-form)

TLC : Rf 0.27 (ethyl acetate: methanol : triethylamine = 6 : 3 : 1);
$^1$H-NMR: δ 1.05-1.18 (m, 6H), 1.49-1.68 (m, 3H), 1.89-2.11 (m, 2H), 2.24-2.50 (m, 4H), 2.57-2.75 (m, 2H), 2.92-3.11 (m, 2H), 4.22-4.36 (m, 1H), 6.63-8.09 (m, 9H).

Example 51(5) : 1-(2-chloro-6-methylphenyl)-5-{(2,4-difluorophenyl)[(4-piperidinyloxy)imino]methyl}-2(1H)-pyridinone (the mixture of E-form and Z-form)

TLC : Rf 0.24 (ethyl acetate : methanol : triethylamine = 6 : 3 : 1);
$^1$H-NMR : δ 1.49-1.72 (m, 3H), 1.87-2.10 (m, 2H), 2.11-2.20 (m, 3H), 2.59-2.77 (m, 2H), 2.92-3.13 (m, 2H), 4.24-4.38 (m, 1H), 6.63-8.09 (m, 9H).

Example 51(6) : 5-{(2,4-difluorophenyl)[(4-piperidinyloxy)imino]methyl}-1-(2-ethyl-6-methylphenyl)-2(1H)-pyridinone (the mixture of E-form and Z-form)

TLC : Rf 0.24 (dichloromethane : methanol : ammonia water = 90 : 10 : 1);
$^1$H-NMR: δ 1.06-1.18 (m, 3H), 1.53-1.81 (m, 2H), 1.95-2.17 (m, 5H), 2.19-2.59 (m, 3H), 2.68-2.85 (m, 2H), 2.93-3.14 (m, 2H), 4.24-4.41 (m, 1H), 6.67&6.75 (dd&dd, J=9.61, 0.82Hz&J=9.70, 0.55Hz, 1H), 6.82-8.07 (m, 8H).

Example 51(7) : 5-{(2,4-difluorophenyl)[(4-piperidinyloxy)imino]methyl}-1-(2,6-diimethaxyphenyl)-2(1H)-pyridinone (the mixture of E-form and Z-form)

TLC : Rf 0.22 (dichloromethane : methanol : ammonia water = 90 : 10 : 1);
$^1$H-NMR : δ 1.66-1.86 (m, 2H), 1.95-2.17 (m, 2H), 2.72-2.91 (m, 2H), 2.93-3.16 (m, 2H), 3.76&3.79 (s&s, 6H), 4.28-4.42 (m, 1H), 6.57-8.01 (m, 9H).

Example 51(8) : 5-((2,4-difluorophenyl){[(4-piperidinyl)oxy]imino}methyl)-1-(2-isopropyl-6-methylphenyl)-2(1H)-pyridinone (the mixture of E-form and Z-form)

Example 51(9) : 5-((2,4-difluorophenyl){[(4-piperidinyl)oxy]imino}methyl)-1-mesityl-2(1H)-pyridinone (the mixture of E-form and Z-form)
TLC : Rf 0.22 (dichloromethane : methanol : ammonia water = 90 : 10 : 1);
$^1$H-NMR : δ 1.64-1.86 (m, 2H), 1.92-2.45 (m, 12H), 2.69-3.18 (m, 4H), 4.30-4.41 (m, 1H), 6.64-8.07 (m, 8H).

Example 51(10) : 5-((2,4-difluorophenyl){[(4-piperidinyl)oxy]imino}methyl)-1-(2,6-diiisopropylphenyl)-2(1H)-pyridinone (the mixture of E-form and Z-form)

Example 51(11) : 5-((2,4-difluorophenyl){[(4-piperidinyl)oxy]imino}methyl)-1-(2-methoxyphenyl)-2(1H)-pyridinone (the mixture of E-form and Z-form)

Example 51(12) : 2'-chloro-5-((2,4-difluorophenyl){[(4-piperidinyl)oxy]imino}methyl)-4'-methyl-2H-1,3' -bipyridin-2-one (the mixture of E-form and Z-form)

Example 51(13) : 5-((2,4-difluorophenyl){[(4-piperidinyl)oxy]imino}methyl)-1-(1-phenylethyl)-2(1H)-pyridinone (the mixture of E-form and Z-form)

Example 51(14) : 5-{(2,4-difluorophenyl)[(4-piperidinyloxy)imino]methyl}-1-phenyl-2(1H)-pyridinone (E form or Z-form)

TLC : Rf 0.30 (dichloromethane : methanol: ammonia water = 90 : 10: 1);
$^1$H-NMR: δ 1.51-1.75 (m, 2H), 1.90-2.04 (m, 2H), 2.61-2.82 (m, 2H), 2.85-3.07 (m, 2H), 4.21-4.39 (m, 1H), 6.68 (d, J=9.70Hz, 1H), 6.83-7.00 (m, 2H), 7.09-7.36 (m, 4H), 7.35-7.57 (m, 3H), 7.92 (dd, J=9.70, 2.56Hz, 1H).

Example 51(15) : 5-{(2,4-difluorophenyl)[(4-piperidinyloxy)imino]methyl}-1-(2-methylphenyl)-2(1H)-pyridinone (E form or Z-form)

TLC : Rf 0.25 (dichloromethane : methanol : ammonia water = 90 : 10 : 1);
$^1$H-NMR : δ 1.60-1.79 (m, 2H), 1.91-2.07 (m, 2H), 2.13 (s, 3H), 2.71-2.86 (m, 2H), 2.90-3.06 (m, 2H), 4.26-4.40 (m, 1H), 6.70 (d, J=9.70Hz, 1H), 6.82-7.04 (m, 3H), 7.09-7.24 (m, 2H), 7.23-7.40 (m, 3H), 7.97 (dd, J=9.70, 2.56Hz, 1H).

Example 51(16) : 5-{(2,4-difluorophenyl)[(2-pyridinylmethoxy)imino]methyl}-1-(4-fluoro-2,6-dimethylphenyl)-2(1H)-pyridinone (the mixture of E-form and Z-form)

TLC : Rf 0.39 (ethyl acetate);

$^1$H-NMR : δ 2.03 (s, 6H), 5.26-5.38 (m, 2H), 6.61-7.02 (m, 5H), 7.14-8.01 (m, 6H), 8.48-8.61 (m, 1H).

Example 52(1) - Example 52(13)

By the same procedure as a series of reactions of Example 48 using the compound prepared in Example 51 (1) - Example 51(13) instead of the compound prepared in Example 47 (Z-form), the compounds of the present invention having the following physical data were obtained. The geometrical isomer due to double bond (E/Z) has not been decided except for that described clearly.

Example 52(1) : 1-(2-chlorophenyl)-5-((2,4-difluorophenyl){[(1-methyl-4-piperidinyl)oxy]imino}methyl)-2(1H)-pyridinone (the mixture of E-form and Z-form)

TLC : Rf 0.50 (dichloromethane : methanol: ammonia water = 90 : 10 : 1);
$^1$H-NMR : δ 1.61-2.62 (m, 11H), 4.10-4.36 (m, 1H), 6.63&6.70 (d&d, J=9.6&9.9Hz, 1H), 6.78-8.08 (m, 9H).

Example 52(2) : (Z-form) 1-(2,6-difluorophenyl)-5-((Z)-(2,4-difluorophenyl){[(1-methyl-4-piperidinyl)oxy]imino}methyl)-2 (1H)-pyridinone;(E-form) 1-(2,6-difluorophenyl)-5-((E)-(2,4-difluorophenyl){[(1-methyl-4-piperidinyl)oxy]imino}methyl)-2 (1H)-pyridinone

(Z-form) : white amorphous

TLC : Rf 0.52 (ethyl acetate : methanol : triethylamine =8:1.1);
$^1$H-NMR: δ 1.70-1.84 (m, 2H), 1.89-2.01 (m, 2H), 2.15-2.33 (m, 5H), 2.43-2.61 (m, 2H), 4.18-4.31 (m, 1H), 6.70 (d, J=9.9Hz, 1H), 6.83-7.09 (m, 5H), 7.14-7.24 (m, 1H), 7.30-7.48 (m, 1H), 7.99 (dd, J=9.9, 2.6Hz, 1H).

(E-form) : white amorphous

TLC : Rf 0.50 (ethyl acetate : methanol : triethylamine = 8 : 1 : 1);
$^1$H-NMR: δ 1.76-1.91 (m, 2H), 1.98-2.11 (m, 2H), 2.19-2.35 (m, 5H), 2.51-2.66 (m, 2H), 4.23-4.35 (m, 1H), 6.64 (d, J=9.7Hz, 1H), 6.82-6.98 (m, 2H), 7.02-7.12 (m, 2H), 7.36-7.59 (m, 3H), 7.83-7.89 (m, 1H).

Example 52(3): 5-((2,4-difluorophenyl){[(1-methyl-4-piperidinyl)oxy]imino}methyl)-1-(2-methoxy-6-methylphenyl)-2 (1H)-pyridinone (the mixture of E-form and Z-form)

TLC : Rf 0.35 (dichloromethane: methanol : ammonia water = 90 : 10 : 1);
$^1$H-NMR: δ 1.69-1.89 (m, 2H), 1.90-2.15 (m, 5H), 2.18-2.38 (m, 5H), 2.44-2.65 (m, 2H), 3.74&3.77 (s&s, 3H), 4.17-4.32 (m, 1H), 6.63&6.71 (dd&dd, J=9.70, 0.73Hz&J=9.79, 0.64Hz, 1H), 6.80-8.04 (m, 8H).

Example 52(4) : 1-(2,6-diethylphenyl)-5-((2,4-difluorophenyl){[(1-methyl-4-piperidinyl)oxy]imino}methyl)-2(1H)-pyridinone (the mixture ofE-form and Z-form)

TLC : Rf 0.57 (ethyl acetate : methanol : triethylamine = 7 : 2 : 1);
$^1$H-NMR : δ 1.02-1.21 (m, 6H), 1.63-1.85 (m, 2H), 1.86-2.10 (m, 2H), 2.13-2.64 (m, 11H), 4.15-4.33 (m, 1H), 6.61-8.11 (m, 9H).

Example 52(5): 1-(2-chloro-6-methylphenyl)-5-((2,4-difluorophenyl){[(1-methyl-4-piperidinyl)oxy]imino}methyl)-2 (1H)-pyridinone (the mixture of E-form and Z-form)

TLC : Rf 0.56 (ethyl acetate : methanol : triethylamine = 7 : 2 : 1);
$^1$H-NMR : δ 1.49-1.66 (m, 2H), 1.69-1.87 (m, 2H), 1.88-2.08 (m, 2H), 2.09-2.30 (m, 6H), 2.39-2.68 (m, 2H), 4.17-4.33 (m, 1H), 6.61-8.10 (m, 9H).

Example 52(6) : 5-((2,4-difluorophenyl){[(1-methyl-4-piperidinyl)oxy]imino}methyl)-1-(2-ethyl-6-methylphenyl)-2 (1H)-pyridinone (the mixture of E-form and Z-form)

TLC : Rf 0.40 (dichloromethane : methanol : ammonia water = 90 : 10 : 1);
$^1$H-NMR: δ 1.04-1.19 (m, 3H), 1.49-2.14 (m, 7H), 2.19-2.66 (m, 9H), 4.18-4.35 (m, 1H), 6.66&6.75 (d&d, J=9.70Hz&J=9.70Hz, 1H), 6.80-8.08 (m, 8H).

Example 52(7) : 5-((2,4-difluorophenyl){[(1-methyl-4-piperidinyl)oxy]imino}methyl)-1-(2,6-dimethoxyphenyl)-2(1H)-pyridinone (the mixture ofE-form and Z-form)

TLC : Rf 0.37 (dichloromethane : methanol : ammonia water = 90 : 10 : 1);
$^1$H-NMR: δ 1.55-2.12 (m, 4H), 2.14-2.40 (m, 5H), 2.43-2.66 (m, 2H), 3.76&3.79 (s&s, 6H), 4.16-4.34 (m, 1H), 6.56-8.00 (m, 9H).

Example 52(8) : 5-((2,4-difluorophenyl){[(1-methyl-4-piperidinyl)oxy]imino}methyl)-1-(2-isopropyl-6-methylphenyl)-2(1H)-pyridinone (the mixture of E-form and Z-form)

TLC : Rf 0.37 (ethyl acetate : methanol: triethylamine = 18 : 1 : 1);
$^1$H-NMR: δ 1.01-1.26 (m, 6H), 1.68-1.86 (m, 2H), 1.87-2.11 (m, 5H), 2.13-2.30 (m, 5H), 2.41-2.75 (m, 3H), 4.15-4.32 (m, 1H), 6.61-8.12 (m, 9H).

Example 52(9) : 5-((2,4-difluorophenyl){[(1-methyl-4-piperidinyl)oxy]imino}methyl)-1-mesityl-2(1H)-pyridinone (the mixture of E-form and Z-form) white amorphous

TLC : Rf 0.48 (dichloromethane : methanol: ammonia water = 90 : 10 : 1);
$^1$H-NMR : δ 1.69-1.88 (m, 2H), 1.90-2.12 (m, 8H), 2.18-2.38 (m, 8H), 2.43-2.67 (m, 2H), 4.16-4.32 (m, 1H), 6.66&6.74 (d&d, J=9.70Hz&J=9.70Hz, 1H), 6.80-8.07 (m, 7H).

Example 52(10) : 5-((2,4-difluorophenyl){[(1-methyl-4-piperidinyl)oxy]imino}methyl)-1-(2,6-diisopropylphenyl)-2(1H)-pyridinone (the mixture of E-form and Z-form)

TLC : Rf 0.54 (ethyl acetate : methanol : triethylamine = 8 : 1 : 1);
$^1$H-NMR : δ 0.97-1.27 (m, 12H), 1.58-1.86 (m, 2H), 1.88-2.09 (m, 2H), 2.10-2.32 (m, 5H), 2.40-2.71 (m, 4H), 4.15-4.32 (m, 1H), 6.60-8.14 (m, 9H).

Example 52(11): 5-((2,4-difluorophenyl){[(1-methyl-4-piperidinyl)oxy]imino}methyl)-1-(2-methoxyphenyl)-2(1H)-pyridinone (the mixture ofE-form and Z-form)

TLC : Rf 0.31 (ethyl acetate : methanol : triethylamine = 18 : 1 : 1);
$^1$H-NMR : δ 1.71-1.87 (m, 2H), 1.89-2.09 (m, 2H), 2.15-2.37 (m, 5H), 2.41-2.69 (m, 2H), 3.74-3.85 (m, 3H), 4.16-4.36 (m, 1H), 6.55-7.99 (m, 10H).

Example 52(12) : (Z-form) 2'-chloro-5-((Z)-(2,4-difluorophenyl){[(1-methyl-4-piperidinyl)oxy]imino}methyl)-4'-methyl-2H-1,3'-bipyridine-2-one ; (E-form) 2'-chloro-5-((E)-(2,4-difluorophenyl){[(1-methyl-4-piperidinyl)oxy]imino}methyl)-4'-methyl-2H-1,3'-bipyridine-2-one

(Z-form) :

TLC : Rf 0.47 (ethyl acetate : methanol : triethylamine = 8 : 1 : 1);
$^1$H-NMR : δ 1.71-1.85 (m, 2H), 1.88-2.03 (m, 2H), 2.14-2.31 (m, 8H), 2.42-2.59 (m, 2H), 4.17-4.32 (m, 1H), 6.74 (d, J=9.9Hz, 1H), 6.77-6.82 (m, 1H), 6.84-7.02 (m, 2H), 7.16-7.25 (m, 2H), 8.08 (dd, J=9.9, 2.6Hz, 1H), 8.32 (d, J=4.9Hz, 1H).

(E-form) :

TLC : Rf 0.43 (ethyl acetate : methanol : triethylamine = 8 : 1 : 1);
$^1$H-NR4R : δ 1.8 (m, 2H), 2.0 (m, 2H), 2.2 (m, 8H), 2.6 (m, 2H), 4.3 (m, 1H), 6.6 (d, J=9.7Hz, 1H), 6.9 (m, 2H), 7.5 (m, 4H), 8.3 (d, J=5.1Hz, 1H).

Example 52(13) : 5-((2,4-difluorophenyl){[(1-methyl-4-piperidinyl)oxy]imino}methyl)-1-(1-phenylethyl)-2(1H)-pyridinone (the mixture of E-form and Z-form)

TLC : Rf 0.35 (ethyl acetate : methanol : triethylamine = 18 : 1 : 1);
$^1$H-NMR : δ 1.56-1.77 (m, 5H), 1.82-2.03 (m, 2H), 2.08-2.40 (m, 5H), 2.40-2.72 (m, 2H), 4.07-4.24 (m, 1H), 6.24-8.00 (m, 12H).

Example 53 : (E-form) 5-((E)-(2,4-difluorophenyl){[(1-ethyl-4-piperidinyl)oxy]imino}methyl)-1-(2,6-dimethylphenyl)-2 (1H)-pyridinone; (Z-form) 5-((Z)-(2,4-difluorophenyl) {[(1-ethyl-4-piperidinyl)oxy]imino} methyl)-1-(2,6-dimethylphenyl)-2(1H)-pyridinone

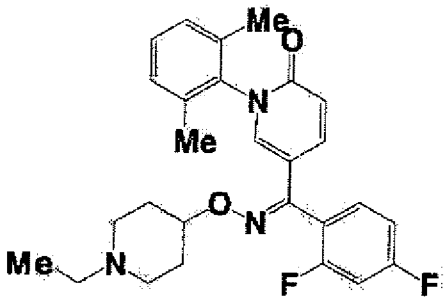

(E)-form

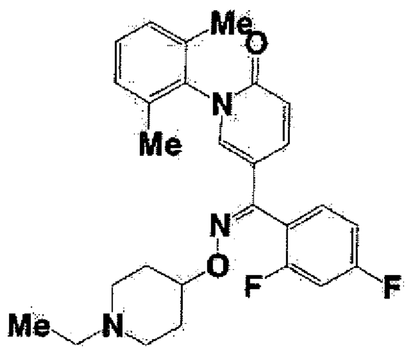

(Z)-form

By the same procedure as a series of reactions of Example 47 → Example 49 using the compound prepared in Example 20 instead of the compound prepared in Example 46, the compounds of the present invention having the following physical data were obtained.

(E-form) :

TLC : Rf 0.35 (dichloromethane: methanol : ammonia water = 90 : 10 : 1);
$^1$H-NMR: δ 1.06-1.16 (m, 3H), 1.44-1.71 (m, 2H), 1.74-1.96 (m, 2H), 2.11 (s, 6H), 2.23-2.56 (m, 4H), 2.57-2.77 (m, 2H), 4.23-4.38 (m, 1H), 6.66 (d, J=10.25Hz, 1H), 6.79-6.90 (m, 1H), 6.90-7.01 (m, 1H), 7.13-7.20 (m, 2H), 7.21-7.31 (m, 1H), 7.40-7.53 (m, 1H), 7.54-7.62 (m, 1H), 7.63-7.69 (m, 1H).

(Z-form) : white crystal (melting point: 175-178 °C)

TLC : Rf 0.33 (dichloromethane : methanol : ammonia water = 90 : 10 : 1);
$^1$H-NMR : δ 1.08.-1.31 (m, 3H), 1.45-1.65 (m, 4H), 1.73-2.03 (m, 2H), 2.06 (s, 6H), 2.41-2.79 (m, 4H), 4.35 (m, 1H), 6.75 (d, J=9.70Hz, 1H), 6.85 (d, J=2.56Hz, 1H), 6.87-6.93 (m, 1H), 6.93-6.99 (m, 1H), 7.11-7.28 (m, 4H), 8.02 (dd, J=9.70, 2.56Hz, 1H).

Example 54 : 5-{(2,4-difluorophenyl)[(4-piperidinylmethoxy)imino]methyl}-1-(2,6-dimethylphenyl)-2(1H)-pyridinone (the mixture of E-form and Z-form)

By the same procedure as a series of reactions of Example 47 using the compound prepared in Example 20 instead of the compound prepared in Example 46, and tert-butyl 4-(aminooxymethyl)-1-piperidinecarboxylate instead of tert-butyl 4-(aminooxy)-1-piperidinecarboxylate, the compound of the present invention having the following physical data was obtained. The geometrical isomer due to double bond (E/Z) has not been decided. TLC : Rf 0.15 (dichloromethane : methanol : ammonia water = 90 : 10 : 1);
$^1$H-NMR: δ 1.42-2.28 (m, 11H), 2.64-2.94 (m, 2H), 3.31-3.45 (m, 2H), 4.03&4.09 (d&d, J=6.41Hz&J=6.96Hz, 2H), 6.69&6.76 (d&d, J=9,89Hz&J=9.70Hz, 1H), 6.81-8.04 (m, 8H).

Example 55 : 5-((2,4-difluorophenyl){[(1-methyl-4-piperidinyl)methoxy]imino}methyl)-1-(2,6-dimethylphenyl)-2(1H)-pyridinone (the mixture of E-form and Z-form)

By the same procedure as a series of reactions of Example 48 using the compound prepared in Example 54 instead of the compound prepared in Example 47 (Z-form), the compound of the present invention having the following physical data was obtained. The geometrical isomer due to double bond (E/Z) has not been decided.
TLC : Rf 0.41 (dichloromethane : methanol: ammonia water = 90: 10 : 1);

$^1$H-NMR: δ 1.29-2.12 (m, 13H), 2.33 (s, 3H), 2.86-3.00 (m, 2H), 4.01&4.08 (d&d, J=6.41Hz&J=6.59Hz, 2H), 6.67&6.75 (d&d, J=9.70Hz&J=9.70Hz, 1H), 6.81-8.06 (m, 8H).

Example 56 : the compound (56a) 1-(2,6-dichloroophenyl)-5-(2-fluoro-4-methylbenzoyl)pyridin-2(1H)-one; the compound (56b) 1-(2,6-dichloroophenyl)-5-(4-fluoro-2-methylbenzoyl)pyridin-2(1H)-one

By the same procedure as a series of reactions of Example 45 → Example 46 using 1-fluora-3-methylbenzene instead of 1,3-difluorobenzene and 2,6-dichloroaniline instead of 4-fluoro-2,6-dimethylaniline, the compounds of the present invention having the following physical data were obtained respectively.

The compound (56a):

TLC : Rf 0.41 (hexane : ethyl acetate = 3 : 2);
$^1$H-NMR : δ 2.40 (s, 3H), 6.74 (d, J=9.89Hz, 1H), 6.97 (d, J=10.98Hz, 1H), 7.03-7.11 (m, 1H), 7.33-7.42 (m, 1H), 7.42-7.53 (m, 3H), 7.62-7.68 (m, 1H), 7.99-8.07 (m, 1H).

The compound (56b) :

TLC : Rf 0.47 (hexane : ethyl acetate = 3 : 2);
$^1$H-NMR : δ 2.38 (s, 3H), 6.77 (dd, J=9.70, 0.64Hz, 1H), 6.89-7.05 (m, 2H), 7.31-7.44 (m, 2H), 7.45-7.52 (m, 3H), 8.06 (dd, J=9.70, 2.56Hz, 1H).

Example 57(1) - Example 57(2)

By the same procedure as a series of reactions af Example 47 using the compound (56a) or the compound (56b) prepared in Example 56 instead of the compound prepared in Example 46, the compounds of the present invention having the following physical data were obtained. The geometrical isomer due to double bond (E/Z) has not been decided.

Example 57(1) : 1-(2,6-dichlorophenyl)-5-{(2-fluoro-4-methylphenyl)[(4-piperidinyloxy)imino]methyl}-2(1H)-pyridinone (the mixture of E-form and Z-form)

TLC : Rf 0.39 (chloroform ; methanol : triethylamine = 60 : 10 : 1);
$^1$H-NMR: δ 1.50-1.84 (m, 2H), 1.88-2.13 (m, 2H), 2.37 (s, 3H), 2.57-2.79 (m, 2H), 2.89-3.16 (m, 2H), 4.23-4.39 (m, 1H), 6.64&6.73 (d, J=10.80&9.70Hz, 1H), 6.78-8.17 (m, 8H).

Example 57(2) : 1-(2,6-dichlorophenyl)-5-{(4-fluoro-2-methylphenyl)[(4-piperidinyloxy)imino]methyl}-2(1H)-pyridinone (the mixture of E-form and Z-form)

TLC : Rf 0.26 (chloroform ; methanol : triethylamine = 60 : 10 : 1);
$^1$H-NMR : δ 1.45-1.73 (m, 2H), 1.84-2.13 (m, 2H), 2.18&2.25 (s, 3H), 2.55-2.78 (m, 2H), 2.91-3.13 (m, 2H), 4.15-4.39 (m, 1H), 6.54-8.21 (m, 9H).

Biological Examples

It was proven by the following Examples that the compounds of the present invention have inhibitory activity on p38α MAP kinase and TNF-α production.
The whole operation using the conventional method is performed according to a fundamental biological technique. Also, as shown below, the measurement method of the present invention is a method wherein enhancement of measurement precision and/or improvement of measurement sensitivity were made in order to evaluate the compounds of the present invention. Details of such experimental methods were shown below.

(1) Study on p38α MAP kinase inhibitory activity

Using activation transcription factor-2 (hereinafter abbreviated as ATF-2) which is a substrate for p38α MAP kinase, the inhibitory action of the compounds of the present invention was investigated on the phosphorylation by a recombinant human p38α MAP kinase.

Experimental Method

A kinase buffer (25 mM Tris-HCl (pH 7.5), 5 mM β-glycerophosphate, 2 mM dithiothreitol, 0.1 mM $Na_3VO_4$, 10 mM $MgCl_2$) containing a recombinant human p38α MAP kinase was added to a 384-well plate (5 μL)(6.25 ng protein/ well) for fluorescence measurement. After addition of a kinase buffer (5 μL) containing the compound of the present invention, the resulting mixture was incubated at room temperature for 20 minutes. A substrate mixture (5 μL) of biotinylated ATF2 of 5 μg/mL (Upstate Biotechnology #14-432), adenosine triphosphate (90 μmol/L)(Sigma #FL-AAS) and anti-phosphorylated ATF2 antibody (20-fold dilution)(Cell Signaling Technology #9221L) prepared separately was added thereto, and enzyme reaction was carried out at 30 °C for 30 minutes. After the reaction, Hepes buffer (5 μL) containing 0.25 % BSA and 100 mM EDTA was added to stop the enzyme reaction. The amount of a complex of the phosphorylated ATF2 and anti-phosphorylated ATF2 antibody produced by the reaction was measured using an Alpha Screen™ Rabbit Detection kit (Packard #6760607).
The p38α MAP kinase inhibitory activity, which is the effect of the compound of the present invention, was calculated as an inhibition rate (%) according to the following equation:

$$\text{Inhibition rate (\%)} = \{(A_C - A_X) / (A_C - A_B)\} \times 100$$

wherein
$A_B$ is a measured value without addition of the enzyme;
$A_C$ is a measured value with addition of the enzyme in the absence of a test compound; and
$A_X$ is a measured value with addition of the enzyme in the presence of a test compound.
Inhibition rate of compounds with each concentration was calculated, and a value indicating 50 % inhibition ($IC_{50}$) was determined from the inhibition curve.
As a result, it was confirmed that the compound of the present invention has p38 MAP kinase inhibitory activity. For example, the $IC_{50}$ values of the compounds in Examples 7, 48 and 52(9) were 4.9 nM, 34 nM, 20.5 nM and 3.8 nM, respectively.

(2) Inhibitory activity against TNF-α production using human cell lines

Using THP-1 which is a human monocyte cell line, the inhibitory effect of the compound of the present invention against TNF-α production stimulated by lipopolysaccharide (LPS) was studied.

Experimental Method

Each 50 μL of lipopolysaccharide (LPS; Difco #3120-25-0) prepared to a concentration of 40 ng/mL using RPMI-1640 medium containing 10 % fetal calf serum (hereinafter abbreviated to RPMI-1640) and RPMI-1640 containing the compound of the present invention was added to a 96-well plate for cell culture. One hundred μL of the cell suspension of THP-1 (Dainippon Pharmaceutical Co., Ltd, #06-202) prepared to a cell density of 2 x $10^6$ cells/mL using RPMI-1640 was added and cultured for 90 minutes at 37 °C in an incubator (5 % $CO_2$, 95 % air). After completion of the reaction, the culture medium supernatant was recovered and the amount of produced TNF-α was measured using an ELISA kit (Invitrogen, #850090192).
The inhibitory activity against TNF-α production, which is the effect of the compound of the present invention, was calculated as an inhibition rate (%) by the following equation:

$$\text{Inhibition rate (\%)} = \{(A_C - A_X) / (A_C - A_B)\} \times 100$$

wherein
$A_B$ is a measured value without LPS induction;
$A_C$ is a measured value with LPS induction in the absence of a test compound; and
$A_X$ is a measured value with LPS induction in the presence of a test compound. Inhibition rate of compounds with each concentration was calculated, and a value indicating 50 % inhibition ($IC_{50}$) was determined from the inhibition curve.
As a result, the compound of the present invention showed the inhibitory activity against TNF-α production. For example, the $IC_{50}$ values of the compounds described in Examples 48, and 52(9) were 2.4 nM, and 7.1 nM, respectively.

(3) Rat cytokine-production model

The in vivo effect of the compound of the present invention was studied on TNF-α production induced by lipopolysaccharide (LPS) in rats.

Experimental Method

A vehicle containing the compound of the present invention was orally administered to male Lew mice (Charles River Japan, Inc.), and after 2 hours, lipopolysaccharide (LPS, 055:B5, Difco) was intravenously administered at the dose of 10 μg/kg (5 animals/group). Only a vehicle was orally administered to a control group (5 animals). Ninety minutes after the LPS treatment, heparinized blood collection was performed via the abdominal cava vein under anesthesia with ether, and blood plasma was obtained by centrifugation (12,000 rpm, 3 minutes, 4 °C). The obtained blood plasma sample was stored at -80°C until it was used. TNF-α in the blood plasma was measured using an ELISA kit from Genzyme/Techne (#10516).

The inhibitory activity of the compound of the present invention against TNF-α production was calculated as an inhibition rate (%) according to the following equation:

$$\text{Inhibition rate (\%)}=\{(A_C - A_X)/A_C\} \times 100$$

wherein

$A_C$ is a measured value in case where no test compound was administered under LPS induction, and

$A_X$ is a measured value in case where a test compound was administered under LPS induction.

The results showed that the compound of the present invention has inhibitory activity against TNF-α production. For example, the compound of Example 48, and 52(9) of the present invention at the dose of 3 mg/kg showed an inhibition of 72 % against in vivo TNF-α production induced by LPS stimulation.

Formulation Examples

Formulation Example 1

1-(2,6-dichlorophenyl)-5-[(2,4-difluorophenyl)(hydroxyimino)methyl]pyridine-2-(1H)-one (a mixture of (E)-form and (Z)-form) (5.0 kg), carboxymethylcellulose calcium (disintegrator) (0.2 kg), magnesium stearate (lubricant) (0.1 kg) and microcrystalline cellulose (4.7 kg) were admixed in a conventional manner, and tabletted to obtain 100,000 tablets containing an active ingredient of 50 mg/tablet.

Formulation Example 2

1-(2,6-dichlorophenyl)-5-[(2,4-difluorophenyl)(hydroxyimino)methyl]pyridine-2-(1H)-one (a mixture of (E)-form and (Z)-form) (2.0 kg), mannitol (20 kg), and distilled water (500 L) were admixed in a conventional manner, filtered with a dust filter, filled in ampoules (5 ml each), and heat-sterilized in an autoclave to obtain 100,000 ampoules containing an active ingredient of 20 mg/ampoule.

Industrial Applicability

Since the compounds represented by general formula (I) of the present invention, or their salts, N-oxides or hydrates, or prodrugs thereof have a low toxicity, they can be used as raw materials for medicaments. Also, they are useful as an agent for the prevention and/or treatment of cytokine-mediated diseases such as rheumatoid arthritis and so on, because they have p38 MAP kinase inhibitory activity.

## Claims

**1.** A compound represented by the formula (I):

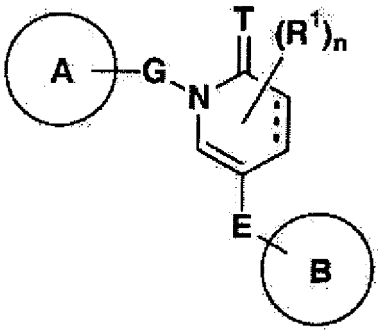

(I)

wherein ring A and ring B each independently represent an cyclic group which may be substituted;
E represents a spacer having 1 to 4 atoms in the main chain thereof;
G represents a bond, an oxygen atom, a sulfur atom which may be oxidized or a methylene group which may be substituted;
$R^1$ represents a substituent;
T represents an oxygen atom or a sulfur atom;
represents a single bond or a double bond;
n represents 0 or an integer of 1 to 5, and
wherein when n represents 2 or more, each of $R^1$'s are the same or different, a salt thereof, an N-oxide thereof, a solvate thereof, or a prodrug thereof.

**2.** The compound according to claim 1, wherein ring A is a benzene ring which may be substituted.

**3.** The compound according to claim 1, wherein ring B is a benzene ring which may be substituted.

**4.** The compound according to claim 3, wherein ring B is substituted by a fluorine atom.

**5.** The compound according to claim 1, wherein E is C1-4 alkylene, C1-4 alkylene substituted by hydroxy, C1-4 alkylene substituted by oxo, C1-4 alkylene substituted by C1-4 alkylene, C1-4 alkylene substituted by C1-4 alkoxy, -S-, -C(=N-$NH_2$)- or -C(=N-$OR^2$)- in which $R^2$ represents a hydrogen atom or a substituent.

**6.** The compound according to claim 1, wherein E is -C(=N-$OR^2$)- in which $R^2$ has the same meaning as $R^2$ defined in claim 5.

**7.** The compound according to claim 1, wherein is a double bond.

**8.** The compound according to claim 1, wherein T is an oxygen atom.

**9.** The compound according to claim 1, wherein G is a bond.

**10.** The compound according to claim 1, which is represented by the formula (Ia-2) or the formula (Ia-3):

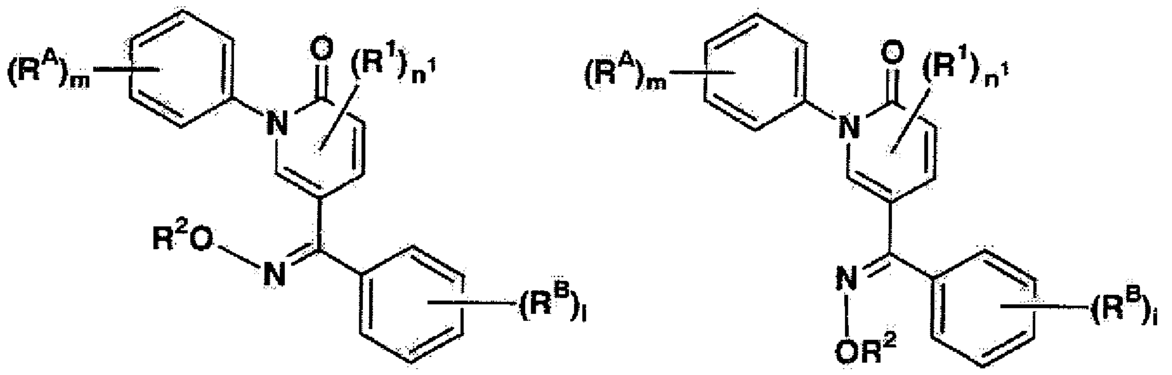

(Ia-2) (Ia-3)

wherein $R^A$ and $R^B$ each independently represent a substituent;
m and i each independently represents 0 or an integer of 1 to 5, wherein when m represents 2 or more, each of $R^A$'s are the same or different, and when i represents 2 or more, each of $R^B$'s are the same or different;
$n^1$ represents 0 or an integer of 1 to 3, wherein when $n^1$ represents 2 or more, each of $R^1$'s are the same or different; and
$R^1$ and $R^2$ have the same meanings as $R^1$ and $R^2$ defined in claim 1 and 5, or a mixture of the compound represented by the formula (Ia-2) and the compound represented by the formula (Ia-3) in an arbitrary ratio.

**11.** The compound according to claim 10, which is represented by the formula (Ia-3):

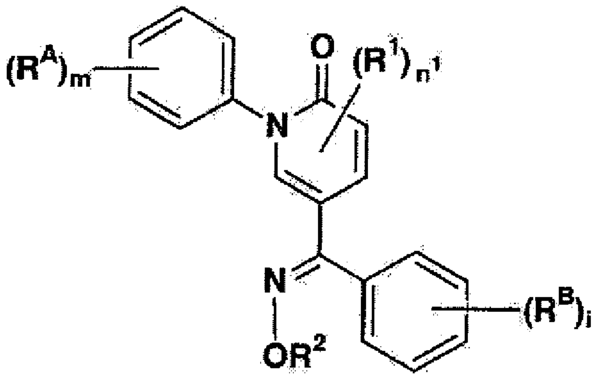

(Ia-3)

wherein $R^1$, $R^2$, $R^A$, $R^B$, m, i and $n^1$ have the same meanings as $R^1$, $R^2$, $R^A$, $R^B$, m, i and $n^1$ defined in claim 1, 5 and 10.

**12.** The compound according to claim 10 or 11, wherein $R^2$ is

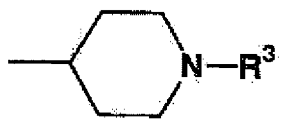

in which $R^3$ represents a hydrogen atom or a substituent.

**13.** The compound according to claim 12, wherein $R^3$ is a hydrogen atom, methyl, ethyl, or dimethylaminoethyl.

**14.** The compound according to claim 10 to 13,
wherein $R^A$ is methyl, ethyl, methoxy, or a halogen atom; and
m is an integer of 1 to 5, and
wherein when m is 2 or more, each of $R^A$'s are the same or different.

**15.** The compound according to claim 10 to 13, wherein $R^B$ is methyl or a halogen atom; and i is an integer of 1 to 5, and wherein when i is 2 or more, each of $R^B$'s are the same or different.

**16.** The compound according to claim 1, which is
5-((2,4-difluorophenyl) {[(1-methyl-4-piperidinyl)oxy]imino }methyl)-1-(2,6-dimethylphenyl)-2(1H)-pyridinone,
5-{ (2,4- difluorophenyl) [ ( {1-[2-(dimethylamino) ethyl]- 4- piperidinyl} oxy) imino] methyl}- 1-(2,6- dimethylphenyl)- 2 (1H)-pyridinone,
5-{(Z)-(2,4-difluorophenyl)[(4-piperidinyloxy)imino]methyl}-1-(4-fluoro-2,6-dimethylphenyl)-2(1H)-pyridinone,
5-((Z)-(2,4-difluorophenyl) {[(1-methyl-4-piperidinyl)oxy]imino}methyl)-1-(4-fluoro-2,6-dimethylphenyl)-2(1H)-pyridinone,
5-((Z)-(2,4-difluorophenyl){[(1-ethyl-4-piperidinyl)oxy]imino}methyl)-1-(4-fluoro-2,6-dimethylphenyl)-2(1H)-pyridinone,
5-((E)-(2,4-difluorophenyl) {[(1-methyl-4-piperidinyl)oxy]imino}methyl)-1-(4-fluoro-2,6-dimethylphenyl)-2(1H)-pyridinone,
1-(2,6-difluorophenyl)-5-{ (2,4-difluorophenyl)[(4-piperidinyloxy)imino]methyl}-2(1H)-pyridinone,
5-{(2,4-difluorophenyl)[(4-piperidinyloxy)imino]methyl}-1-(2-methoxy-6-methylphenyl)-2(1H)-pyridinone,
1-(2,6-diethylphenyl)-5-{(2,4-difluorophenyl)[(4-piperidinyloxy)imino]methyl}-2(1H)-pyridinone,
1-(2-chloro-6-methylphenyl)-5-{(2,4-dilluorophenyl)[(4-piperidinyloxy)imino]methyl}-2(1H)-pyridinone,
5-{(2,4-difluorophenyl)[(4-piperidinyloxy)imino]methyl}-1-(2-ethyl-6-methylphenyl)-2(1H)-pyridinone,
5-{(2,4-difluorophenyl)[(4-piperidinyloxy)imino]methyl}-1-(2,6-dimethoxyphenyl)-2(1H)-pyridinone,
1-(2,6-difluorophenyl)-5-((Z)-(2,4-difluorophenyl){[(1-methyl-4-piperidinyl)oxy]imino}methyl)-2(1H)-pyridinone,
1-(2,6-difluorophenyl)-5-((E)-(2,4-difluorophenyl){[(1-methyl-4-piperidinyl)oxy]imino}methyl)-2(1H)-pyridinone,
5-((2,4- difluorophenyl) { [ (1- methyl- 4- piperidinyl) oxy] imino} methyl)- 1-(2- methoxy- 6- methylphenyl)- 2 (1H)-pyridinone,
1-(2,6-diethylphenyl)-5-((2,4-difluorophenyl){[(1-methyl-4-piperidinyl)oxy]imino}methyl)-2(1H)-pyridinone,
1-(2-chloro-6-methylphenyl)-5-((2,4-difluorophenyl){[(1-methyl-4-piperidinyl)oxy]imino}methyl)-2(1H)-pyridinone,
5-((2,4-difluorophenyl){[(1-methyl-4-piperidinyl)oxy]imino}methyl)-1-(2-ethyl-6-methylphenyl)-2(1H)-pyridinone,
5-((2,4-difluorophenyl){[(1-methyl-4-piperidinyl)oxy]imino}methyl)-1-(2,6-dimethoxyphenyl)-2(1H)-pyridinone,
5-((2,4-difluorophenyl){ [(1-methyl-4-piperidinyl)oxy]imino}methyl)-1-mesityl-2(1H)-pyridinone,
5-((E)-(2,4-difluorophenyl) {[(1-ethyl-4-piperidinyl)oxy]imino}methyl)-1-(2,6-dimethylphenyl)-2(1H)-pyridinone,
5-((Z)-(2,4-difluorophenyl) {[(1-ethyl-4-piperidinyl)oxy]imino}methyl)-1-(2,6-dimethylphenyl)-2(1H)-pyridinone,
5-{(2,4-difluorophenyl)[(4-piperidinylmethoxy)imino]methyl}-1-(2,6-dimethylphenyl)-2(1H)-pyridinone,
5-((2,4-difluorophenyl){[(1-methyl-4-piperidinyl)methoxy]imino}methyl)-1-(2,6-dimethylphenyl)-2(1H)-pyridinone,
1-(2,6-dichlorophenyl)-5-{(2-fluoro-4-methylphenyl)[(4-piperidinyloxy)imino]methyl}-2(1H)-pyridinone, or
1-(2,6-dichlorophenyl)-5-{(4-fluoro-2-methylphenyl)[(4-piperidinyloxy)imino]methyl}-2(1H)-pyridinone.

**17.** The compound according to claim 1, which is
5-((2,4-difluorophenyl){[(1-methyl-4-piperidinyl)oxy]imino}methyl)-1-(2,6-dimethylphenyl)-2(1H)-pyridinone,
5-((Z)-(2,4-difluorophenyl){[(1-methyl-4-piperidinyl)oxy]imino}methyl)-1-(4-fluoro-2,6-dimethylphenyl)-2(1H)-pyridinone,
1-(2,6-difluorophenyl)-5-((Z)-(2,4-difluorophenyl){[(1-methyl-4-piperidinyl)oxy]imino}methyl)-2(1H)-pyridinone,
5-((2,4-difluorophenyl) {[(1-methyl-4-piperidinyl)oxy]imino}methyl)-1-mesityl-2(1H)-pyridinone, or
5-((Z)-(2,4-difluorophenyl){[(1-ethyl-4-piperidinyl)oxy]imino}methyl)-1-(2,6-dimethylphenyl)-2(1H)-pyridinone.

**18.** A compound represented by the formula (I-R):

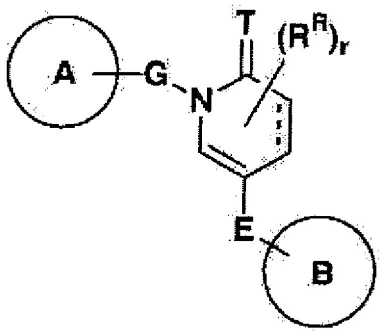

(I-R)

wherein $R^R$ represents a substituent; and
r represents an integer of 2 to 5,
wherein each of $R^R$'s are the same or different, and other symbols have the same meanings as defined in claim 1, and wherein at least two of $R^R$'s are taken together with a carbon atom which bound them to represent a cyclic group which may be substituted, a salt thereof, an N-oxide thereof, a solvate thereof, or a prodrug thereof

**19.** A pharmaceutical composition comprising the compound represented by the formula (I) according to claim 1, a salt thereof, an N-oxide thereof, a solvate thereof, or a prodrug thereof.

**20.** The composition according to claim 19, which is a p38 MAP kinase inhibitor and/or a TNF-α production inhibitor.

**21.** The composition according to claim 19, which is an agent for the prevention and/or treatment of cytokine-mediated diseases.

**22.** The composition according to claim 21, wherein the cytokine-mediated disease is an inflammatory disease, a cardiovascular disease, a respiratory disease and/or a bone disease.

**23.** The composition according to claim 22, wherein the inflammatory disease is rheumatoid arthritis.

**24.** A pharmaceutical composition comprising the compound represented by the formula (I) according to claim 1, a salt thereof, an N-oxide thereof, a solvate thereof, or a prodrug thereof, in conbination with one or two or more kinds selected from a non-steroidal anti-inflammatory agent, a disease modifying anti-rheumatic drug, an anticytokine protein preparation, a cytokine inhibitor, an immunomodulator, a steroidal agent, an adhesion molecule inhibitor, an elastase inhhibitor, a cannabinoid-2 receptor stimulant, a prostaglandin, a prostaglandin synthase inhibitor, a phosphodiesterase inhibitor and a metalloproteinase inhibitor.

**25.** A method for the inhibition of p38 MAP kinase and/or the inhibition of TNF-α production in mammal, which comprises administering to a mammal an effective amount of the compound represented by the formula (I) according to claim 1, a salt thereof, an N-oxide thereof, a solvate thereof, or a prodrug thereof

**26.** A method for the prevention and/or treatment of cytokine-mediated diseases in mammal, which comprises administering to a mammal an effective amount of the compound represented by the formula (I) according to claim 1, a salt thereof, an N-oxide thereof, a solvate thereof, or a prodrug thereof

**27.** Use of the compound represented by the formula (I) according to claim 1, or a salt thereof, an N-oxide thereof a solvate thereof or a prodrug thereof for the manufacture of an agent for the inhibition of p38 MAP kinase and/or the inhibition of TNF-α production.

**28.** Use of the compound represented by the formula (I) according to claim 1, or a salt thereof, an N-oxide thereof, a solvate thereof, or a prodrug thereof for the manufacture of an agent for the prevention and/or treatment of a cytokine-mediated disease.

## INTERNATIONAL SEARCH REPORT

International application No.
PCT/JP2005/008065

A. CLASSIFICATION OF SUBJECT MATTER
Int.Cl⁷ C07D211/82, A61K31/438, 31/4418, 31/443, 31/4439, 31/444, 31/4545, 31/496, 31/506, 31/5377, 31/55, 45/00, A61P3/00, 5/00, 9/00, 11/00, 13/00, 19/02, 19/08, 25/28, 29/00, 31/00,

According to International Patent Classification (IPC) or to both national classification and IPC

B. FIELDS SEARCHED

Minimum documentation searched (classification system followed by classification symbols)
Int.Cl⁷ C07D211/82, A61K31/438, 31/4418, 31/443, 31/4439, 31/444, 31/4545, 31/496, 31/506, 31/5377, 31/55, 45/00, A61P3/00, 5/00, 9/00, 11/00, 13/00, 19/02, 19/08, 25/28, 29/00, 31/00,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAPLUS(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 2003/076405 A1 (BAYER AG.),<br>18 September, 2003 (18.09.03),<br>Full text<br>& EP 1487794 A1 | 1-5,7-9,<br>19-24,27,28<br>6,10-18 |
| Y | WO 2003/043988 A1 (Ono Pharmaceutical Co., Ltd.),<br>30 May, 2003 (30.05.03),<br>Particularly, examples 167, 172<br>& EP 1447401 A1 & US 2005/085509 A1 | 6,10-18 |
| X<br><br>Y | WO 2003/068230 A1 (PHARMACIA CORP.),<br>21 August, 2003 (21.08.03),<br>Full text<br>& EP 1490064 A1 & US 2004/058964 A1 | 1-4,7-9,<br>18-24,27,28<br>6,10-17 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 May, 2005 (24.05.05) | Date of mailing of the international search report<br>07 June, 2005 (07.06.05) |
| Name and mailing address of the ISA/<br>Japanese Patent Office<br>Facsimile No. | Authorized officer<br><br>Telephone No. |

Form PCT/ISA/210 (second sheet) (January 2004)

INTERNATIONAL SEARCH REPORT

International application No.
PCT/JP2005/008065

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | WO 2004/004720 A1 (Astex Technology Ltd.),<br>15 January, 2004 (15.01.04),<br>Full text<br>(Family: none) | 1-4,7,8,<br>19-24,27,28<br>6,10-18 |
| X | WO 2004/020410 A1 (Bayer Healthcare A.-G.),<br>11 March, 2004 (11.03.04),<br>Full text<br>(Family: none) | 1-4,8,9,18,<br>19 |
| X | WO 2004/022536 A1 (Glenmark Pharmaceuticals Ltd.),<br>18 March, 2004 (18.03.04),<br>Full text<br>(Family: none) | 1-3,8,19-24,<br>27,28 |
| P,X | WO 2005/018557 A2 (Pharmacia Corp.),<br>03 March, 2005 (03.03.05),<br>Full text<br>& NL 1026826 A1 | 1-5,7-9,<br>18-24,27,28 |

Form PCT/ISA/210 (continuation of second sheet) (January 2004)

INTERNATIONAL SEARCH REPORT

International application No.
PCT/JP2005/008065

Continuation of A. CLASSIFICATION OF SUBJECT MATTER
(International Patent Classification (IPC))

Int.Cl[7] 35/00, 43/00, C07D213/64, 213/75, 213/89, 401/12, 405/12, 413/12, 417/12, 471/10

(According to International Patent Classification (IPC) or to both national classification and IPC)

Continuation of B. FIELDS SEARCHED
Minimum documentation searched (International Patent Classification (IPC))

Int.Cl[7] 35/00, 43/00, C07D213/64, 213/75, 213/89, 401/12, 405/12, 413/12, 417/12, 471/10

Minimum documentation searched (classification system followed by classification symbols)

Form PCT/ISA/210 (extra sheet) (January 2004)

INTERNATIONAL SEARCH REPORT

International application No.
PCT/JP2005/008065

**Box No. II Observations where certain claims were found unsearchable (Continuation of item 2 of first sheet)**

This international search report has not been established in respect of certain claims under Article 17(2)(a) for the following reasons:

1. [x] Claims Nos.: 25, 26
because they relate to subject matter not required to be searched by this Authority, namely:
Claims 25 and 26 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
As described in WO 2003/043988 A1, WO 2003/076405 A1, WO 03/068230 A1, etc., the skeleton of the compounds represented by the general formula (I) in the inventions as claimed in claim 1 had been publicly known prior to the application of the present case. Thus, there is no special technical feature exceeding prior art common to the inventions as claimed in the above claim and they cannot be considered as being so linked as to form a single general inventive concept. The same applies to the inventions as claimed in other claims depending on the above claim.

1. [ ] As all required additional search fees were timely paid by the applicant, this international search report covers all searchable claims.
2. [x] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

INTERNATIONAL SEARCH REPORT

International application No.
PCT/JP2005/008065

Concerning "prodrugs" as set forth in claims 1, 18, 24, 27 and 28, only a small part thereof, as cited in p. 39 of the description of the present case, are disclosed in the meaning within PCT Article 5 and thus they are not sufficiently supported in the meaning within PCT Article 6. Although the common technical knowledge at the point of the application is taken into consideration, it is unclear to what extent of compounds are involved in the scope of "prodrugs". Thus, they do not comply with the requirement of clearness in accordance with PCT Article 6 too.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03043988 A
- DE 10002509
- WO 00043384 A
- WO 03076405 A
- WO 01096308 A
- US 286891 A
- US 3095355 A
- WO 0035906 A
- WO 0035909 A
- WO 0035921 A
- WO 0064872 A
- WO 0075118 A


### Non-patent literature cited in the description


- *Nature,* 1994, vol. 372, 739
- Development of Drugs, Molecule Design. Hirokawa Publishing Co, 1990, vol. 7, 163-198
- **RICHARD C. LAROCK.** Comprehensive Organic Transformations: A Guide to Functional Group Preparations. John Wiley & Sons Inc, 1999
- **T.W. GREENE.** Protective Groups in Organic Synthesis. John Wiley & Sons Inc, 1999
- **RICHARD C. LAROCK.** Comprehensive Organic Transformations: A Guide to Functional Group Preparations. John Wiley & Sons Inc, 1999